# EUROPEAN PATENT SPECIFICATION

(11) **EP 3 827 095 B1**
(45) Date of publication and mention of the grant of the patent: **21.05.2025**
(21) Application number: 19840761.1
(22) Date of filing: 23.07.2019
(51) Int. Cl.: C12Q 1/6827, C12Q 1/6869, C12Q 1/6883, C12Q 1/6886, C12Q 1/6806, C12Q 1/6881

(54) **CELL-FREE DNA DAMAGE ANALYSIS AND ITS CLINICAL APPLICATIONS**
SCHADENSANALYSE VON ZELLFREIER DNA UND IHRE KLINISCHEN ANWENDUNGEN
ANALYSE DE L'ALTÉRATION DE L'ADN ACELLULAIRE ET SES APPLICATIONS CLINIQUES

(30) Priority: 23.07.2018 US 201862702080 P; 26.12.2018 US 201862785118 P
(43) Date of publication of application: 02.06.2021
(73) Proprietor: The Chinese University Of Hong Kong, Hong Kong 999077 (HK); Grail, Inc., Menlo Park, California 94025 (US)
(72) Inventor: LO, Yuk-Ming Dennis, Hong Kong (CN); CHIU, Rossa Wai Kwun, Hong Kong (CN); CHAN, Kwan Chee, Hong Kong (CN); JIANG, Peiyong, Hong Kong (CN); CHENG, Suk Hang, Hong Kong (CN)
(74) Representative: J A Kemp LLP
(86) International application number: PCT/CN2019/097329
(87) International publication number: WO 2020/020174

(56) References cited:
- WO-A1-2015/040591
- WO-A1-2015/089333
- WO-A1-2017/012592
- WO-A1-2018/081130
- WO-A1-2018/107598
- US-A1- 2013 224 740
- FLORENT MOULIERE ET AL: "Circulating tumor-derived DNA is shorter than somatic DNA in plasma", PROCEEDINGS OF THE NATIONAL ACADEMY OF SCIENCES, vol. 112, no. 11, 2 March 2015 (2015-03-02), pages 3178 - 3179, XP055223841, ISSN: 0027-8424, DOI: 10.1073/pnas.1501321112
- SNYDER, M.W.ET AL.: "Cell-free DNA Comprises an In Vivo Nucleosome Footprint that Informs Its Tissues-Of-Origin.", CELL., vol. 164, 14 January 2016 (2016-01-14), pages 57 - 68, XP055367434, DOI: 20191008181542Y
- WONG, F.C.K. ET AL.: "Cell-free DNA in maternal plasma and serum: A comparison of quantity, quality and tissue origin using genomic and epigenomic approaches.", CLINICAL BIOCHEMISTRY., vol. 49, 9 September 2016 (2016-09-09), pages 1379 - 1386, XP029819842, DOI: 20191009102610A

## Description

Cell-free DNA has been proven to be particularly useful for molecular diagnostics and monitoring. The cell-free based applications include noninvasive prenatal testing (Chiu RKW et al. Proc Natl Acad Sci USA. 2008; 105:20458-63), cancer detection and monitoring (Chan KCA et al. Clin Chem. 2013;59:211-24; Chan KCA et al. Proc Natl Acad Sci USA. 2013; 110: 1876-8; Jiang P et al. Proc Natl Acad Sci USA. 2015;112:E1317-25), transplantation monitoring (Zheng YW et al. Clin Chem. 2012;58:549-58) and tracing tissue of origin (Sun K et al. Proc Natl Acad Sci USA. 2015;112:E5503-12; Chan KCA; Snyder MW et al. Cell. 2016;164:57-68). Cell-free nucleic acid analysis approaches developed to date include those based on the analysis of single nucleotide variants (SNVs), copy number aberrations (CNAs), cell-free DNA ending positions in the human genome, or methylation markers. US 2013/224740 A1, WO 2018/107598 A1, WO 2015/040591 A1 describe cell -free nucleic acid analysis based on the deferential size distribution of cell-free DNA fragments of cancer or foetal origin. It would be beneficial to identify new nucleic acid analysis approaches for detection of new properties and to add accuracy to existing approaches.

According to the present invention, there is provided a method as defined in claim 1.

Double-stranded cell-free DNA fragments may often have two strands that are not exactly complementary to each other. One strand may extend beyond the other strand, creating an overhang. These overhangs are often repaired to form blunt ends in analysis. However, the "jagged ends" created by these overhangs may be useful in analyzing biological samples. This document describes how jagged ends may be used in analysis and how to measure the jagged ends.

The degree of jagged ends, which may be the quantity or the length of jagged ends, in a sample may reflect the level of a condition in an individual. For example, the degree of jagged ends may be related to a disease, a disorder, a pregnancy-related condition. The jagged ends may be used to determine the fractional concentration of clinically-relevant DNA in a sample. The age of an individual may be related to the degree of jagged ends. Jagged ends from specific tissues may be analyzed, and the degree of jagged ends may determine a level of cancer.

The degree of jagged ends may be measured in various ways. For example, the jagged ends may be repaired using methylated or unmethylated nucleotides, and the resulting change in the level of methylation can indicate the presence and/or length of a jagged end. In some cases, methylated cytosines can be used in end repair to measure the exact length of a jagged end. As another example, the degree of jagged ends may also be determined by aligning portions of the fragments to a reference genome or a complementary strand or measuring other signals from nucleotides added through end repair.

A better understanding of the nature and advantages of embodiments of the present invention may be gained with reference to the following detailed description and the accompanying drawings.
FIG. 1 shows a method of using jagged end values to analyze a biological sample according to embodiments of the present invention.
FIG. 2 shows one example for assessing the degree of 5' overhangs according to embodiments of the present invention.
FIG. 3. Illustration of the calculation of methylation levels along a DNA molecule after mapping to the human reference genome according to embodiments of the present invention.
FIG. 4 shows a method of analyzing a biological sample obtained from an individual to calculate a jagged end value using methylation levels according to embodiments of the present invention.
FIGS. 5A-5B shows representative plots for overhang indices among sonicated liver tissue DNA (A), plasma DNA of a pregnant woman (B) according to embodiments of the present invention.
FIG. 6 shows the difference in overhang indices between sonicated tissue DNA and cell-free DNA samples according to embodiments of the present invention.
FIGS. 7A-7C shows the difference in overhang indices between fetal and maternal DNA molecules in plasma of pregnant women across different trimesters according to embodiments of the present invention.
FIG. 8 shows the overhang indices of fetal DNA molecules were well correlated with fetal DNA fractions according to embodiments of the present invention.
FIG. 9 shows overhang index across different size ranges for plasma DNA molecules from pregnant women according to embodiments of the present invention.
FIG. 10 shows one example of overhang index of maternal and fetal DNA in a particular size range and overhang index ratio between two different size ranges according to embodiments of the present invention.
FIG. 11 shows the overall overhang index ratio correlated with fetal DNA fractions according to embodiments of the present invention. In addition, the plasma DNA exhibited distinct overhang index patterns across different sizes in comparison with sonicated tissue DNA (FIG. 12).
FIG. 12 shows comparison of overhang index across different size ranges between plasma DNA molecules and sonicated DNA according to embodiments of the present invention.
FIG. 13 shows the jagged index between fetal DNA and maternal DNA across different trimesters according to embodiments of the present invention.
FIG. 14 shows the correlation between fetal DNA fraction and jagged end index ratio according to embodiments of the present invention.
FIG. 15 shows an approach for using methylated cytosines in end repair according to embodiments of the present invention.
FIG. 16 shows using methylated cytosines to determine the length of a jagged end according to embodiments of the present invention.
FIG. 17 is a table of DNA samples analyzed using end repair with methylated cytosines according to embodiments of the present invention.
FIG. 18 shows the use of two synthesis double-stranded DNA fragments with jagged ends of known lengths as internal controls according to embodiments of the present invention.
FIGS. 19A and 19B show the sequencing results for two spike-in sequences with known jagged ends having known sequences according to embodiments of the present invention.
FIG. 20 shows representative plots for the proportion of methylated cytosines in plasma DNA of pregnant women using either CH or CG sites according to embodiments of the present invention.
FIG. 21 is a table comparing the relative informative power between approaches using the filling methylated cytosines (mCs) and unmethylated cytosines (Cs) according to embodiments of the present invention.
FIG. 22 shows the distribution of jagged end lengths deduced by the "CC-tag" strategy according to embodiments of the present invention.
FIGS. 23A, 23B, and 24 shows the profile of jagged ends across different size ranges of cell-free DNA fragments according to embodiments of the present invention.
FIG. 25 shows a table with sequencing information and fetal DNA fractions for different pregnant women according to embodiments of the present invention.
FIG. 26 shows a representative plot for one sample for the proportion of methylated cytosines in plasma DNA of pregnant women at CH sites according to embodiments of the present invention.
FIGS. 27A, 27B, 28A, and 28B show the profile of jagged ends across different size ranges for fetal-specific and shared DNA molecules according to embodiments of the present invention.
FIGS. 29A and 29B show the jagged end length distributions in molecules within 140-150 bp according to embodiments of the present invention.
FIGS. 30A, 30B, and 31 show jagged end length versus fetal DNA fraction for molecules of 140 bp, 166 bp, and 200 bp according to embodiments of the present invention.
FIG. 32 shows size distributions for molecules carrying different size jagged end lengths according to embodiments of the present invention.
FIG. 33 shows a method for calculating a jagged end value with CC-tags according to embodiments of the present invention.
FIG. 34 show DNA fragment end ligation-mediated plasma DNA overhang determination according to embodiments of the present invention.
FIG. 35 show DNA fragment end ligation-mediated plasma DNA overhang determination with the use of a genomic common sequence according to embodiments of the present invention.
FIG. 36 show the frequency profile of overhang length in maternal plasma DNA according to embodiments of the present invention.
FIG. 37 show the correlation of overhang length frequency between mapping to the whole genome and adjacent sequences around the common sequence identified in a human genome according to embodiments of the present invention.
FIG. 38 shows a method of analyzing a biological sample obtained from an individual to determine a length of a jagged end using an identifier molecule according to embodiments of the present invention.
FIG. 39 show the relative abundance of a particular overhang length could be inferred from the BS-seq results according to embodiments of the present invention.
FIG. 40 show the relative abundance of a particular overhang length could be inferred from the BS-seq results according to embodiments of the present invention. The x-axis is the overhang length being studied. The y-axis is the relative methylation reduction between two neighboring cycles.
FIG. 41 show the comparison between the ligation-based and BS-seq based approaches according to embodiments of the present invention.
FIG. 42 shows a method of analyzing a biological sample obtained from an individual to determine lengths and amounts of jagged ends using bisulfite sequencing according to embodiments of the present invention.
FIG. 43 show the distribution of size for the fragments being able to be ligated with designed oligonucleotides according to embodiments of the present invention.
FIG. 44 show the relationship between overhang length and fragment size according to embodiments of the present invention.
FIG. 45 show the difference in overhang indices of plasma DNA between cancer and non-cancer subjects according to embodiments of the present invention.
FIG. 46 shows the jagged index ratio across different clinical conditions according to embodiments of the present invention.
FIG. 47 shows the receiver operating characteristic (ROC) analysis for jagged index ratio and hypermethylation according to embodiments of the present invention.
FIG. 48 shows the jagged index ratio across different clinical conditions according to embodiments of the present invention.
FIG. 49 shows combined analysis of clinical conditions using hypermethylation and jagged index ratio according to embodiments of the present invention.
FIG. 50 show the difference in overhang indices of plasma DNA between healthy, inactive systemic lupus erythematosus (SLE) and active SLE subjects according to embodiments of the present invention.
FIG. 51 show the overhang index across different size ranges for healthy controls and HCC patients according to embodiments of the present invention.
FIG. 52A show under curve values of receiver operating characteristic (ROC) analysis for overhang indices across different size ranges between healthy controls and HCC patients. AUC: area under receiver operating characteristic curve according to embodiments of the present invention.
FIG. 52B show the difference in overhang indices of plasma DNA between cancer and non-cancer subjects without any size selection according to embodiments of the present invention.
FIG. 53 shows a heatmap of jagged index across different size range according to embodiments of the present invention.
FIG. 54 show overhang indices across different size ranges for healthy controls, inactive and active SLE patients according to embodiments of the present invention.
FIG. 55 show under curve values of receiver operating characteristic (ROC) analysis for overhang indices across different size ranges between healthy/inactive SLE subjects and active SLE patients according to embodiments of the present invention. AUC: area under receiver operating characteristic curve.
FIG. 56 show circos plot of overhang index between pre- and post-operative plasma DNA of a HCC patient according to embodiments of the present invention. Chromosome ideograms (outside the plots) are oriented pter to qter in a clockwise direction. The overhang of each 1-Mb bin for overhang index of pre-surgery plasma DNA (red rectangle) and post-surgery plasma DNA (blue triangle) were shown in the inner ring. The range of overhang index was from 0% (innermost) to 16% (outermost) and the distance between two lines was 2%. Each dot represented a 1-Mb genomic region.
FIG. 57 show overhang index unevenly distributing around TSS. TSS: transcription start sites according to embodiments of the present invention.
FIG. 58A show overhang index across different tissue-specific open chromatin regions: overhang indices between open and non-open chromatin regions across different tissues in heathy subjects according to embodiments of the present invention.
FIG. 58B show overhang index across different tissue-specific open chromatin regions: overhang indices between open and non-open chromatin regions across different tissues in HCC subjects according to embodiments of the present invention.
FIG. 58C show overhang index across different tissue-specific open chromatin regions: the difference in overhang index between open and non-open chromatin regions across different tissues in control and HCC subjects according to embodiments of the present invention.
FIG. 58D show overhang index across different tissue-specific open chromatin regions: the statistical significance (Mann-Whitney test) of difference in overhang index between open and non-open chromatin regions across different tissues according to embodiments of the present invention.
FIG. 59 shows a method of analyzing a biological sample to determine whether a tissue type exhibits a cancer using jagged end values according to embodiments of the present invention.
FIG. 60 show direct assessment of plasma DNA sticky ends/overhangs through circularization of plasma DNA according to embodiments of the present invention.
FIG. 61 shows a technique for direct assessment of plasma DNA jagged ends through circularization of plasma DNA using a restriction enzyme according to embodiments of the present invention.
FIG. 62 shows a technique for direct assessment of plasma DNA jagged ends through circularization of plasma DNA using a polymerase binding site according to embodiments of the present invention.
FIG. 63 show direct assessment of plasma DNA sticky ends/overhangs through circularization of plasma DNA without random tagging amplification according to embodiments of the present invention.
FIG. 64 shows a method of analyzing a biological sample to determine whether a jagged end exists using a circularized double-stranded nucleic acid molecule according to embodiments of the present invention.
FIG. 65 shows a method of analyzing a biological sample to determine whether a jagged end exists using nucleotide analogs according to embodiments of the present invention.
FIG. 66 shows assessing jagged ends using inosine based sequencing according to embodiments of the present invention.
FIG. 67 shows a method for measuring a jagged end of a double-stranded nucleic acid molecule according to embodiments of the present invention.
FIG. 68 shows an overhang index based age prediction according to embodiments of the present invention.
FIG. 69 illustrates a measurement system according to embodiments of the present invention.
FIG. 70 shows a block diagram of an example computer system usable with systems and methods according to embodiments of the present invention.

A "tissue" corresponds to a group of cells that group together as a functional unit. More than one type of cells can be found in a single tissue. Different types of tissue may consist of different types of cells (e.g., hepatocytes, alveolar cells or blood cells), but also may correspond to tissue from different organisms (mother vs. fetus) or to healthy cells vs. tumor cells. "Reference tissues" can correspond to tissues used to determine tissue-specific methylation levels. Multiple samples of a same tissue type from different individuals may be used to determine a tissue-specific methylation level for that tissue type.

A *"biological sample"* refers to any sample that is taken from a subject (*e.g.*, a human, such as a pregnant woman, a person with cancer, or a person suspected of having cancer, an organ transplant recipient or a subject suspected of having a disease process involving an organ (e.g., the heart in myocardial infarction, or the brain in stroke, or the hematopoietic system in anemia) and contains one or more nucleic acid molecule(s) of interest. The biological sample can be a bodily fluid, such as blood, plasma, serum, urine, vaginal fluid, fluid from a hydrocele (e.g. of the testis), vaginal flushing fluids, pleural fluid, ascitic fluid, cerebrospinal fluid, saliva, sweat, tears, sputum, bronchoalveolar lavage fluid, discharge fluid from the nipple, aspiration fluid from different parts of the body (e.g. thyroid, breast), etc. Stool samples can also be used. In various embodiments, the majority of DNA in a biological sample that has been enriched for cell-free DNA (e.g., a plasma sample obtained via a centrifugation protocol) can be cell-free, e.g., greater than 50%, 60%, 70%, 80%, 90%, 95%, or 99% of the DNA can be cell-free. The centrifugation protocol can include, for example, 3,000 g x 10 minutes, obtaining the fluid part, and re-centrifuging at for example, 30,000 g for another 10 minutes to remove residual cells.

A "sequence read" refers to a string of nucleotides sequenced from any part or all of a nucleic acid molecule. For example, a sequence read may be a short string of nucleotides (e.g., 20-150) sequenced from a nucleic acid fragment, a short string of nucleotides at one or both ends of a nucleic acid fragment, or the sequencing of the entire nucleic acid fragment that exists in the biological sample. A sequence read may be obtained in a variety of ways, e.g., using sequencing techniques or using probes, e.g., in hybridization arrays or capture probes, or amplification techniques, such as the polymerase chain reaction (PCR) or linear amplification using a single primer or isothermal amplification.

An *"ending position"* or *"end position"* (or just *"end*) can refer to the genomic coordinate or genomic identity or nucleotide identity of the outermost base, i.e. at the extremities, of a cell-free DNA molecule, e.g. plasma DNA molecule. The end position can correspond to either end of a DNA molecule. In this manner, if one refers to a start and end of a DNA molecule, both would correspond to an ending position. In practice, one end position is the genomic coordinate or the nucleotide identity of the outermost base on one extremity of a cell-free DNA molecule that is detected or determined by an analytical method, such as but not limited to massively parallel sequencing or next-generation sequencing, single molecule sequencing, double- or single-stranded DNA sequencing library preparation protocols, polymerase chain reaction (PCR), or microarray.

A *"calibration data point"* includes a *"calibration value"* and a measured or known property of the sample or subject, e.g., age or tissue-specific fraction (e.g., fetal or tumor). The calibration value can be a relative abundance as determined for a calibration sample, for which the property is known. The calibration data point can include the calibration value (e.g., a jagged end value, also called an overhang index) and the known (measured) property. The calibration data points may be defined in a variety of ways, e.g., as discrete points or as a calibration function (also called a calibration curve or calibration surface). The calibration function could be derived from additional mathematical transformation of the calibration data points. The calibration function can be linear or non-linear.

*A "site"* (also called a *"genomic site"*) corresponds to a single site, which may be a single base position or a group of correlated base positions, e.g., a CpG site or larger group of correlated base positions. A "locus" may correspond to a region that includes multiple sites. A locus can include just one site, which would make the locus equivalent to a site in that context.

The *"methylation index"* or *"methylation status"* for each genomic site (e.g., a CpG site) can refer to the proportion of DNA fragments (e.g., as determined from sequence reads or probes) showing methylation at the site over the total number of reads covering that site. A "read" can correspond to information (e.g., methylation status at a site) obtained from a DNA fragment. A read can be obtained using reagents (e.g. primers or probes) that preferentially hybridize to DNA fragments of a particular methylation status. Typically, such reagents are applied after treatment with a process that differentially modifies or differentially recognizes DNA molecules depending of their methylation status, e.g. bisulfite conversion, or methylation-sensitive restriction enzyme, or methylation binding proteins, or anti-methylcytosine antibodies, or single molecule sequencing techniques that recognize methylcytosines and hydroxymethylcytosines.

The *"methylation density"* of a region can refer to the number of reads at sites within the region showing methylation divided by the total number of reads covering the sites in the region. The sites may have specific characteristics, e.g., being CpG sites. Thus, the "CpG methylation density" of a region can refer to the number of reads showing CpG methylation divided by the total number of reads covering CpG sites in the region (e.g., a particular CpG site, CpG sites within a CpG island, or a larger region). For example, the methylation density for each 100-kb bin in the human genome can be determined from the total number of cytosines not converted after bisulfite treatment (which corresponds to methylated cytosine) at CpG sites as a proportion of all CpG sites covered by sequence reads mapped to the 100-kb region. This analysis can also be performed for other bin sizes, e.g. 500 bp, 5 kb, 10 kb, 50-kb or 1-Mb, etc. A region could be the entire genome or a chromosome or part of a chromosome (e.g. a chromosomal arm). The methylation index of a CpG site is the same as the methylation density for a region when the region only includes that CpG site. The "proportion of methylated cytosines" can refer the number of cytosine sites, "C's", that are shown to be methylated (for example unconverted after bisulfite conversion) over the total number of analyzed cytosine residues, i.e. including cytosines outside of the CpG context, in the region. The methylation index, methylation density and proportion of methylated cytosines are examples of "methylation levels." Apart from bisulfite conversion, other processes known to those skilled in the art can be used to interrogate the methylation status of DNA molecules, including, but not limited to enzymes sensitive to the methylation status (e.g. methylation-sensitive restriction enzymes), methylation binding proteins, single molecule sequencing using a platform sensitive to the methylation status (e.g. nanopore sequencing (Schreiber et al. Proc Natl Acad Sci 2013; 110: 18910-18915) and by the Pacific Biosciences single molecule real time analysis (Flusberg et al. Nat Methods 2010; 7: 461-465)).

The term *"sequencing depth"* refers to the number of times a locus is covered by a sequence read aligned to the locus. The locus could be as small as a nucleotide, or as large as a chromosome arm, or as large as the entire genome. Sequencing depth can be expressed as 50x, 100x, etc., where "x" refers to the number of times a locus is covered with a sequence read. Sequencing depth can also be applied to multiple loci, or the whole genome, in which case x can refer to the mean number of times the loci or the haploid genome, or the whole genome, respectively, is sequenced. Ultra-deep sequencing can refer to at least 100x in sequencing depth.

*A "separation value"* corresponds to a difference or a ratio involving two values, e.g., two fractional contributions or two methylation levels. The separation value could be a simple difference or ratio. As examples, a direct ratio of x/y is a separation value, as well as x/(x+y). The separation value can include other factors, e.g., multiplicative factors. As other examples, a difference or ratio of functions of the values can be used, e.g., a difference or ratio of the natural logarithms (ln) of the two values. A separation value can include a difference and a ratio.

The term *"classification"* as used herein refers to any number(s) or other characters(s) that are associated with a particular property of a sample. For example, a "+" symbol (or the word "positive") could signify that a sample is classified as having deletions or amplifications. The classification can be binary (e.g., positive or negative) or have more levels of classification (e.g., a scale from 1 to 10 or 0 to 1). The terms *"cutoff"* and *"threshold"* refer to predetermined numbers used in an operation. For example, a cutoff size can refer to a size above which fragments are excluded. A threshold value may be a value above or below which a particular classification applies. Either of these terms can be used in either of these contexts.

The term *"damage"* when describing DNA molecules may refer to DNA nicks, single strands present in double-stranded DNA, overhangs of double-stranded DNA, oxidative DNA modification with oxidized guanines, abasic sites, thymidine dimers, oxidized pyrimidines, blocked 3' end, or a jagged end.

The term *"jagged end"* may refer to sticky ends of DNA, overhangs of DNA, or where a double-stranded DNA includes a strand of DNA not hybridized to the other strand of DNA. *"Jagged end value"* is a measure of the extent of a jagged end. The jagged end value may be proportional to an average length of one strand that overhangs a second strand in double-stranded DNA. The jagged end value of a plurality of DNA molecules may include consideration of blunt ends among the DNA molecules.

Here we have invented new approaches for assessing the extent of cell-free DNA damages. A damaged cell-free DNA molecule may manifest as but not limited to within strand DNA nicks, overhangs of double-stranded DNA, oxidative DNA damage with oxidized guanines, abasic sites, thymidine dimers, oxidized pyrimidines, or blocked 3' end, etc. It was reported in a tumor-bearing mouse study that the presence of a tumor may induce a chronic inflammatory response in vivo, leading to increased systemic levels of DNA damage including double-strand breaks (DSBs) and oxidatively induced non-DSB clustered DNA lesions (Redon CE et al. Proc Natl Acad Scie USA. 2010;107:17992-7). However, the assessment of DNA damages in plasma DNA and its clinical utilities are not readily evident.

We hypothesized that DNA damages of cell-free DNA, which was unappreciated before, may have numerous clinical applications. First, the extent of cell-free DNA damage may reflect the quality of cell-free DNA samples, whether freshly collected or archived samples, whether the samples have been stored and processed well, whether the samples have been subjected to repeated freezing and thawing. Second, cell-free DNA damage may be increased in certain pathologies, such as those associated with inflammation (e.g. oxidative stress caused by intake of certain drugs), immunological attacks and autoimmunity, such as systemic lupus erythematosus. Third, the extent of cell-free DNA damage may be different between cell-free DNA molecules that originated from different tissue or organ sources. In other words, cell-free DNA damage may be associated with a tissue of origin and reflect the identity of the origin of a tumor. In addition, the extent of cell-free DNA damage may be different between fetal and maternal DNA in maternal plasma and provides a means to distinguish between circulating maternal cell-free DNA and circulating fetal cell-free DNA or provides a means to enrich or sort for circulating cell-free fetal DNA.

Cell-free DNA is known to be fragmented naturally in vivo. Cell-free DNA molecules, therefore, exist as short fragments in biological fluids, such as plasma, serum, urine, saliva, pleural fluid, cerebrospinal fluid, peritoneal fluid, synovial fluid and others. Pathologies within organs or tissues may result in different extent or form of fragmentation or damage to the cell-free DNA. In addition, pathologies, processes or conditions (e.g., intake of oxidizing drugs or chemicals) may cause further damage or alternation to the molecular form of the cell-free DNA molecules within the biological fluid after cellular release. In vitro processes (e.g. repeated freezing and thawing, exposure to extremes of temperatures) may induce further damage to the cell-free DNA molecules in a biological fluid sample or a specimen containing cell-free nucleic acids.

Different pathogenic reasons causing cell deaths in a particular organ or tissue might result in alterations in the relative presentation of DNA damages present in cell-free DNA molecules. For example, the overhangs of double-stranded DNA would bear the relationship with the tissue of origin. Therefore, embodiments of the present invention for analyzing cell-free DNA damages would offer new possibilities for detecting or monitoring, but not limited to, cancer detection, organ damages, immune diseases as well as performing noninvasive prenatal testing etc. Additionally, new techniques for performing measurements of DNA damage, e.g., referred to as jagged ends, are provided.

### I. EXAMINING OVERHANGS OF CELL-FREE DNA MOLECULES

Cell-free DNA ends would be classified into two forms according to modalities of ends. One form of cell-free DNA would be present in blood circulation with blunt ends and the other would carry sticky ends. A sticky end is an end of a double-stranded DNA that has at least one outermost nucleotide not hybridized to the other strand. Sticky ends are also called overhangs or jagged ends. Without intending to be bound by any particular theory, it is thought that the jagged ends may be related to how cell-free DNA fragments. For example, DNA may fragment in stages, and the size of the jagged end may reflect the stage of fragmentation. The number of jagged ends and/or the size of an overhang in a jagged end may be used to analyze a biological sample with cell-free DNA and provide information of about the sample and/or the individual from which the sample is obtained.

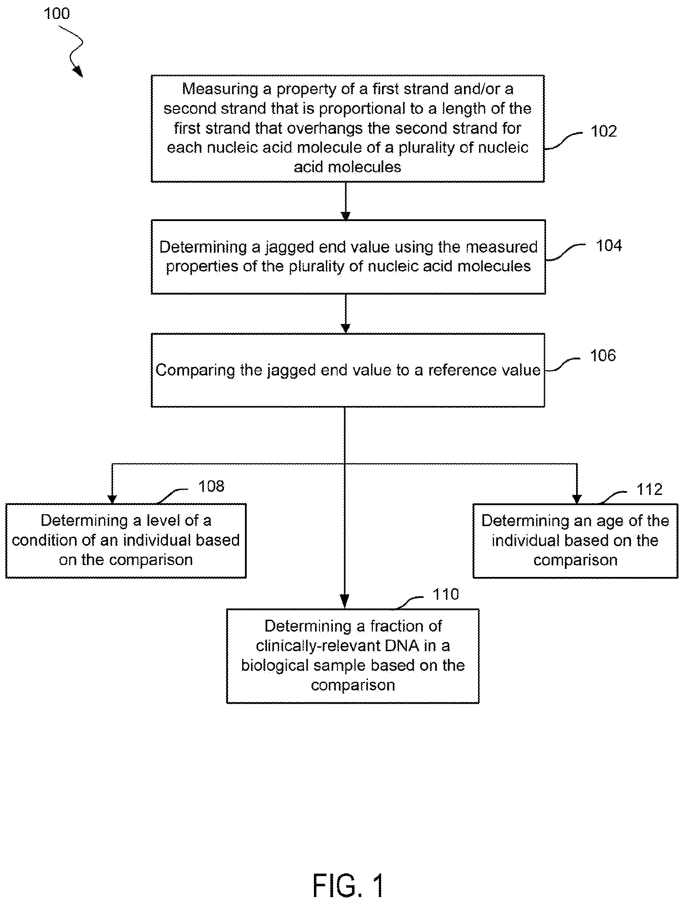

**FIG. 1** shows a method 100 using jagged end values to analyze a biological sample. The biological sample may be obtained from an individual. The biological sample may include a plurality of nucleic acid molecules, which are cell-free. Each nucleic acid molecule of the plurality of nucleic acid molecules may be double-stranded with a first strand having a first portion and a second strand. The first portion of the first strand of at least some of the plurality of nucleic acid molecules may overhang the second strand, may not be hybridized to the second strand, and may be at a first end of the first strand. The first end may be a 3' end or a 5' end.

At block 102, method 100 may include measuring a property of a first strand and/or a second strand that is proportional to a length of the first strand that overhangs the second strand. The property may be measured for each nucleic acid of a plurality of nucleic acids. The property may be measured by any technique described herein.

The property may be a methylation status at one or more sites at end portions of the first and/or second strands of each of the plurality of nucleic acid molecules. The jagged end value may include a methylation level over the plurality of nucleic acid molecules at one or more sites of end portions of the first and/or second strands.

In some embodiments, method 100 may include measuring sizes of nucleic acid molecules. The plurality of nucleic acid molecules may have sizes within a specified range. The specified range may be from 140 to 160 bp, any range less than the entire range of sizes present in the biological sample, or any range described herein. The size range may be based on the size of the shorter strand or the longer strand. The size range may be based on the outermost nucleotides of molecules after end repair. If the 5' end protrudes, then 5' to 3' polymerase mediated elongation will occur and the size may be the longer strand. If the 3' end protrudes, without a DNA polymerase with a 3' to 5' synthesis function, the 3' protruded single-strand may be trimmed and the size may then be the shorter strand.

In embodiments, method 100 may include analyzing nucleic acid molecules to produce reads. The reads may be aligned to a reference genome. The plurality of nucleic acid molecules may be reads within a certain distance range relative to a transcription start site.

At block 104, the jagged end value using the measured properties of the plurality of nucleic acid molecules may be determined.

If the first plurality of nucleic acid molecules are in a specified size range, methods may include measuring the property of each nucleic acid molecule of a second plurality of nucleic acid molecules. The second plurality of nucleic acid molecules may have sizes with a second specified size range. Determining the jagged end value may include calculating a ratio using the measured properties of the first plurality of nucleic acid molecules and the measured properties of the second plurality of nucleic acid molecules. The jagged end value may include the jagged end ratio or the overhang index ratio described herein.

At block 106, the jagged end value may be compared to a reference value. The reference value or the comparison may be determined using machine learning with training data sets.

The comparison may be used to determine different information regarding the biological sample or the individual. In embodiments, the comparison may include at least one of block 108, 110, or 112.

At block 108, a level of a condition of an individual may be determined based on the comparison. The condition may include a disease, a disorder, or a pregnancy. The condition may be cancer, an auto-immune disease, a pregnancy-related condition, or any condition described herein. As examples, cancer may include hepatocellular carcinoma (HCC), colorectal cancer (CRC), leukemia, lung cancer, or throat cancer. The auto-immune disease may include systemic lupus erythematosus (SLE). Various data below provides examples for determined a levels of a condition.

When block 108 is implemented, the reference value can be determined using one or more reference samples of subjects that have the condition. As another example, the reference value is determined using one or more reference samples of subjects that do not have the condition. Multiple reference values can be determined from the reference samples, potentially with the different reference values distinguishing between different levels of the condition.

In some embodiments, the comparison to the reference can involve a machine learning model, e.g., trained using supervised learning. The jagged end values (and potentially other criteria, such as copy number, size of DNA fragments, and methylation levels) and the known conditions of training subjects from whom training samples were obtained can form a training data set. The parameters of the machine learning model can be optimized based on the training set to provide an optimized accuracy in classifying the level of the condition. Example machine learning models include neural networks, decision trees, clustering, and support vector machines.

At block 110, a fraction of clinically-relevant DNA in a biological sample may be determined based on the comparison. Clinically-relevant DNA may include fetal DNA, tumor-derived DNA, or transplant DNA. The reference value may be obtained using nucleic acid molecules from one or more reference subjects having a known fraction of clinically-relevant DNA. Methods for determining the fraction of clinically-relevant DNA may include treating the plurality of nucleic acid molecules by a protocol before measuring the property of the first strand and/or the second strand. The nucleic acid molecules from one or more reference subjects may be treated by the same protocol as the plurality of nucleic acid molecules having the property measured.

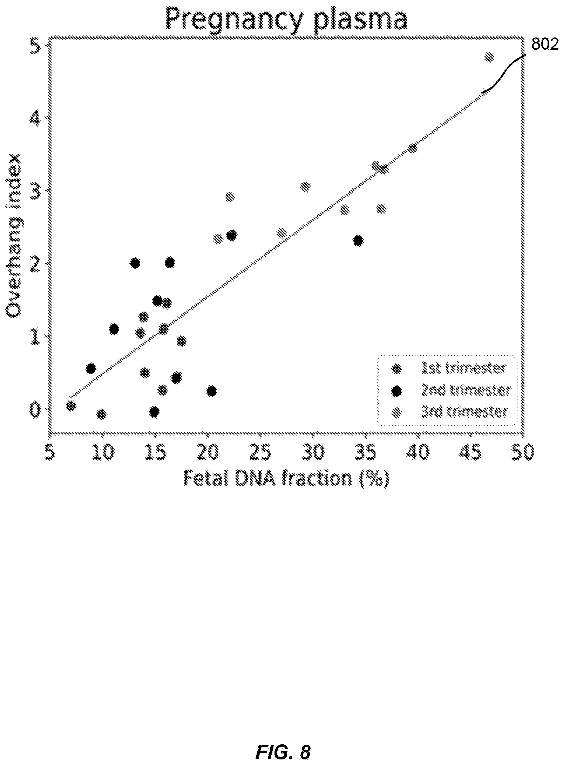

As described below, calibration data points can include a measured jagged end value and a measured/known fraction of the clinically-relevant DNA, e.g., as described for FIGS. 8, 11, 14, 27A, 30A, 30B, and 31. Such figures show calibration data points whose calibration values can be used as reference values to determine the fraction for a new sample. The measured jagged end value for any sample whose fraction is measured via another technique (e.g., using a tissue-specific allele) can be correspond to a reference value. As another example, a calibration curve (function) can be fit to the calibration data points, and the reference value can correspond to a point on the calibration curve. Thus, a measured jagged end value of a new sample can be input into the calibration function, which can output the faction of the clinically-relevant DNA.

As examples, the fractions of clinically-relevant DNA can be determined by a number of methods, for example but not limited to determining of the tissue-specific (e.g., fetal, tumor, or transplant) alleles in the sample, the quantification of targets on chromosome Y for male pregnancies, and the analysis of tissue-specific methylation markers. Using on this information, the clinically-relevant DNA fraction in the tested DNA sample (e.g., plasma or serum) can be determined based on the calibration curve, e.g., curve 802 in FIG. 8.

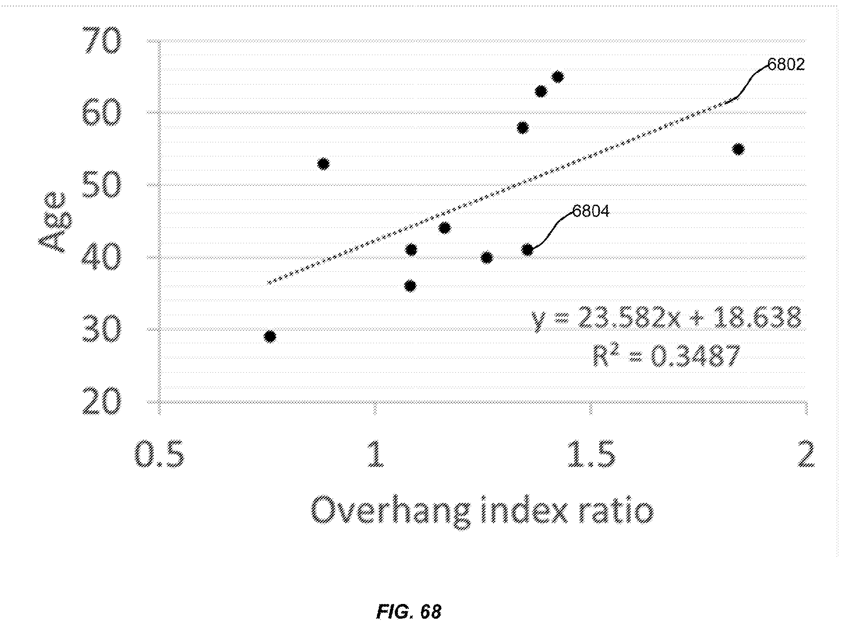

At block 112, an age of the individual may be determined based on the comparison. FIG. 68 shows such an example, where the calibration curve 6802 can be used to determine an age (e.g., a genetic age) of an individual using a jagged end value.

Methods related to blocks 108, 110, and 112 are described in more detail below.

### II. MEASURING JAGGED ENDS USING METHYLATION STATUS AFTER REPAIRING WITH UNMETHYLATED CYTOSINES

In the conventional library preparation protocols, normally the end repair of double-stranded DNA fragments will be performed before they are ligated with the universal adaptors. Such end repair will fill up sticky ends using DNA polymerase to form blunt ends. Such end repair can be conducted with adenines (As), guanines (Gs), thymines (Ts) and unmethylated cytosines (Cs). Therefore, in the traditional library preparation protocols, the overhang information cannot be reflected and traced from the ultimate sequencing results. The resulting lack of methylation in sections used to form blunt ends following end repair can be used to measure jagged ends.

### A. Determining methylation levels and jagged end values

In this patent application, one embodiment includes using sodium bisulfite to treat the end-repaired DNA molecules, and the newly filled-in unmethylated Cs would be converted Uracils (Us) that are amplified by PCR as Ts, while the original methylated Cs residing within the molecules remain unmodified. Therefore, after sequencing, because single-stranded DNA converted by sodium bisulfite cannot be paired to its complementary strand and bisulfite sequencing library produced in this way are strand-specific (namely Watson and Crick strand), the adjacent nucleotides close to 3' end (3' end adjacent nucleotides) of one strand DNA molecules will give rise to low methylation levels because of the filling of unmethylated Cs in gaps proximal to ends, in comparison to the adjacent nucleotides proximal to 5' end (5' end adjacent nucleotides) of the same strand. The adjacent nucleotides proximal to end would be defined by those nucleotides having relative distance to its said end of, but not limited to, 1, 2, 3, 4, 5, 6, 7, 8, 9, 10, 15, 20, 25, 30, 35, 40, 50 bases, or any range defined by any two of these numbers of bases. One embodiment for calculating the extent of the overhang in a DNA molecule is to determine the difference in methylation levels between 5' end adjacent nucleotides and 3' end adjacent nucleotides and such difference could be a ratio or subtraction.

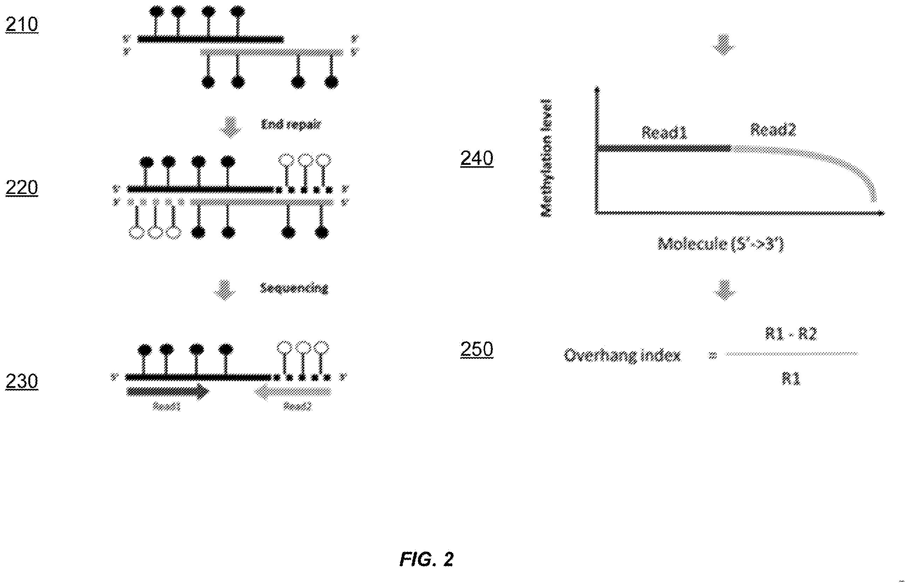

FIG. 2 illustrates one example showing how the degree of overhangs of cell-free DNA molecules (i.e. overhang index) can be deduced. Diagrams 210, 220, 230: Filled lollipops represent methylated CpG sites, and unfilled lollipops represent unmethylated CpG sites. Diagrams 220 and 230: Dash line represents newly filled-up nucleotides. Diagram 230: The red arrow is the first read (read 1) in sequencing results and the cyan arrow represents the secondary read (read 2). Graph 240: graph of methylation level in read1 and read2 from 5' to 3'. Equation 250: R1: the methylation level of read1. R2: the methylation level of read2.

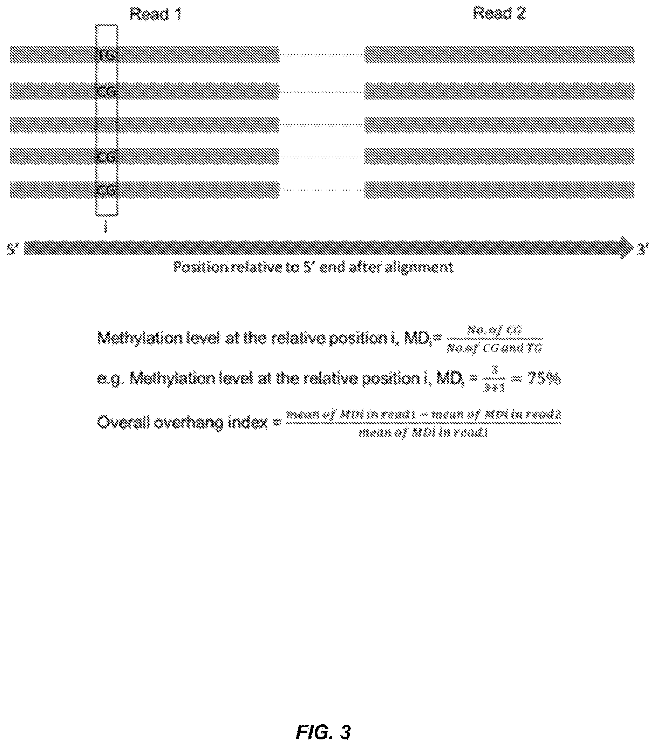

All DNA molecules from the Watson and Crick strand were stacked, respectively, according to relative positions and orientations after they were mapped to the human reference genome **(****FIG. 3****).** The stacked molecules were used for calculating the overall overhang index according to the positions relative to 5' end in the alignment results as shown in FIG. 3.

FIG. 3 is an illustration of the calculation of methylation levels along a DNA molecule after mapping to the human reference genome. The methylation level at a particular position i relative to the closest end (i.e. 5' end for read 1) was quantified by the ratio of the number of Cs to the total number of Cs and Ts. The first read (having 5' end, i.e. read 1) would have a higher averaged methylation level than the second read (having 3' end, i.e. read 2) because the 3' gaps in the second read would be filled in by unmethylated Cs which would be converted to Ts in bisulfite sequencing results.

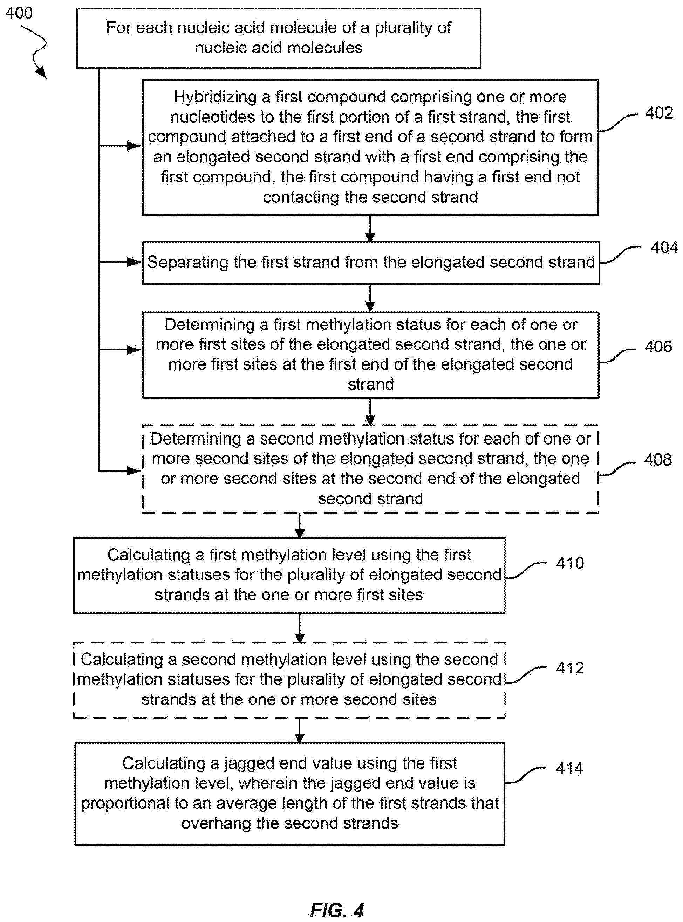

**FIG. 4** shows a method 400 of analyzing a biological sample obtained from an individual. The biological sample may include a plurality of nucleic acid molecules. The plurality of nucleic acid molecules may be cell-free. Each nucleic acid molecule of the plurality of nucleic acid molecules may be double-stranded with a first strand having a first portion and a second strand. The first portion of the first strand of at least some of the plurality of nucleic acid molecules may overhang the second strand, may not be hybridized to the second strand, and may be at a first end of the first strand.

At block 402, a first compound including one or more nucleotides may be hybridized to the first portion of the first strand for each nucleic acid molecule of the plurality of nucleic acid molecules. The first compound may be attached to a first end of the second strand to form an elongated second strand with a first end including the first compound. The first compound may include a first end not contacting the second strand. The one or more nucleotides may be unmethylated. In other implementations, certain nucleotides (e.g., cytosine) are all methylated, with the other nucleotides not being methylated. The first compound may be hybridized to the first portion one nucleotide at a time.

At block 404, the first strand may be separated from the elongated second strand for each nucleic acid molecule of the plurality of nucleic acid molecules.

At block 406, a first methylation status for each of one or more first sites of the elongated second strand may be determined for each nucleic acid molecule of the plurality of nucleic acid molecules. The one or more first sites may be at the first end of the elongated second strand.

At block 408, a second methylation status for each of one or more second sites of the elongated second strand may optionally be determined for each nucleic acid molecule of the plurality of nucleic acid molecules. The one or more second sites may be at the second end of the elongated second strand. The one or more second sites may include the outermost 30 sites at the second end of the elongated second strand. In some examples, the methylation status for the second sites may not need to be determined and may instead be assumed to be an average methylation status. The average methylation status may be known from a known frequency of methylated CpG sites in a particular region of the genome. In some instances, the average methylation status may be determined from reference samples taken from the same individual from which the biological sample is obtained and/or from other individuals.

At block 410, a first methylation level is calculated using the first methylation statuses for the plurality of elongated second strands at the one or more first sites. The first methylation level may be a mean or median of the first methylation statuses.

At block 412, a second methylation level may optionally be calculated using the second methylation statuses for the plurality of elongated second strands at the one or more second sites. The second methylation level may be a mean or median of the second methylation statuses. In some embodiments, the second methylation level may be assumed to be an average methylation level. The average methylation level may be based on a known frequency of methylated CpG sites in a particular region of the genome. In some instances, the average methylation level may be determined from reference samples taken from the same individual from which the biological sample is obtained and/or from other individuals. For example, the second methylation level may be assumed to be a value from 70% to 80%.

At block 414, a jagged end value using the first methylation level and the second methylation level may be calculated. A difference between the first methylation level and the second methylation level may be proportional to an average length of the first strands that overhang the second strands. Calculating the jagged end value may be by calculating a difference between the first methylation level and the second methylation level and dividing the difference by the first methylation level (e.g., overall overhang index in FIG. 3).

The jagged end value calculated in block 414 may be used in any of the methods described with FIG. 1.

### B. Jagged end differences in fetal and maternal DNA

Experiments show that measured jagged end values differ between fetal DNA and maternal DNA. As a result, jagged end values may be used to determine fetal DNA fraction and stage of pregnancy. The jagged end values may be determined through analysis of methylation levels or by any technique described herein. In addition, jagged end values may be used to determine fraction of other clinically-relevant DNA, such as cancer/tumor DNA or transplant DNA.

### C. Differential overhang index between sonicated tissue DNA and cell-free DNA fragments

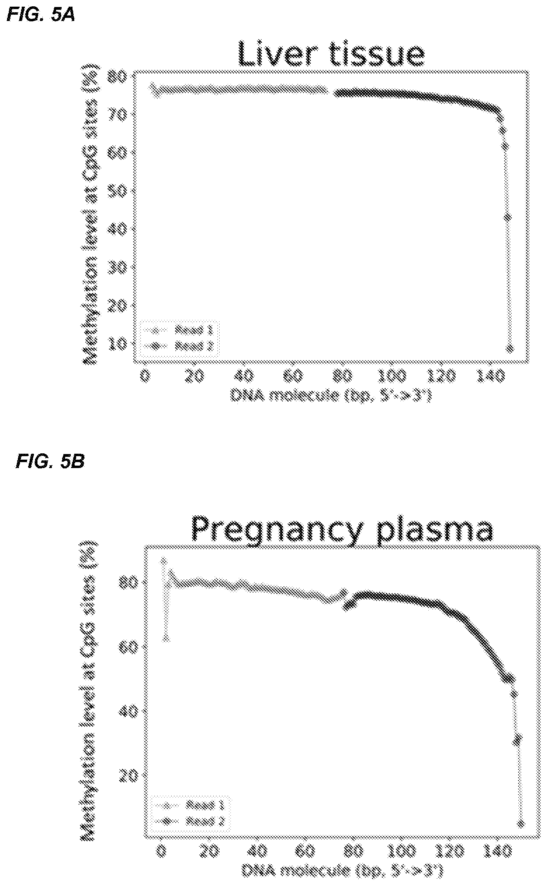

First, we analyzed 8 sonicated tissue DNA samples and 47 cell-free DNA samples from healthy subjects using massively pair-end bisulfite sequencing (75 bp x 2). A median of 132.9 million paired-end reads was achieved for each sample (range: 1.2-261.8 million). In FIGS. 5A and 5B, cell-free DNA turned out to bear longer 3' gaps indicating by the fact that the drop of methylation levels started at 120 bp (30 bp away from the 3' end) while sonicated DNA showed the drop of methylation levels beginning at 145 bp (only 5 bp away from the 3' end).

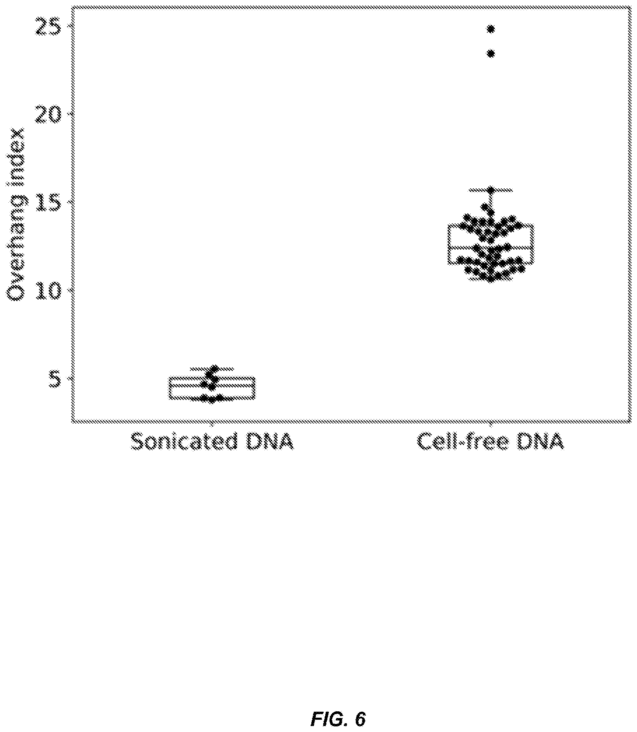

FIG. 6 shows boxplots for the difference in overhang indices between sonicated tissue DNA and cell-free DNA samples. The overhang indices of cell-free DNA samples were significantly higher than that of sonicated DNA samples (P-value < 0.0001, Mann-Whitney test), suggesting our new method can distinguish the ways how DNA would be cleaved by quantifying the overhang index.

### D. Differential overhang index between fetal and maternal DNA molecules

To assess the difference in overhang index between fetal and maternal DNA molecules respectively, we genotyped the maternal buffy coat and fetal samples using a microarray platform (Human Omni2.5, Illumina). We obtained peripheral blood samples from 10 pregnant women from each of the first (12-14 weeks), second (20-23 weeks), and third (38-40 weeks) trimesters and harvested the plasma and maternal buffy coat samples each case. Fetal samples were also obtained by chorionic villus sampling, amniocentesis, or sampling of the placenta. There was a median of 195,331 informative single nucleotide polymorphism loci (range: 146,428-202,800) for which the mother was homozygous and the fetus was heterozygous. There was a median of 190,706 informative single nucleotide polymorphism loci (range: 150,168 - 193,406) for which the mother was heterozygous and the fetus was homozygous. Plasma DNA molecules that carried the fetal-specific alleles were identified as derived from the fetus. Plasma DNA molecules that carried the maternal-specific alleles were identified as derived from the fetus. The median fetal DNA fraction among those samples was 17.1% (range: 7.0%-46.8%). A median of 103 million (range: 52-186 million) mapped paired-ended reads was obtained for each case. 92% of genome-wide CpGs were sequenced.

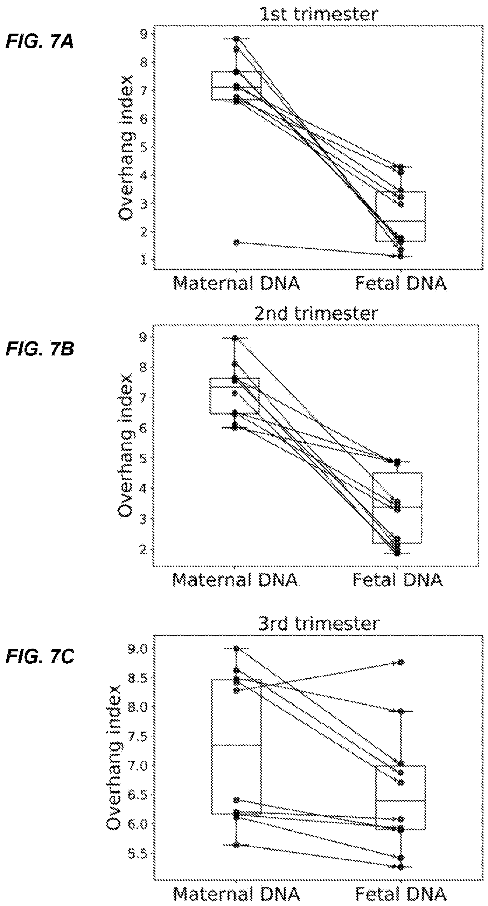

All the fetal DNA molecules from the Watson strand were stacked and used for calculating the overall overhang index as shown in FIG. 3. The averaged methylation levels at relative positions of read1 and read2 could be deduced by the ratio of the number of Cs to the total number of Cs and Ts sequenced at that particular position. The difference in averaged methylation levels between read1 and read2 (FIG. 3) could be used for indicating the overall overhang index in a sample because the end repairs would only occur in the read2. Similarly, all the maternal DNA molecules from the Watson strand were stacked and used for calculating the maternal overall overhang index according to sequencing cycles. As shown in the FIGS. 7A-7C, the overhang index of fetal DNA is significantly lower than that of maternal DNA in plasma of pregnant women in pregnant subjects of the first trimester (P-value = 0.005, Mann-Whitney test) (7A), second trimester (P-value = 0.005, Mann-Whitney test) (7B), and third trimester (P-value = 0.02, Mann-Whitney test) (7C), respectively. Furthermore, overhang indices of fetal DNA molecules were found to be correlated with fetal DNA fractions (FIG. 8, P-value <0.0001, r = 0.86). Such data suggested the overhangs of cell-free DNA molecules may bear the information of the tissue of origin.

### E. The size-banded overhang index analysis

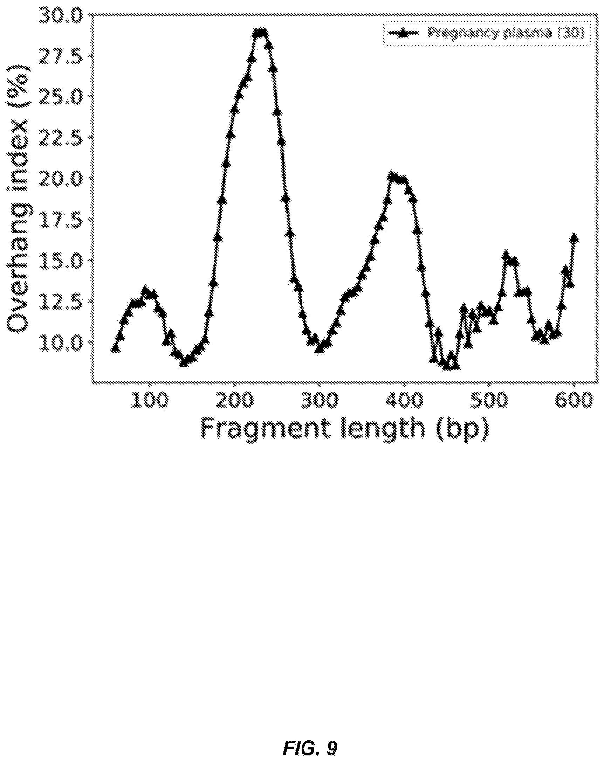

We further study the relationship between overhang indices and size ranges to be analyzed. It has been demonstrated that nonhematopoietically derived DNA is shorter than hematopoietically derived DNA in plasma (Zheng YW et al. Clin Chem. 2012;58:549-58). To visualize and study the relationship between overhang indices and fragment sizes, we pooled all sequenced fragments from 30 pregnant samples. Interestingly, the overhang index was unevenly distributed across the different size ranges being analysis (FIG. 9), showing wave-like and nonrandom patterns.

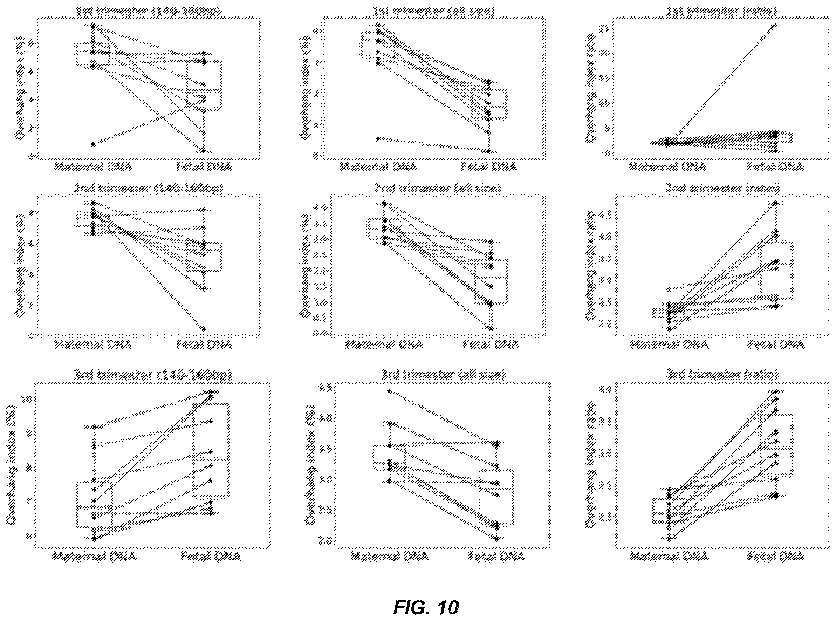

There were multiple major peaks of overhang index occurring at around 100 bp, 240 bp, 400 bp, and 560 bp, respectively. The distance between two adjacent major peaks in FIG. 9 was found to be around 160 bp, suggesting that such overhang indices might be related with nucleosome structures. The maximum of overhang index was present at around 230 bp. The unevenness of overhang index across different sizes may also suggest a particular size range might enhance the separation between samples with different clinical conditions. To shed light on this end, we partitioned the plasma DNA molecules into different size windows including but not limited to 80-100 bp, 100-120 bp, 120-140 bp, 140-160 bp, 160-180 bp, 180-200 bp, 200-220 bp, 220-240 bp, and 240-260 bp, and quantified overhang indices among different subjects. FIG. 10 showed the overhang index a representative size range of 140-160 bp across samples from different trimesters. The overhang index ratios of overhang index for those molecules with a size range 140-160 bp to all fragments were found to be significantly higher in fetal DNA molecules than that of maternal DNA molecules, suggesting that the short fetal DNA molecules would have relatively higher overhang abundance compared with the maternal DNA molecules within the same individual.

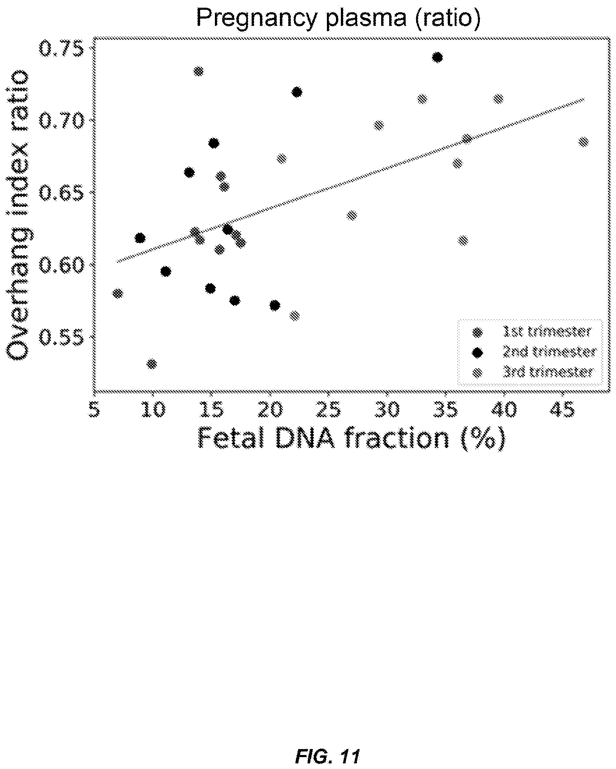

FIG. 11 indicated that the overall overhang index ratio of fragments including maternal and fetal DNA molecules correlated with the fetal DNA fraction (r=0.5, P=0.02), suggesting that the size-range based overhang index analysis would be used for informing the tissue of origin for plasma DNA molecules.

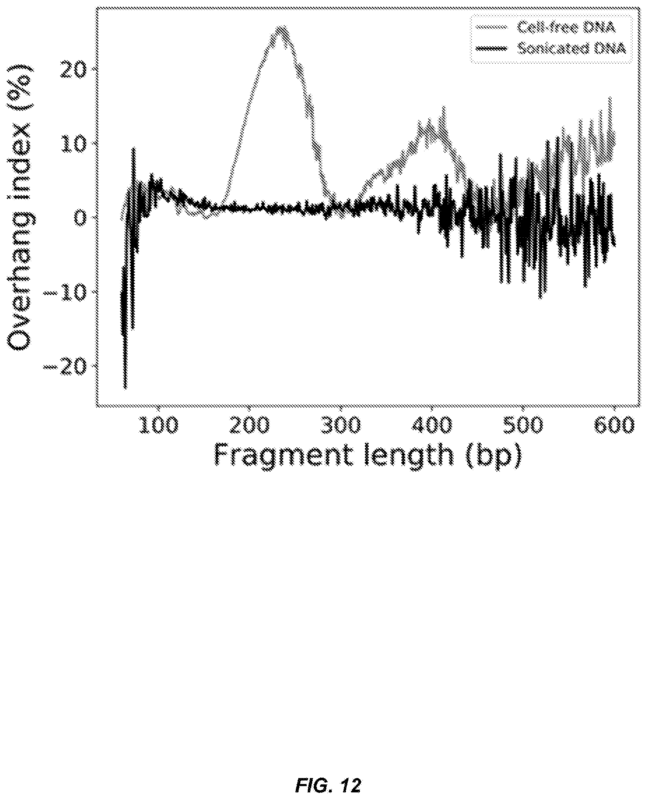

FIG. 12 shows a comparison of the overhang index across different size ranges between plasma DNA molecules and sonicated DNA.

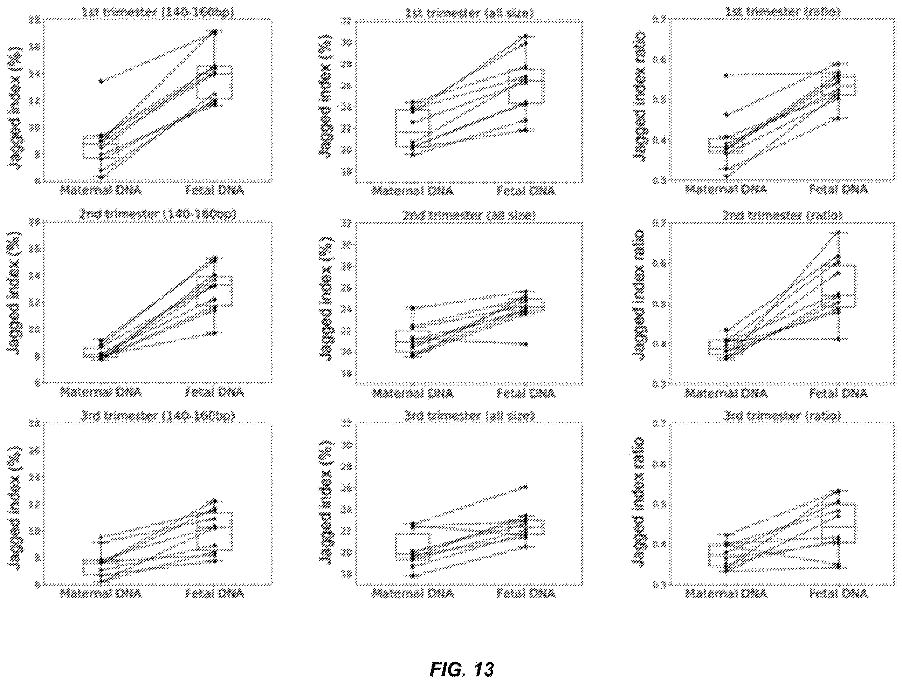

FIG. 13 shows additional results of the jagged index between fetal DNA maternal DNA across different trimesters. An experimental protocol with the use of mild clean-up conditions (MinElute PCR Purification Kit) was used to analyze the pregnant cases. In FIG. 10, the experimental protocol used GeneRead DNA FFPE Kit. The fetal DNA and maternal DNA molecules were identified by taking advantage of the genotypic difference between the fetal and maternal genomes. With these results, the fetal DNA molecules were found to carry more jagged ends because the jagged index of fetal DNA was significantly higher than that of maternal DNA. These results are different from FIG. 10, which showed that fetal DNA molecules were less likely to include jagged ends. However, the jagged index ratio for a size range of 140-160 bp of fetal DNA molecules was found to be higher than that of maternal DNA molecules. The jagged index ratio was consistent with the results in the third column of FIG. 10, which are based on another clean-up condition.

When determining the fractional concentration of clinically-relevant DNA using jagged ends, the same experimental protocol should be used for both the reference samples and the sample to be tested.

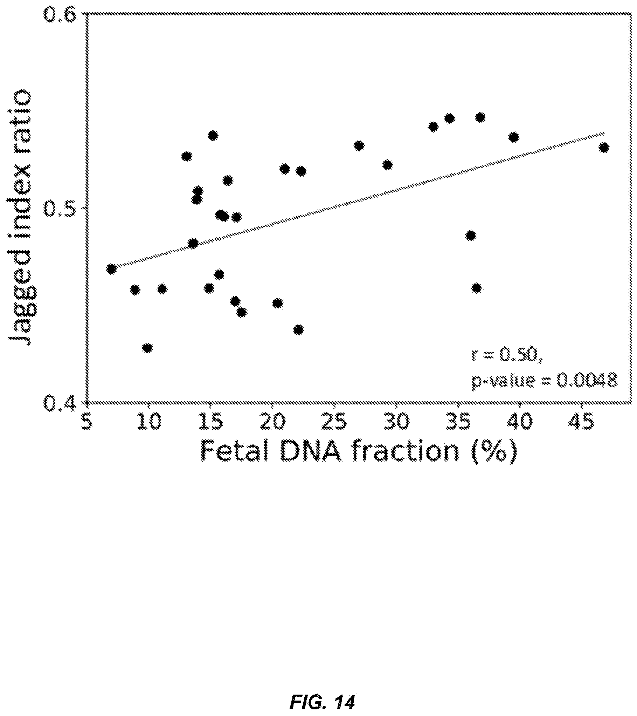

FIG. 14 shows the correlation between fetal DNA fraction and jagged end index ratio (r=0.5 and p-value=0.0048). FIG. 14 shows a correlation consistent with FIG. 11.

### III. MEASURING JAGGED ENDS USING METHYLATION STATUS AFTER REPAIRING WITH METHYLATED CYTOSINES

As discussed above, end repair can be conducted with adenines (As), guanines (Gs), thymines (Ts), and unmethylated cytosines (Cs). However, end repair can be modified to use methylated cytosines (mCs) in place of unmethylated cytosines. The resulting methylation in sections used to form blunt ends following end repair can be used to measure jagged ends. In addition, using methylated cytosines for end repair can also result in measuring the precise length of a jagged end or the identification of a blunt end.

### A. A principle for examining jagged ends of plasma DNA molecules

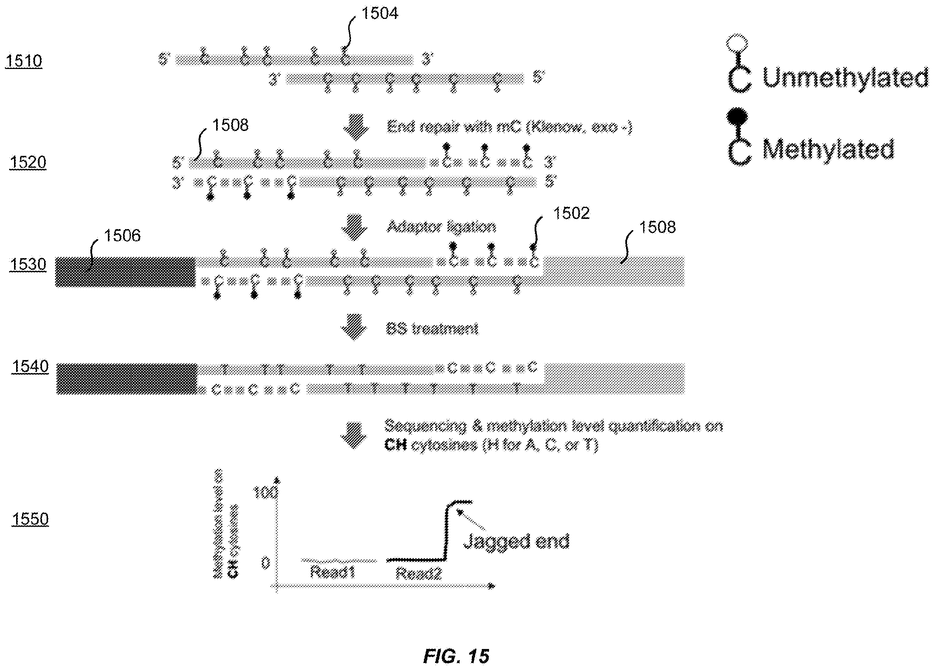

**FIG. 15** shows an approach for using -ribonucleoside triphosphates (dNTP), including dATP (A), dGTP (G), dTTP (T), and methylated dCTP (mC) instead of unmethylated dCTP (C), to fill up the jagged ends in order to form blunt ends during the end repair process in library preparation. In FIG. 15, filled lollipops (e.g., 1502) represented methylated cytosines (mCs), and the unfilled lollipops (e.g., 1504) represented unmethylated cytosines (Cs). In diagram 1510, a double-stranded DNA molecule with a jagged end is shown. The double-stranded DNA molecule includes unmethylated cytosines in both strands. The DNA molecule may include some CpG sites in the DNA molecule that may be methylated.

Diagram 1520 shows a DNA molecule after end repair with methylated cytosines. The dashed lines represented newly filled-up nucleotides. The cytosines of the newly filled up are methylated while the DNA molecule before end repair includes unmethylated cytosines. "Klenow, exo -" means that polymerase fragments retain polymerase activity but lack both 5' to 3' and 3' to 5' exonuclease activity. As a result, additional jagged ends are not introduced by exonuclease.

Diagram 1530 shows the end-repaired DNA molecule after ligating sequencing adaptors 1506 and 1508.

Diagram 1540 shows the DNA molecule after bisulfite treatment. After the bisulfite treatment, the newly filled-in methylated Cs in the end-repaired DNA molecules remained unchanged, whereas the original unmethylated Cs residing within the molecules were converted to Uracils (Us) that were subsequently amplified as Ts by PCR. The adjacent nucleotides close to the 3'end (3' end adjacent nucleotides) of a DNA molecule would show an increase of methylation levels because of the filling of mCs in gaps proximal to 3' ends, compared to the adjacent nucleotides proximal to the 5'end (5' end adjacent nucleotides) of the same molecule. Because the DNA molecule before end repair may have included methylated CpG sites, some Cs, besides the mCs added in the end repair, may remain as mCs after end repair. To account for these mCs, the analysis of Cs may be limited to CH (where H is A, C, or T) sites and exclude CpG sites. Since CH sites account for ~19.2% of dinucleotide contexts in the human genome, a substantial proportion of molecules with jagged ends could be detected.

Diagram 1550 shows a graph of the methylation level of CH cytosines across two reads. Diagram 1550 is similar to graph 240, with the x-axis of diagram 1550 may going from 5' to 3'. The methylation level of read 1 is near 0 for CH cytosines. Read 1 corresponds to the 5' end of top strand 1508 in diagrams 1510-1540. The methylation level of read 2 is near 0 until close to the 3' end, when the methylation level nears 100. The increased methylation level is a result of the methylated cytosines (e.g., 1502) in the nucleotides provided in end repair.

The increased methylation level can be correlated with the jagged end. The length of the jagged end can be determined from the increase in the methylation level. The length of the jagged end can also be determined by analyzing where thymines and methylated cytosines appear after bisulfite treatment.

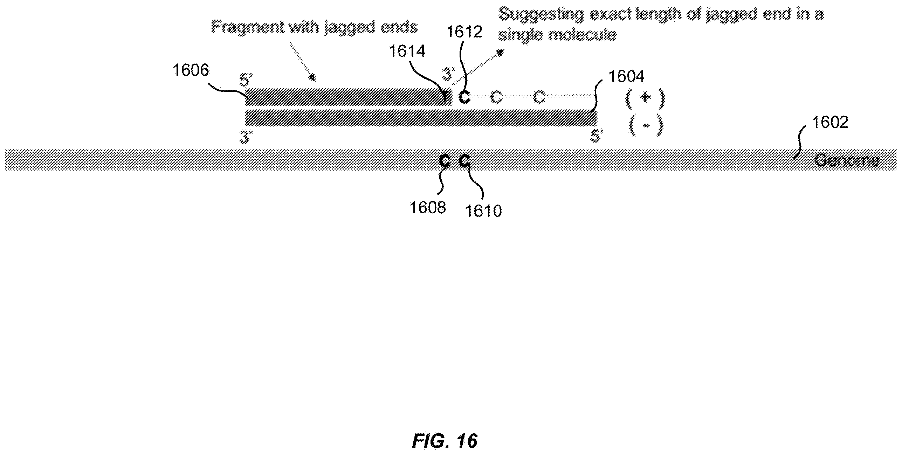

**FIG. 16** show how this approach using methylated cytosines for end repair enables accurately deducing the exact length of a jagged end. Genome 1602 shows that there are two consecutive Cs. A DNA fragment with a jagged end has a first strand 1604 and a second strand 1606. Genome 1602 may be the sequence of second strand 206. Cytosine 1608 may be at the 3' end of first strand 1606. Cytosine 1610 may be added to the 3' end of first strand 1606 with end repair. With the use of methylated cytosines in end repair, this cytosine is methylated cytosine 1612. In this configuration, this "CC" tag in the genome would be converted into a "TC" pattern in the sequencing results. The unmethylated cytosine, corresponding to cytosine 1608, would be converted to thymine 1614 with bisulfite treatment. Methylated cytosine 1612, corresponding to cytosine 1610, remains methylated cytosine. By using this "TC" pattern, we can exactly determine the jagged end length. We refer to this technique as a "CC-tag" strategy.

While consecutive CCs may be analyzed to determine the exact jagged end length, nonconsecutive CCs may also be informative in determining the jagged end length. For example, CC may be separated by several nucleotides that are not C. If one C converts to T and the other remains C, then a range for the jagged end length can be determined. The maximum length of the jagged end can be deduced by the position of the T, and the minimum length of the jagged end can be deduced by the position of the C nearest the T on the 3' end.

### B. Spike-in sequences with known jagged ends

Nucleic acid molecules having a known jagged end length with a known sequence can be used in end repair to verify results using end repair with methylated cytosines. These known sequences (i.e., spike-in sequences) can also be used to determine a quantity (e.g., a concentration, a molar quantity) of jagged ends.

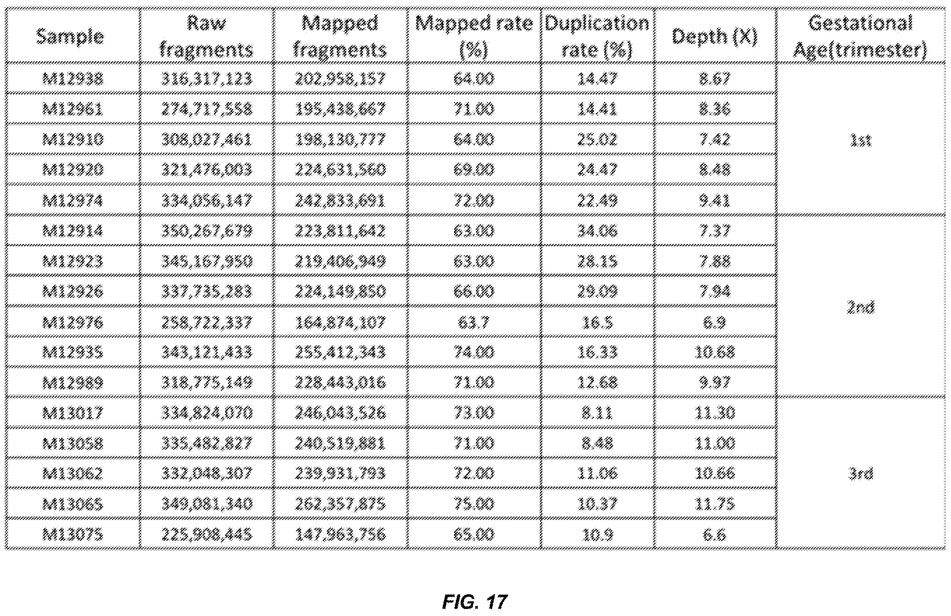

**FIG. 17** shows a table of 16 plasma DNA samples analyzed using end repair with methylated cytosines. We analyzed 16 plasma DNA samples from the first (12-14 weeks), second (20-23 weeks), and third (38-40 weeks) trimesters using massively paired-end bisulfite sequencing (75 bp x 2). A median of 206.9 million paired-end reads was achieved for each sample (range: 148.0-262.4 million). "Sample" refers to the identification of the sample. "Raw fragments" refers to the number of fragments sequenced. "Mapped fragments" represents the number of the fragments that can be mapped. "Mapped rate" is the percentage of the raw fragments that are mapped. "Duplication rate" is the percentage of DNA fragments that would be removed through the process in which all but one duplicated fragment with the identical start and end mapping genomic coordinates was filtered. "Gestational age (trimester)" is the trimester of the pregnancy of the female from which the sample is taken.

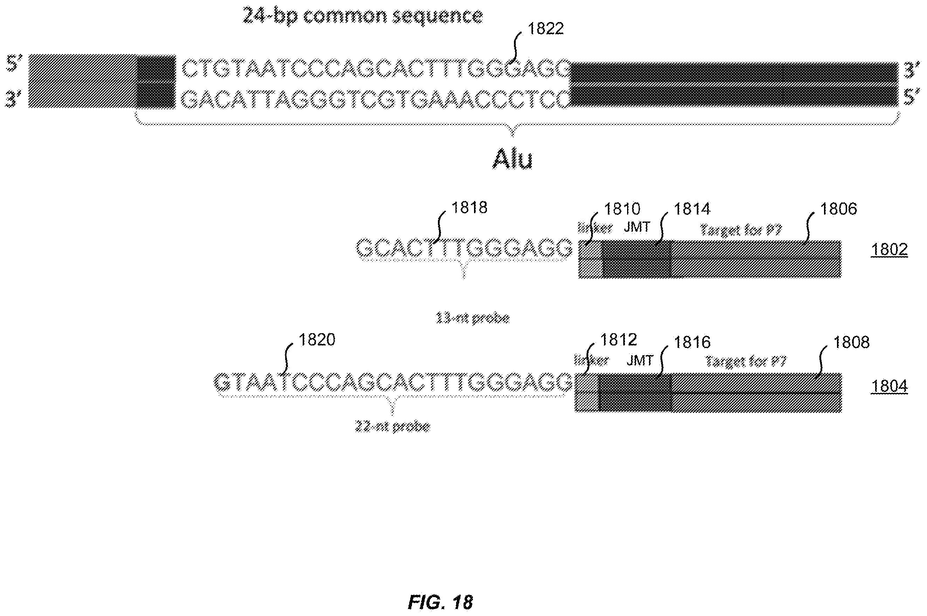

**FIG. 18** shows the use of two synthetic double-stranded DNA fragments 1802 and 1804 with jagged ends of known lengths as internal controls. These internal controls can verify that the use of methylated cytosines is effective in analyzing jagged ends. Each of the two double-stranded synthetic DNA consisted of a target sequence for P7 (annealing sites for a sequencing adaptor, Illumina) (target sequences 1806 and 1808), a linker DNA (1810 and 1812), a jagged end molecular tag (JMT) (1814 and 1816). Double-stranded DNA fragment 1802 includes 13-nt probe 1818, and double-stranded DNA fragment 1804 includes 22-nt probe 1820. The 13-nt and 22-nt single-stranded fragments are subsequences of the 24-bp common sequence of Alu 1822. The 13-nt and 22-nt fragments 1818 and 1820 are showed as examples. Other lengths of the common sequence may be used as controls. JMT 1814 and 1816 are each a string of 6 nucleotides that allow one to differentiate the synthetic DNA control with 13-nt jagged end from the synthetic DNA control with 22-nt jagged end.

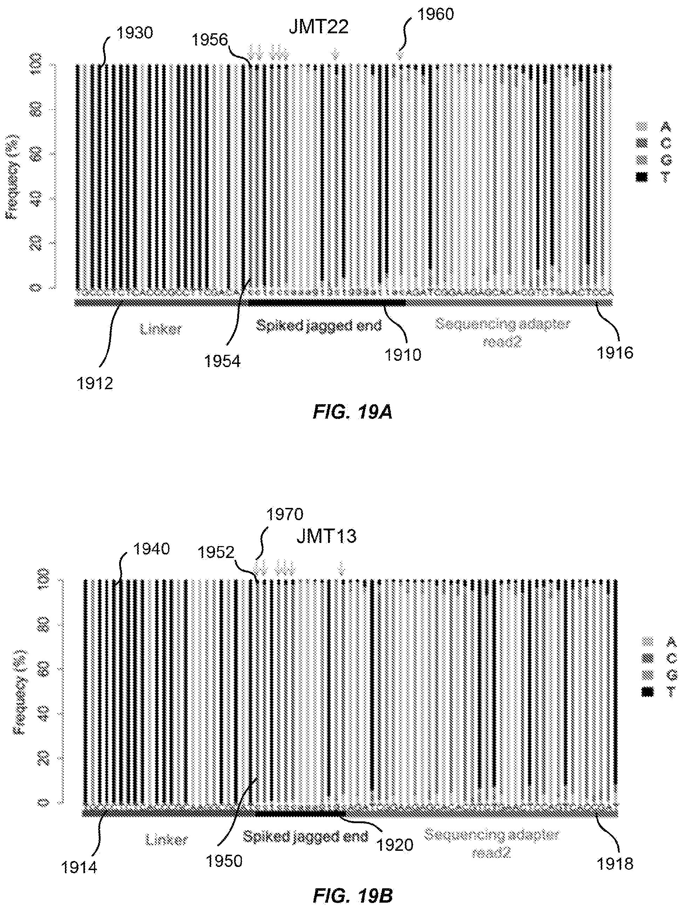

**FIGS. 19A** and **19B** show sequencing base compositions for two spike-in sequences with known jagged ends having known sequences. Synthetic double-stranded DNA fragments are used, similar to those fragments in FIG. 18. FIG. 19A shows using a 22-nt known spike-in sequence and FIG. 19B shows using a 13-nt known spike-in sequence, with both sequences complementary to jagged ends and having methylated cytosines. The horizontal orange bars (1910 and 1920) in the x-axis indicate the presence of jagged ends in the spike-in sequences. The horizontal dark blue bars 1912 and 1914 represent linkers similar to linkers 1810 and 1812. These linkers do not have methylated cytosines. The horizontal light blue bars 1916 and 1918 are sequencing adapters. The sequencing adapters may also be methylated. The vertical bars, colored with green, blue, gray, and red, represent the frequencies of A, C, G, and T, respectively. For example, vertical bars 1930 and 1940 indicate T. Some vertical bars have multiple colors, with each color representing percentage of that base.

Vertical bar 1950 and vertical bar 1954 both correspond to a methylated cytosine in the spiked jagged end. The methylated cytosine is sequenced as a cytosine, as indicated by vertical bar 1950 and vertical bar 1954 both indicating C. The arrows (e.g., 1960 and 1970) represent the filling of methylated cytosines (mCs) in jagged ends. On top of vertical bar 1950 is vertical bar 1952, which indicates T. On top of vertical bar 1954 is vertical bar 1956, which indicates T. These indications of T may be the result of sequencing error, as the percentage of T is low.

We observed all the cytosines within the jagged end (denoted in lowercase letters) were unchanged because of the incorporation of mCs during the end-repair step. By contrast, unmethylated Cs within double strand (as shown in the linker region in capital letters) were nearly all converted to Ts. The results suggest high efficiency of bisulfite conversion for nucleotides within double-stranded DNA as well as the successful incorporation of mCs in jagged ends.

Including a known quantity of molecules with a known extent of jagged ends can allow the determination of the actual quantities of the other jagged end species originally present in the sample. For example, if samples are tested with and without adding the spiked-in jagged ends, the percentage of jagged end species for the spiked in species would be higher in the test with the added spiked-in jagged ends than without. Because we know the spiked-in amount and the resultant percentage increase, the quantities (e.g. , concentration, molar amount) of the other species of jagged ends in the sample can be determined.

### C. Determination of plasma DNA jagged ends

The methylation levels resulting from using methylated cytsosines for end repair can be compared to methylation levels resulting from using unmethylated cytosines for end repair. The effectiveness of both approaches can be compared.

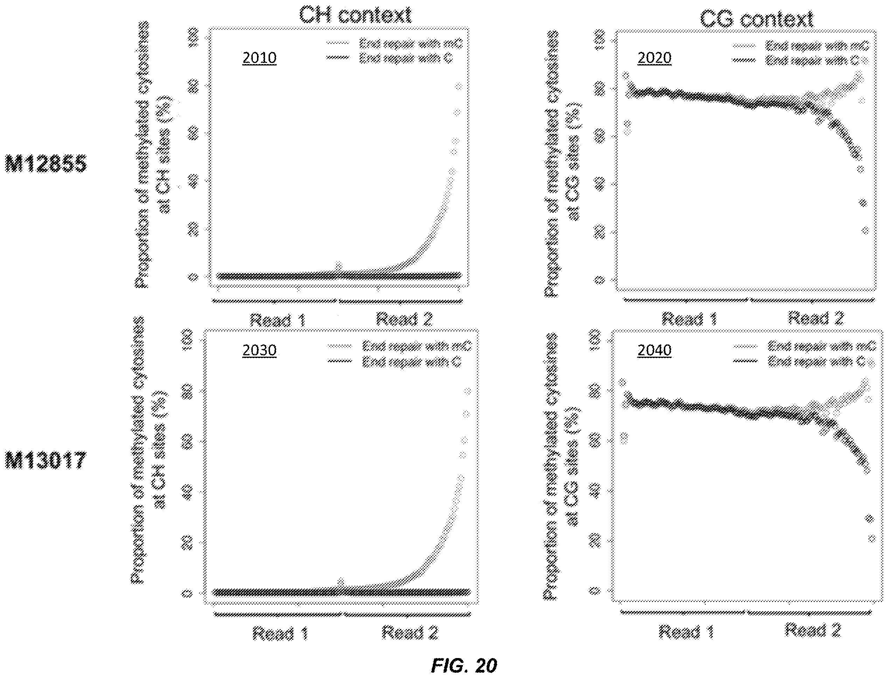

**FIG. 20** shows representative plots for the proportion of methylated cytosines in plasma DNA of pregnant women at CH and CG contexts in order to validate the approach of using methylated cytosines for end repair. We end-repaired two aliquots for each sample (cases M12855 and M13017) using both methylated Cs (i.e. mCs) and unmethylated Cs (i.e. Cs) for each case during the library preparation, respectively. We analyzed the proportion of methylation levels in both the CH and CG dinucleotide contexts of the human genome. Those CH sites, meaning dinucleotides that are NOT CpGs, in the human genome were reported to exhibit very low methylation levels in general, approximately 0% (Hyun Sik Jang et al. Gene 8(6):2-20). For the samples end-repaired with mCs, the proportion of methylated cytosines in the context of CH was observed to be close to 0% in the 5' end of a molecule (read 1) for all samples regardless of whether they were end-repaired with mCs or Cs (Graphs 2010 and 2030).

This observation indicated that such 5' part of the cell-free DNA molecules were double-stranded in nature, and there was very little incorporation of the dNTPs as a result of end repair. On the contrary, the proportion of methylated cytosines rapidly increased up to 80% along the 3' direction from the position of 25 bp in the read 2 sequences of cell-free DNA molecules. Read 2 sequences correspond to their 3' ends (Graphs 2010 and 2030). These data indicated that jagged ends were present toward the 3' end of cell-free DNA molecules because there was an increase in mC incorporation as a result of end repair. In contrast, the proportion of methylated cytosines at CH sites remained close to 0% for the samples end-repaired with Cs (Graphs 2010 and 2030) because the newly incorporated unmethylated Cs during end repair will not elevate the methylation level of the molecules where the baseline level of methylation at the CH dinucleotide sites was ~ 0%. In summary, mC incorporation interpreted in the CH dinucleotide context result in an increase in methylated cytosines and thereby revealed the presence of jagged ends in plasma DNA or cell-free DNA.

For the CG context, also termed CpG dinucleotides, we observed a high proportion of methylated Cs in the 5' end of a molecule (i.e. read 1), which was largely consistent with a previous study in which the methylation level on CpG sites was approximately 80% in the human genome (Hyun Sik Jang et al. Gene 8(6):2-20). The proportion of methylated cytosines gradually rose up to almost 100% along the 3' direction from the position of 25 bp in the read 2, suggesting the incorporation of mCs along the plasma DNA jagged ends during the end repair (Graphs A520 and A540). This observation was related to the incorporation of mCs to fill in the jagged end during the end-repair process, elevating the background methylation of 80% at CpGs to 100% by the *in vitro* process of end repair. In addition, there was a significant decrease in the proportion of methylated cytosines across the corresponding positions of the read 2 when we used unmethylated Cs for the end-repair process (Graphs A520 and A540). These data revealed the presence of jagged ends because the generally hypermethylated CpGs are replaced by unmethylated Cs during the *in vitro* end-repair process. Methylated cytosines could be used in the CG context to determine jagged ends, though because of the background methylation level of about 80%, the sensitivity of such a technique would be limited.

These results revealed that the approach of repairing with methylated cytosines instead of unmethylated cytosines allowed us to detect jagged ends. The approach utilizing the filling of mCs during the end-repair process in library preparation, thus allowing for jagged end analysis in the context of CH, may greatly improve the resolution in jagged end analysis. Such CH sites in the human genome are much more prevalent than CG sites (271 million CH sites versus 28 million CG sites).

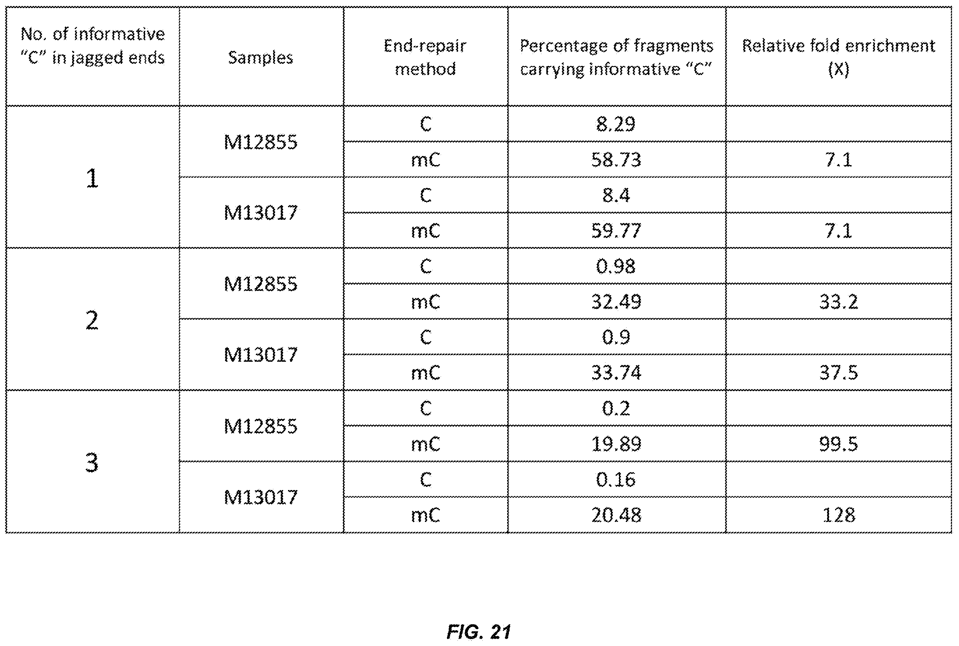

**FIG. 21** shows the relative informativeness comparison between approaches using the filling methylated cytosines (mCs) and unmethylated cytosines (Cs). "No. of informative 'C' in jagged ends" is the number of cytosines in the jagged end that are either methylated when using the methylated cytosine approach or unmethyalted when using the unmethylated cytosine approach. "Samples" refers to the identification of the sample. "End-repair method" refers to the type of cytosines used in end repair. "C" indicates unmethylated cytosines, and "mC" indicates methylated cytosines. "Percentage of fragments carrying informative 'C‴ is the percentage of DNA fragments in the sample that have either an unmethylated C or a methylated C, depending upon the end-repair method. "Relative fold enrichment (X)" is the ratio of the percentage of fragments carrying mC in the methylated cytosine approach over the the percentage of fragments carrying C in the unmethylated cytosine approach. As shown in the table in FIG. 21, we analyzed the percentage of fragments carrying cytosines that could be inferred to be associated with jagged ends (i.e. informative "C" in jagged ends). We observed that the method using the filling of methylated cytosines could detect a much higher proportion of fragments carrying jagged ends.

For example, when considering at least one informative "C" in jagged ends for a molecule, there were 58.73% of fragments that could be inferred to be associated with jagged ends by the method with the filling of mCs, which was much higher than that inferred by the method with the filling of Cs (8.29%). In other words, the method with the filling of mCs could enrich 7.1-fold more information than the method with the filling of unmethylated Cs. When considering at least two informative "C" in jagged ends, the method with the filling of mCs could enrich greater than 30-fold more information than the method with the filling of unmethylated Cs. Filling in with unmethylated Cs restricts informative Cs to CG sites, while filling in with methylated Cs allows for the more prevalent CH sites to include the informative Cs.

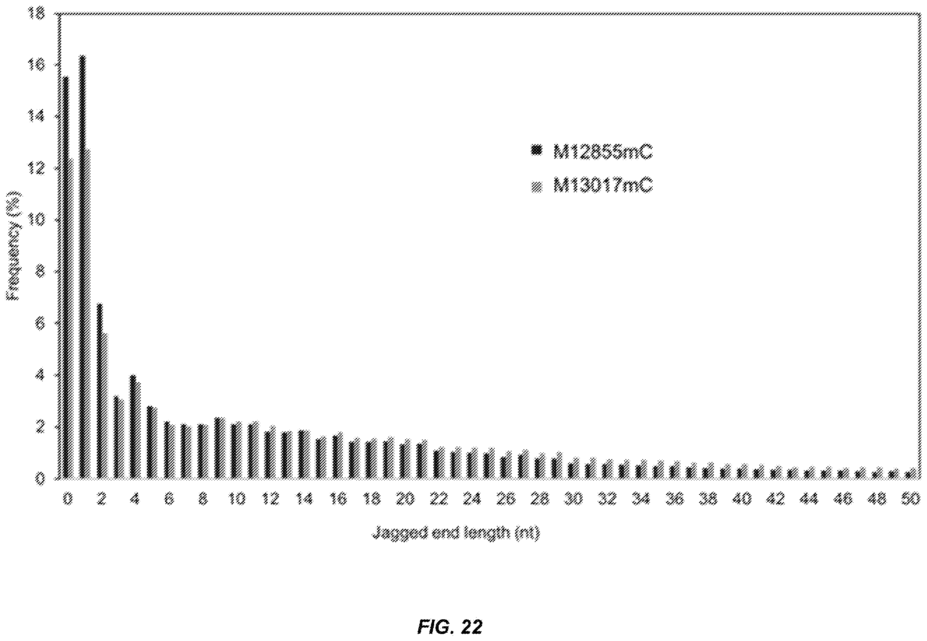

**FIG. 22** shows the distribution of jagged end lengths deduced by the "CC-tag" strategy. The "CC-tag" approach offers the possibility to measure jagged ends at single-base resolution. Using this approach, FIG. 22 reveals that the jagged ends with 1-4 bp in length were much more abundant (~25%) among the pool of the jagged ends, and jagged ends with 1 bp appeared to be most frequent. Generally, the longer the jagged end, the lower the relative frequency would be seen in plasma DNA or cell-free DNA. With the use of the "CC-tag" approach, we could also determine the number of molecules with blunt ends (i.e. jagged end with 0 bp in size). The proportion of molecules with blunt ends ranged from 12.4% to 15.5%.

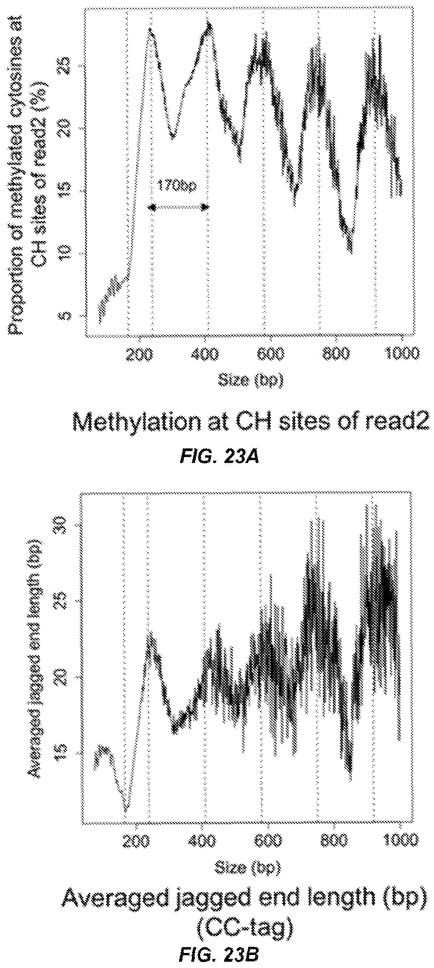

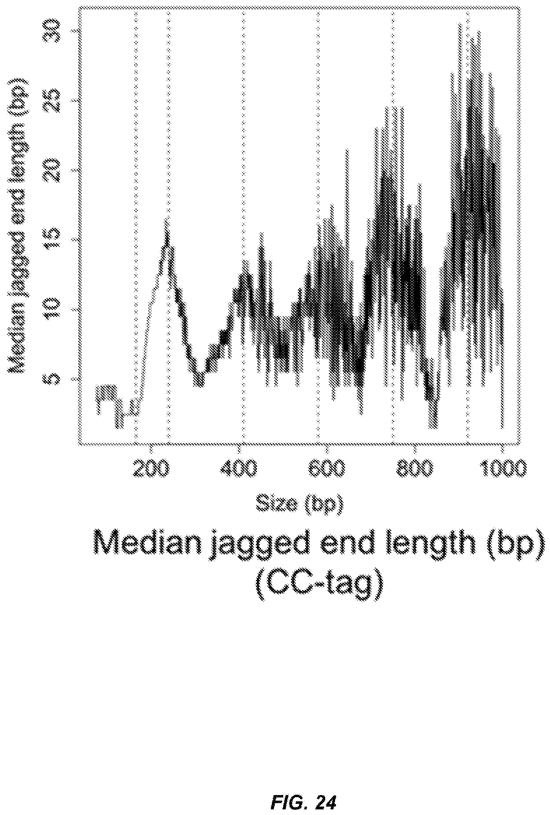

**FIGS. 23A,** 23B, and 24 show the profile of jagged ends across different size ranges of cell-free DNA fragments. FIG. 23A analyzes methylation levels of CH dinucleotides, as in the technique of FIG. 15. FIGS. 23B and 24 use the CC-tag approach of FIG. 16. In FIG. 23A, the vertical axis is the proportion of methylated cytosines among CH dinucelotides in read 2 sequences, reflecting methylated cytosines near the 3' end of the molecules and indicating jagged ends. The higher the methylated "CH" cytosine level in read 2 signifies a higher degree of jagged ends in DNA molecules, which could be due to (1) molecules with longer jagged ends and/or (2) increased number of molecules carrying jagged ends. The horizontal axis is the size of the DNA fragments whose average proportion is measured. Accordingly, we analyzed the relationship between the proportions of methylated cytosines among CH dinucelotides in read 2 sequences, namely 3' ends of the plasma or cell-free DNA molecules where the jagged ends are located, across different cell-free DNA sizes.

**FIG. 23A** shows the proportion of methylation levels at CH sites of read 2 across different size ranges. The higher the methylation levels, the more jagged ends would be expected. As shown in FIG. 23A, the methylation levels were unevenly distributed across different size ranges, exhibiting wave-like nonrandom patterns. When the size was smaller than 160 bp, the methylation level was lower than 10%. The methylation level continuously increased when the fragment size was larger than 160 bp and reached to a peaked value of ~ 28% at 240 bp. The increase in methylation level suggests a higher degree of jagged ends from longer jagged ends or more molecules with jagged ends. The distance between two consecutive major peaks of methylation level was found to be ~170 bp, which was highly consistent with nucleosomal phasing patterns and reminiscent of the distance between nucleosomes. This may suggest that the jagged end could be affected by chromatin structures. The chromatin structure may increase degradation, leading to jagged ends.

**FIG. 23B** shows the average jagged end length across different size ranges based on "CC-tag" approach. The vertical axis shows the average jagged end length. The horizontal axis is the size of the DNA fragments whose jagged length end length is measured. In FIG. 23A, the proportion of methylation levels at CH sites may result from at least one of length and amount of jagged ends. In contrast, in FIG. 23B, the exact length of the jagged ends are determined using the CC-tag method. In general, the higher the methylation level in FIG. 23A, the longer length deduced by the CC-tag method in FIG. 23A.

**FIG. 24** shows the median jagged end length across different size ranges based on "CC-tag" approach. The average and median jagged end length gave rise to similar patterns to the proportion of methylated cytosines at CH sites proximal to the 3' end of a molecule. The wave-like signals of jagged-end length is reminiscent of nucleosome structures. Chromatin structures may therefore play a role in the length of jagged ends.

### D. Differential jagged ends between the fetal and maternal DNA molecules

To evaluate if the jagged end has different characteristics between the cell-free maternal and cell-free fetal DNA molecules in maternal plasma (e.g. whether the jagged end is feasible to inform tissues of origin), we genotyped the maternal buffy coat and fetal tissue samples using a microarray platform (Human Omni2.5, Illumina).

Fetal samples were also obtained by chorionic villus sampling, amniocentesis, or sampling of placenta, depending on which type of tissue DNA samples was available. There was a median of 201,352 informative single nucleotide polymorphism (SNP) loci (range: 178,623-208,552) for which the mother was homozygous and the fetus was heterozygous. Plasma DNA molecules that carried the fetal-specific alleles were identified as derived from the fetus.

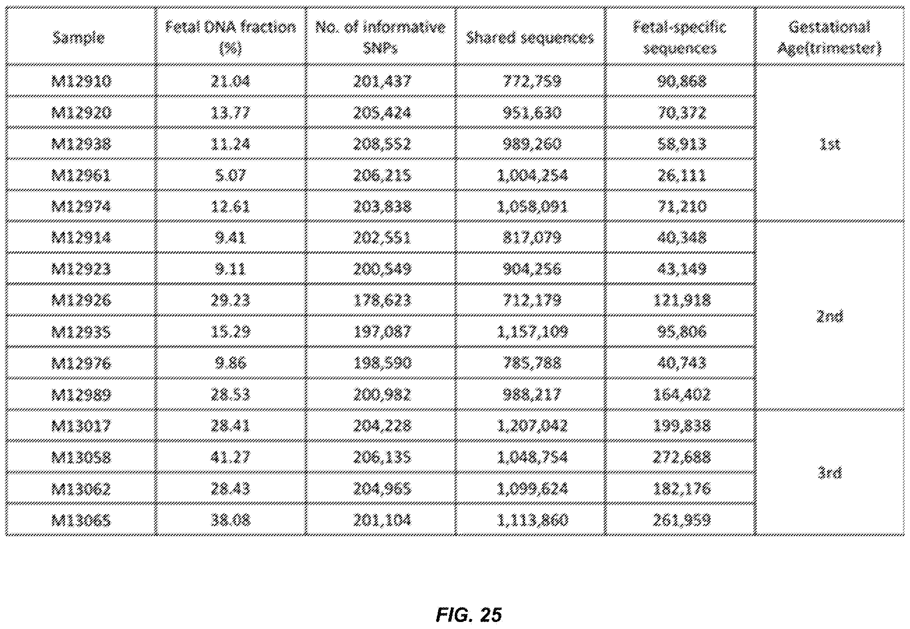

**FIG. 25** shows a table with sequencing information and fetal DNA fractions for different pregnant women. "Sample" refers to the identification of the sample. "Fetal DNA fraction (%)" is the percentage of DNA fragments in the sample that are fetal-derived. "No. of informative SNPs" is the number of SNPs for which the mother is homozygous and the fetus is heterozygous determined by microarray-based SNP genotyping. "Shared sequences" is the number of DNA fragments having alleles common to both the fetus and the pregnant female. "Fetal-specific sequences" is the number of DNA fragments with alleles that are present only in the fetus. The median fetal DNA fraction among those samples was 20.1% (range: 5.1%-41.3%). "Gestational age (trimester)" is the trimester of the pregnancy of the female from which the sample is taken.

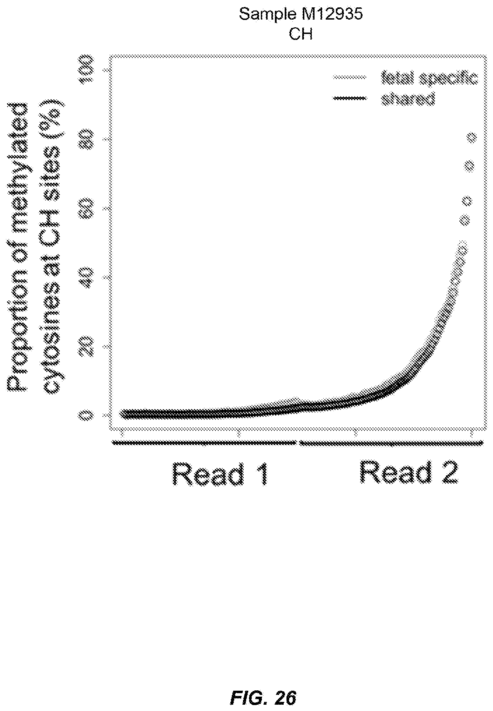

**FIG. 26** shows a representative plot for one sample for the proportion of methylated cytosines in plasma DNA of pregnant women at CH sites. We first examined the proportion of methylated cytosines at the CH context for read 1 and read 2 among those plasma DNA fragments carrying fetal-specific and shared alleles (i.e. predominantly of maternal origin). Both fetal-specific and shared fragments showed a significant increase in the methylation level in regions proximal to the 3' end of a molecule (i.e. read 2). The fetal-specific molecules exhibited a slightly higher methylation level than shared ones, suggesting jagged ends were present in both the maternal DNA and fetal DNA molecules. The results for the other samples were substantially similar.

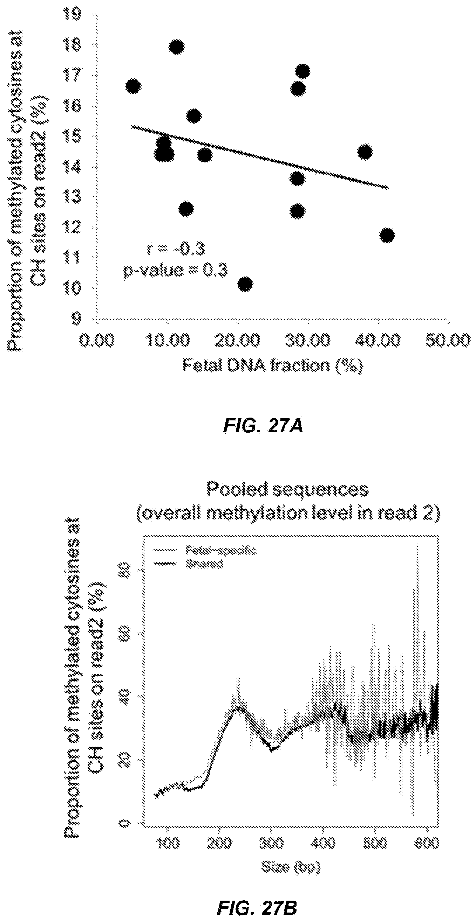

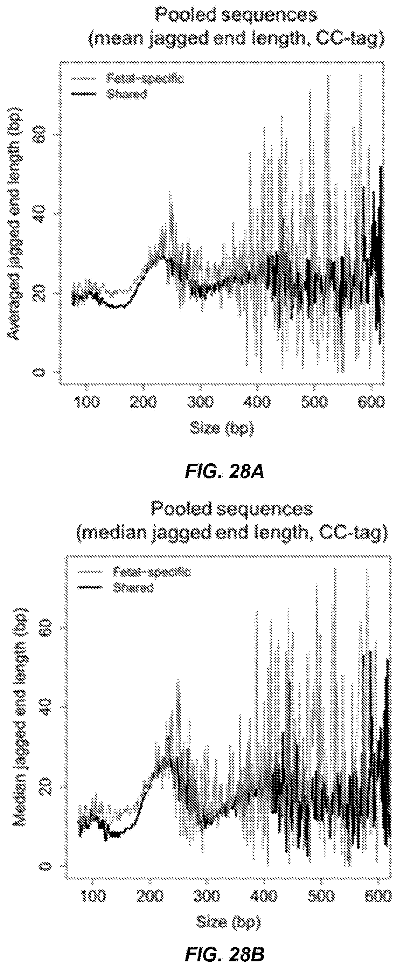

**FIGS. 27A, 27B****,** **28A and 28B** show the profile of jagged ends across different size ranges for fetal-specific and shared DNA molecules. To investigate the relationship between jagged ends and fetal DNA fractions, we correlated the proportion of methylated Cs at CH sites on read 2 and fetal DNA fractions. We found that there was a negative relationship between fetal DNA fraction and the proportion of methylated Cs at CH sites on read 2 (FIG. 27A). This may be caused by the fact that the fetal DNA contained more shorter fragments than maternal DNA, and the shorter DNA molecules generally bore a lower degree of jagged ends than longer DNA molecules (FIG. 27B). In other words, the samples with higher fetal DNA fraction would result in a decrease in the quantity and/or length of jagged ends. It may suggest that jagged ends would be confounded by plasma DNA sizes.

To overcome this confounding factor of plasma DNA size, we examined the jagged end across different sizes. For plasma DNA molecules carrying fetal-specific alleles, a larger proportion of methylated cytosines in the CH context at a size range of 140-200 bp was observed compared with that of sequences carrying shared alleles (FIG. 27B). The larger proportion of methylated cytosines indicates a higher degree of jagged ends from longer and/or a larger amount of jagged ends. We also used the "CC-tag" approach to determine the exact jagged end length in fetal-specific and shared DNA molecules and found that the values of both the average and median jagged end length in fetal-specific molecules were larger than shared ones at a size range of 100-200 bp (FIG. 28A and 28B). The results revealed that jagged end length distribution was indeed affected by sizes and the difference between fetal-specific and shared fragments occurred mainly within the size range of 100-200 bp. These results suggest that restricting analysis of jagged ends to certain size ranges of cell-free DNA fragments may help provide additional information for a sample, such as fetal DNA fraction, tumor DNA fraction, age of a subject, organ transplantation DNA fraction, or the level of an immune response.

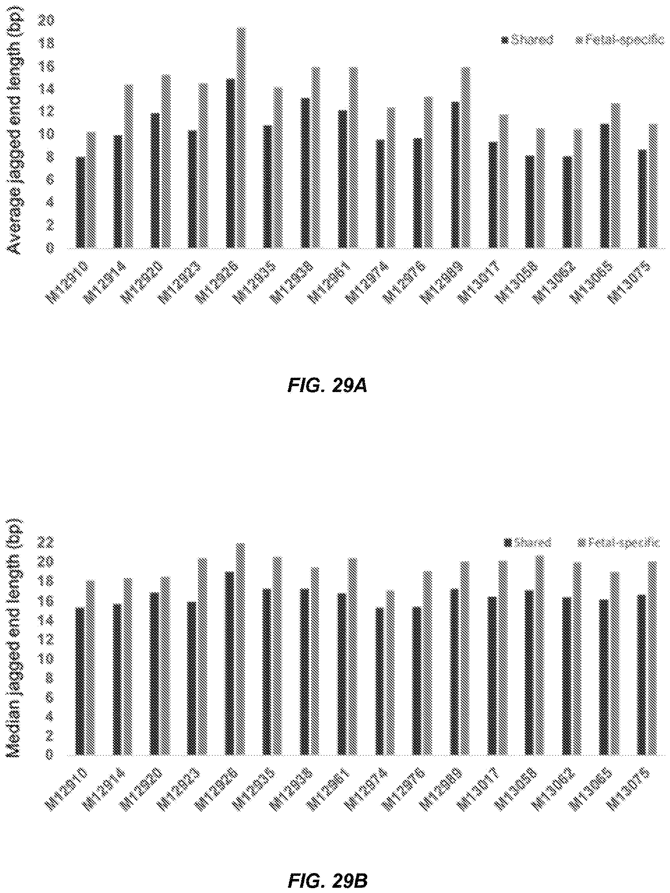

**FIGS. 29A** and **29B** show the jagged end length distributions in molecules within 140-150 bp. In FIG. 29A, the vertical axis is the mean average jagged end length for DNA fragments having a size within 140-150 bp, and the horizontal axis is the identification of the sample. In FIG. 29B, the vertical axis is the median jagged end length for DNA fragments having a size within 140-150 bp, and the horizontal axis is the identification of the sample. We further examined averaged jagged end length of fetal-specific and shared molecules within the range of 140-150 bp, and found that fetal-specific fragments contained a longer jagged end (median: 13.73 bp; 10.24 - 19.38 bp) than the shared ones (median: 10.16 bp; 8.02 - 14.91 bp) (p-value: 0.0014, Mann Whitney U test) (FIG. 29A). The median jagged end length of fetal-specific and shared molecules distributing at 140-150 bp showed a similar pattern to the averaged values (p-value< 0.0001, Mann Whitney U test) (FIG. 29B). These results were consistent with the observation using the alternative method with the filling of unmethylated cytosines, in which the jagged index of shared DNA molecules inferred from the CG context was slightly smaller than that of fetal-specific DNA molecules.

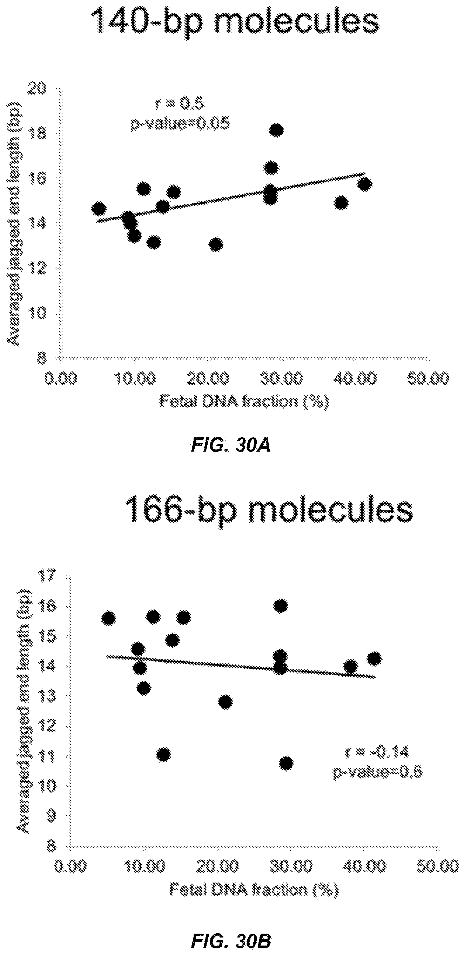

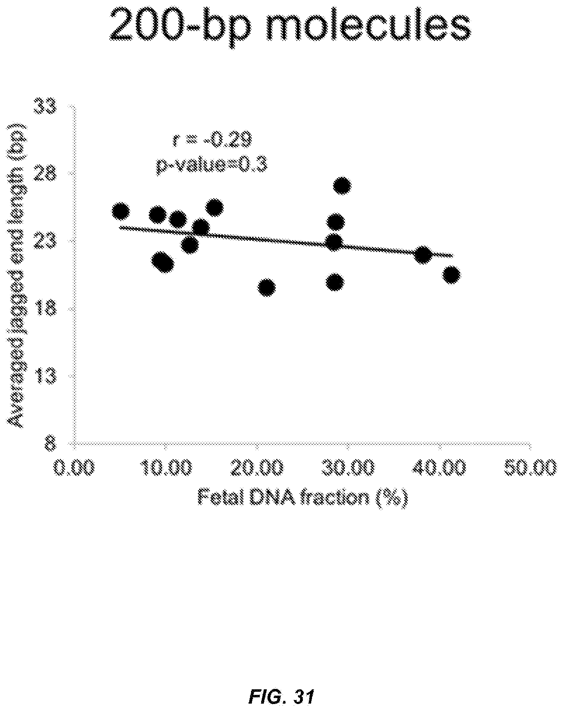

**FIG. 30A, 30B****,** and 31 show jagged end length versus fetal DNA fraction for molecules of 140 bp, 166 bp, and 200 bp. Considering the jagged end length varied depending on different sizes as we mentioned above, we fixed the size of molecules to 140 bp, 166 bp, and 180 bp and then assessed their relative jagged end lengths. Such size-banded analysis revealed a positive correlation between the averaged jagged end length and fetal DNA fraction in the plasma of pregnant women for 140 bp (FIG. 30A). The jagged end length at 166 bp or 200 bp did not show positive correlations with the fetal DNA fraction (FIG. 30B and 31). Taken together, the results we described here may suggest that the jagged ends originating from those molecules ranging from 140 bp to 150 bp likely carried placenta-specific jagged ends.

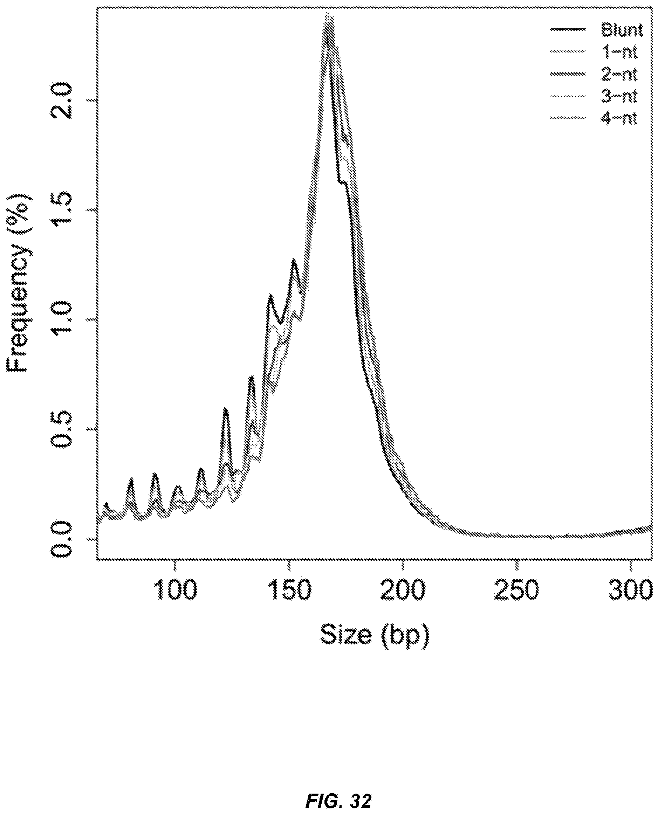

**FIG. 32** shows size distributions for molecules carrying different size jagged end lengths (blunt, 1 nt, 2 nt, 3 nt, and 4 nt). We classified molecules into different groups according to their jagged end lengths. We performed their relative size distributions of plasma DNA molecules for each group with different jagged end lengths. We observed that size distributions bore a much sharper 10 bp periodicities below 155 bp for those molecules with blunt ends. On the other hand, we found that as the jagged end length became longer, their relative periodicity was observed to be weaker, suggesting that jagged ends would vary according to different chromatin structures. The periodicity may correspond with the nucleosomal distance. DNA molecules may form blunt ends at certain locations relative to the nucleosome, thereby resulting in more blunt ends for certain sizes of DNA molecules. FIG. 32 also shows that smaller jagged ends are more prevalent at these peaks, consistent with the data in FIG. 22.

### E. Example method using methylated cytosines to repair jagged ends

Analyzing a biological sample using methylated cytosines to repair jagged ends may be similar to method 400 in FIG. 4. The biological sample may be the biological sample described with FIG. 4 or any biological sample described herein. The biological sample may include a plurality of nucleic acid molecules. The plurality of nucleic acid molecules may be cell-free. Each nucleic acid molecule of the plurality of nucleic acid molecules may be double-stranded with a first strand having a first portion and a second strand. The first portion of the first strand of at least some of the plurality of nucleic acid molecules may overhang the second strand, may not be hybridized to the second strand, and may be at a first end of the first strand.

The plurality of nucleic acid molecules may have sizes with a size range. The size range may be smaller than the range of sizes of all cell-free nucleic acid molecules in the biological sample. As examples, the size range may be 100 to 200 bp, 140 to 200 bp, or 140 to 150 bp. The sizes of a second plurality of nucleic acid molecules in the biological sample may be determined. The second plurality of nucleic acid molecules may include all cell-free nucleic acid molecules in the biological sample. Sizes may be determined by sequencing and aligning the sequence reads to a reference genome. The second plurality of nucleic acid molecules may be filtered to nucleic acid molecules having sizes with the size range.

Similar to block 402, a first compound including one or more nucleotides may be hybridized to the first portion of the first strand for each nucleic acid molecule of the plurality of nucleic acid molecules. The first compound may be attached to a first end of the second strand to form an elongated second strand with a first end including the first compound. The first compound may include a first end not contacting the second strand. The one or more nucleotides may be either all methylated or all unmethylated.

The one or more nucleotides may be all methylated. The methylated nucleotides may be one type of nucleotide, such as cytosines. The first compound may include nucleotides other than the methylated nucleotides. The methylated cytosines in the first compound may be adjacent to an adenine, a cytosine, or a thymine. The methylated cytosines in the first compound may not be adjacent to a guanine. The direction of the adjacency from the cytosine to another nucleotide may be in the 5' to 3' direction.

Similar to block 404, the first strand may be separated from the elongated second strand for each nucleic acid molecule of the plurality of nucleic acid molecules.

Similar to block 406, a first methylation status for each of one or more first sites of the elongated second strand may be determined for each nucleic acid molecule of the plurality of nucleic acid molecules. The one or more first sites may be at the first end of the elongated second strand. The first sites may exclude cytosines adjacent to a guanine, or may include cytosines adjacent to an adenine, a cytosine, or a thymine. The methylation status may be of cytosines adjacent to an adenine, a cytosine, or a thymine.

Unlike block 408, a second methylation status for each of one or more second sites at the second end of the elongated second strand may not be determined. The second sites may exclude cytosines adjacent to a guanine, or may include cytosines adjacent to an adenine, a cytosine, or a thymine. The methylation status may be of cytosines adjacent to an adenine, a cytosine, or a thymine, or may exclude the methylation status of cytosines adjacent to a guanine. Cytosines that are adjacent to adenine, cytosine, or thymine are unlikely to be methylated in the second strand. As a result, the second methylation status may be assumed to be not methylated for the one or more second sites.

Similar to block 410, a first methylation level is calculated using the first methylation statuses for the plurality of elongated second strands at the one or more first sites. The first methylation level may be a mean, median, a percentile, or another statistical value of the first methylation statuses.

Unlike block 412, a second methylation level may not be calculated using the second methylation statuses for the plurality of elongated second strands at the one or more second sites. Because few cytosines adjacent to adenine, cytosine, or thymine are methylated, the second methylation level would be close to zero and need not be calculated.

Similar to block 414, a jagged end value using the first methylation level may be calculated. The jagged end value may be proportional to an average length of the first strands that overhang the second strands. Calculating the jagged end value may be by calculating a difference between the first methylation level and the second methylation level and dividing the difference by the first methylation level (e.g., overall overhang index in FIG. 3).

Control nucleic acid molecules having known lengths of jagged ends (e.g., spike-in sequences of FIG. 18) may be used to determine quantities of jagged ends in a sample. As an example, a plurality of control nucleic acid molecules may be added (spiked-in) to the biological sample, such that they are hybridized concurrently with the hybridizing of nucleic acid molecules originally from the biological sample. In some implementations, the control nucleic acid molecules may be hybridized by first compounds with nucleotides that are all methylated or all unmethylated. The first methylation level may include the methylation statuses of sites from the repaired jagged end of the control nucleic acid molecule. A jagged end value may be determined using one or more methylation levels, e.g., as described above.

Accordingly, the jagged end value may be calculated using methylation statuses or other techniques (e.g., as described herein) from repaired control nucleic acid molecules. This jagged end value determined with the control nucleic acid molecules may be compared to a reference value. The reference value may be obtained without hybridizing control nucleic acid molecules. As an example, the reference value may be obtained without spike-in sequences (e.g., molecules from FIG. 18).

A quantity (e.g., an absolute quantity) of nucleic acids with jagged ends can be determined using the comparison of the jagged end value to the reference value, in combination with the known quantity of the second plurality of nucleic acid molecules that were added. The known amount added can be used to calibrate the absolute amount for the given frequencies measured. Thus, since a known amount of control nucleic acid molecules were added, a relative amount at a particular length can be converted to an absolute amount, e.g., a molar mass or volume.

As an example, the reference value may be a jagged end value determined without control nucleic acid molecules. The jagged end value with control nucleic acid molecules may increase over the reference value. The increase in jagged end value may be proportional to the known quantity of control nucleic acid molecules. The quantity of jagged ends without control nucleic acid molecules can be determined, which may include calculating a ratio of the reference value and the increase in jagged end value and multiplying by the known quantity. In a similar manner, a quantity at a particular length of overhang can be determined based on the frequency at the particular length, the frequency at the known length of the added control nucleic acid molecules, and the known amount of control nucleic acid molecules at the known length that were added to the biological sample.

For example, the jagged end value may increase from a first value when no control nucleic acid molecules are included to a second value when control nucleic acid molecules are included. The increase from the first value to the second value may be attributed to the presence of control nucleic acid sequences, and the magnitude of the increase may therefore reflect the known quantity of control nucleic acid molecules (e.g., a molar concentration). Based on the relationship of the magnitude of the increase to the known quantity, a quantity for the first value and/or the second value can also be determined. This calculated quantity may reflect the total concentration of jagged ends. As an example, if the jagged end value increases from *x* to 1.1*x* when including 1 M control nucleic acid molecules, then the 0.1*x* increase may reflect a concentration of 1 M. The quantity of the jagged ends without the control nucleic acid may be calculated to be 10 M (*x*/0.1*x* × 1 M). In some embodiments, the relationship may not be linear, and the calculation of the quantity of jagged ends may involve non-linear regression or other statistical analysis. Such non-linearity may be partly governed by the kinetics of the method used to detect the jagged ends. For example, some methods may be more efficient for short jagged ends than long jagged ends.

In some embodiments, the amount of jagged ends of certain lengths can also be calculated. A jagged end value can be calculated for certain lengths, and the magnitude of this value can be related to a quantity based on the increase in jagged end value from control nucleic acid molecules and the known quantity of control nucleic acid molecules. The control nucleic acid molecules may also be limited to certain lengths of jagged ends. For example, 1 M control nucleic acid molecules having 13-nt jagged ends may increase the jagged end value from *x* to 1.1*x.* The jagged end value for a 20-nt jagged end may be 0.5*x*. The concentration of the 20-nt jagged ends may be calculated to be 5 M (0.5*x*/0.1*x* × 1M).

In other implementations, other techniques of measurement of the jagged end can be used in conjunction with the control nucleic acid molecules. Accordingly, various techniques can be used to determine a jagged end value using nucleic acid molecules from the biological sample and a plurality of control nucleic acid molecules (e.g., as the cell-free fragments and the control molecules are mixed together), wherein an overhang length of each of the control nucleic acid molecules is known. Then, the jagged end value can be compared to a reference value, the reference value obtained without hybridizing the first compounds to the plurality of control nucleic acid molecules. And, a quantity of jagged ends can be calculated using the comparison of the jagged end value to the reference value and using the known quantity of the second plurality of nucleic acid molecules.

The jagged end value calculated in block 414 may be used in any of the methods described with FIG. 1. For example, the jagged end value may be used to determine a fraction of clinically-relevant DNA, such as fetal DNA, in a biological sample.

### F. Example CC-tag method

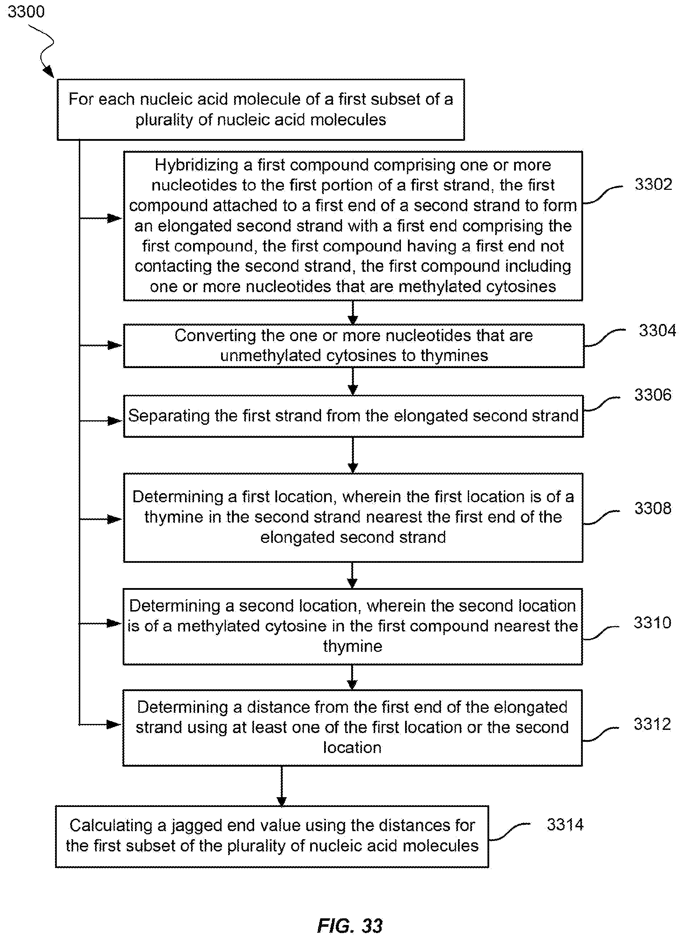

**FIG. 33** shows a method 3300 for calculating a jagged end value with CC-tags. Method 3300 involves analyzing a biological sample obtained from an individual. The biological sample includes a plurality of nucleic acid molecules. The nucleic acid molecules are cell-free. Each nucleic acid molecule of the plurality of nucleic acid molecules is double-stranded with a first strand having a first portion at an end and a second strand. The first portion of the first strand of a first subset of the plurality of nucleic acid molecules has no complementary portion from the second strand. The first portion of the first strand is not hybridized to the second strand and is at a first end of the first strand.

At block 3302, a first compound is hybridized to the first portion of the first strand for each nucleic acid molecule of a first subset of the plurality of nucleic acid molecules. The first compound may be attached to a first end of the second strand to form an elongated second strand with a first end including the first compound. The first compound may have a first end not contacting the second strand. The first compound may include one or more nucleotides that are methylated cytosines. The first subset may include one nucleic acid molecule or a plurality of nucleic acid molecules.

At block 3304, the one or more nucleotides that are unmethylated cytosines are converted to thymines for each nucleic acid molecule of the first subset.

At block 3306, the first strand may be separated from the elongated second strand for each nucleic acid molecule of the first subset.

At block 3308, a first location is determined, where the first location is of a thymine in the second strand nearest the first end of the elongated second strand for each nucleic acid molecule of the first subset.

At block 3310, a second location is determined, where the second location is of a methylated cytosine in the first compound nearest the thymine. The second location may be on the 3' side of the first location. The methylated cytosine may not be adjacent to a guanine.

At block 3312, a distance from the first end of the elongated second strand may be determined using at least one of the first location or the second location for each nucleic acid molecule of the first subset. The distance may be the length of the jagged end. As described with FIG. 16, a TC may indicate the boundary of a jagged end. In some instances, a thymine may not be directly adjacent to the methylated cytosine. In those instances, the distance may be a range of lengths instead of a single length. For example, the first location may indicate the longest possible jagged end, and the second location may indicate the shortest possible jagged end. The distance may then be presented as a range from the shortest length to the longest length. In some embodiments, the distance may be an average of the shortest length and the longest length.

At block 3314, a jagged end value may be calculated using the distances for the first subset of the plurality of nucleic acid molecules.

In some embodiments, analysis may include a second subset of the plurality of nucleic acid molecules. The first portion of each nucleic acid molecule of the second subset of the plurality of nucleic acid molecules has a complementary portion from the second strand and is hybridized to the second strand. The second subset may include nucleic acid molecules with no jagged ends, only blunt ends. The second subset may include one nucleic acid molecule or a plurality of nucleic acid molecules.

Unmethylated cytosines in the nucleic acid molecules of the second subset may be converted to thymines. The conversion of unmethylated cytosines in the second subset may be substantially at the same time as the conversion in block 3304.

A thymine may be determined to be at the end of the second strand. As a result, the second strand may be determined to be not elongated. The nucleic acid molecule may be identified as not having a jagged end. The distance of the thymine to the end of the second strand may be determined. This distance may be zero when the thymine is located at the end of the second strand. The jagged end value may be calculated using the distances for the second subset.

The jagged end value calculated in block 3314 may be used in any of the methods described with FIG. 1. For example, the jagged end value may be used to determine a fraction of clinically-relevant DNA, such as fetal DNA, in a biological sample.

### IV. PLASMA DNA END LIGATION-MEDIATED OVERHANG DIRECT DETERMINATION

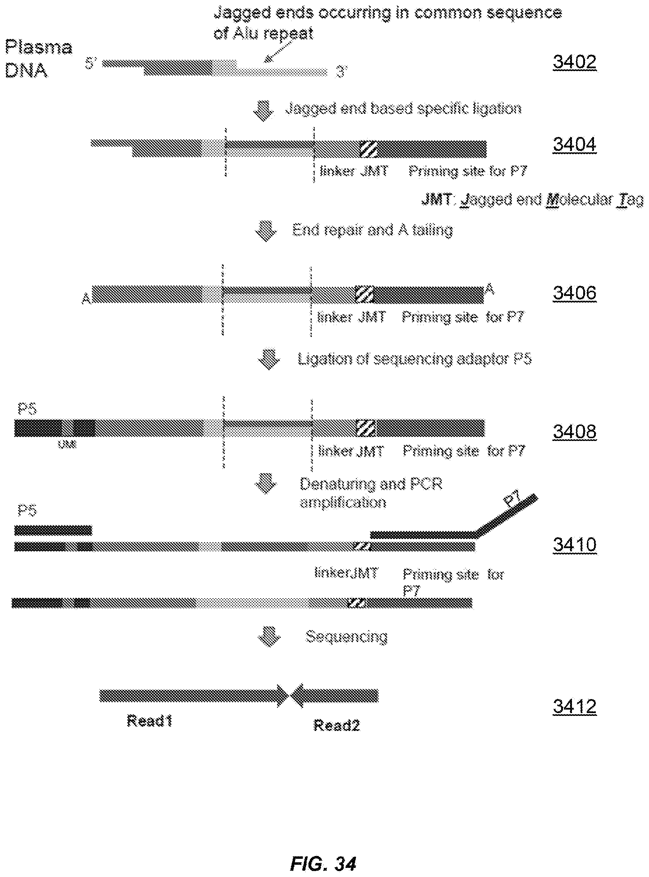

Another embodiment to assess the plasma DNA overhang is to ligate double-stranded sequence adaptors carrying a single-stranded synthesized oligonucleotide (overhang probe) with sequence tag allowing tracing back the probe sequence compositions and length to a plasma DNA. Such synthesized oligonucleotides are able to be annealed and ligated to the plasma DNA carrying overhangs which are complementary to the design oligonucleotides. By sequencing the sequence tag on adaptors allows us to infer the plasma DNA overhang sequences and their corresponding sizes. FIG. 34 illustrates the principle of DNA end ligation-mediated overhang direct determination.

Stage 3402 shows a double-stranded DNA molecule with jagged ends. The jagged end occurs in the common sequences of the Alu repeat. The common sequences of the Alu repeat may have thousands of copies in the human genome.

As shown in stage 3404, a common sequence could be hybridized to a synthesized probe (red bar between dash lines). Such a probe is linked to an adaptor which comprises linker (green), jagged end molecular tag (JMT, rectangle filled with diagonal stripes), and priming site for sequencing adaptor (i.e. Illumina P7). Because the length of the common sequence is finite, the types of synthesized probes could be enumerated. A particular type of synthesized probe corresponds to a unique JMT sequence. The types of probes would be equal to the length of the common sequence. For example, if the length of the common sequence is 24-nt, the types of probes to be synthesized is 24 and the number of unique JMT sequence would be 24.

At stage 3406, after jagged end specific ligation with the corresponding probe, end repair and A-tailing will be carried out.

At stage 3408, subsequently, sequencing adaptors (e.g. Illumina P5) will be ligated to repaired molecules.

At stage 3410, P5 ligated molecules could be denatured and amplified by P5 and P7 primers though PCR amplification, producing the molecules that are suited for sequencing in Illumina platform.

At stage 3412, paired-end sequencing is performed. Read2 contains the JMT sequence which allows for tracing the original probes being hybridized to the molecules carrying the jagged ends of interest. Read1 is expected to carry the common sequence and its flanking sequence, allowing for identifying its genomic origin.

Such a method could be generalized to studying jagged ends of any plasma DNA molecule by synthesizing random probes tagged to unique JMT adaptors, thus enabling the feasibility of detecting the jagged ends in a genome-wide manner.

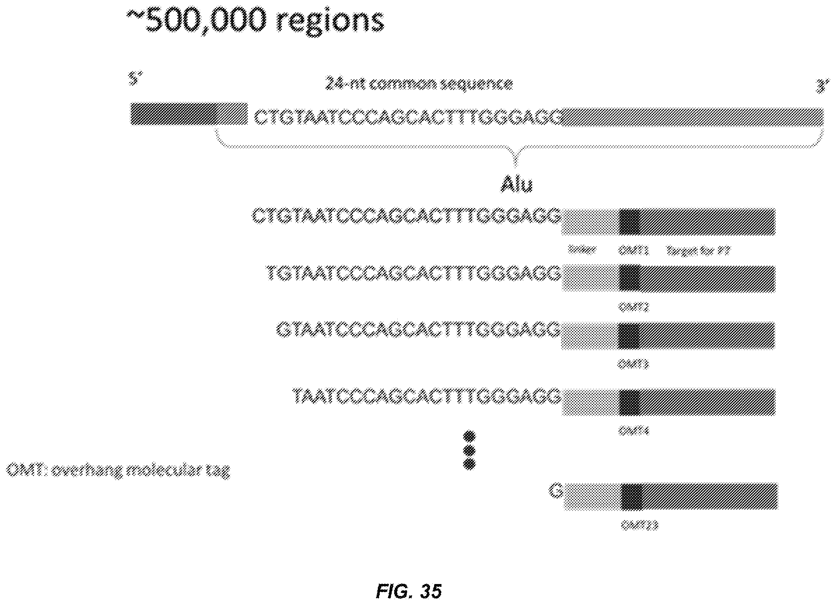

One embodiment in ligation-based plasma DNA overhang assessment is to search for a common sequence which is present in a human genome with numerous copies, for example, the common sequence present in Alu repeats. Through synthesizing the finite number of ligating oligonucleotides would allow us to determine all the plasma DNA overhangs occurring in such a common sequence which is present in a human genome with around 500,000 copies (FIG. 35).

The synthesized oligonucleotides cover all combinations of overhangs originating from such a common sequence occurring with 500,000 copies in a human genome. Therefore, the plasma DNA overhangs generating from this common region can be identified by sequencing the plasma DNA molecules specifically ligated with the limited number of designed oligonucleotides.

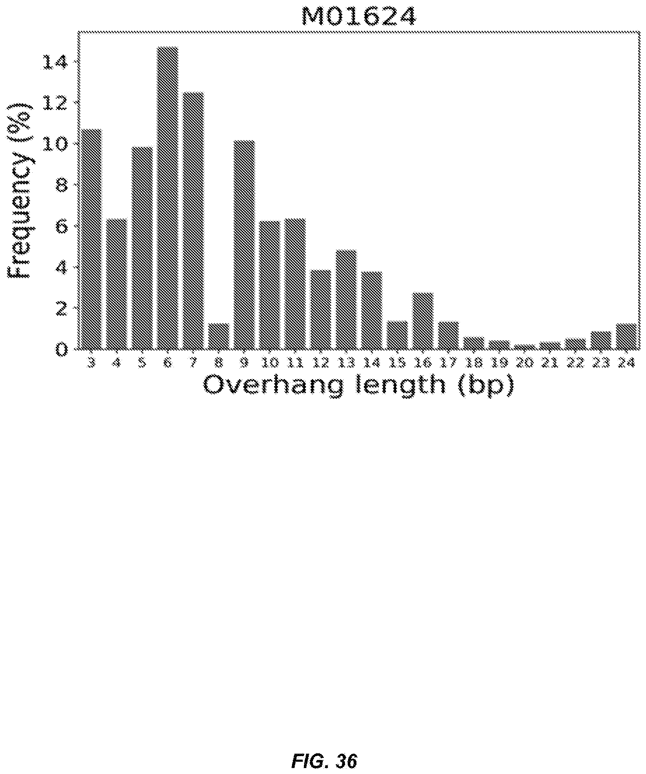

Using the strategy based on a common sequence mediated overhang determination, we sequenced one plasma DNA sample of a pregnant woman after the plasma DNA molecules are ligated with the designed oligonucleotides as shown in FIG. 35. We obtained 32 million paired sequencing reads in our first trial where we started oligonucleotides covering from 3-nt to 24-nt overhangs (i.e. in total 22 types of oligonucleotides which uniquely labeled by a molecular tag in the adaptor). There were 16.3 million (51%) first end reads (read1) was uniquely mapped to a human genome and 12.1 million (37%) first end reads were mappable but aligned to multiple genomic locations. Thus, a total of 88% sequencing reads could be aligned to a human reference for the downstream data analysis. Then, we attempted to identify the OMT sequence in the paired second read (read2) of a fragment with a mappable read1. There were 12.8 million (45%) of fragments with a mappable read1 bearing a valid OMT sequence, suggesting the ligation process is successfully achieved. The frequency and percentage for each sequenced OMT identified in the ligated maternal plasma DNA of case M01624 were calculated. FIG. 36 showed the frequency distribution of overhang length of maternal plasma DNA. Most of the plasma DNA molecules (71%) carry overhangs below 10 nt (nucleotides) in length but there is still a small population (9%) of plasma DNA molecules carrying an overhang above 16 nt in length. Such a relative distribution may be linked to a certain pathophysiology. The remaining ones are between 10 nt and 16 nt in size. In comparison with a certain control group, the relative change in the frequencies of overhang length may inform the patient's status, for example including but not limited to, inflammation, trauma, cancer and/or organ damages etc.

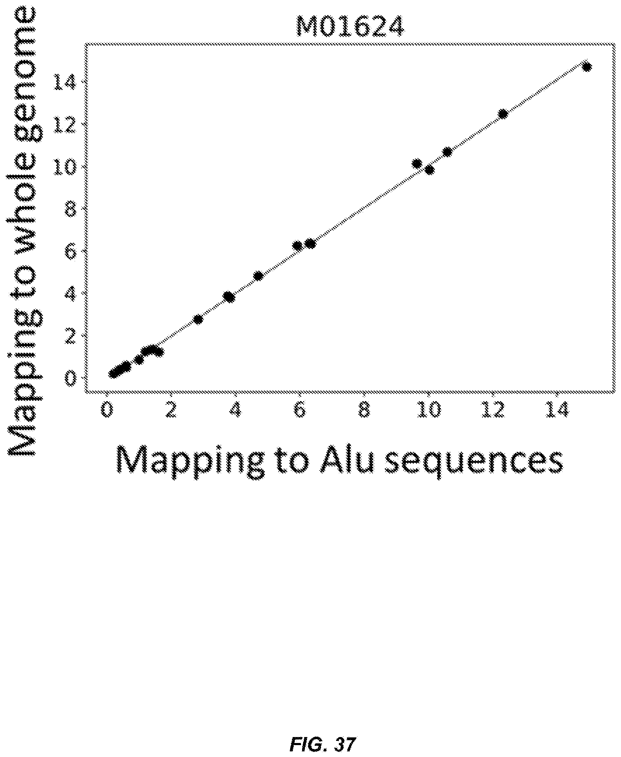

On the other hand, the sequencing reads can be mapped to sequences around the common sequence mined from a human genome, which can speed up the bioinformatics data analysis. As shown in **FIG. 37****,** the inferred frequencies of plasma DNA overhang lengths were highly consistent using two aligning strategies (mapping to the whole genome vs. Alu sequences bearing the common sequence). The sharp reduction of overhang with 8 nt is likely due to secondary structures of that synthesized adaptor because, through in-silico second structure prediction, we found a special self-annealing stem loop formed between the OMT sequence and oligonucleotide with 8 nt. Such a self-annealing issue could be solved by changing the sequence context of OMT sequence in a new design. In addition, the adaptors carrying oligonucleotides targeting to ligate 0-nt, 1-nt and 2-nt overhangs can be also designable.

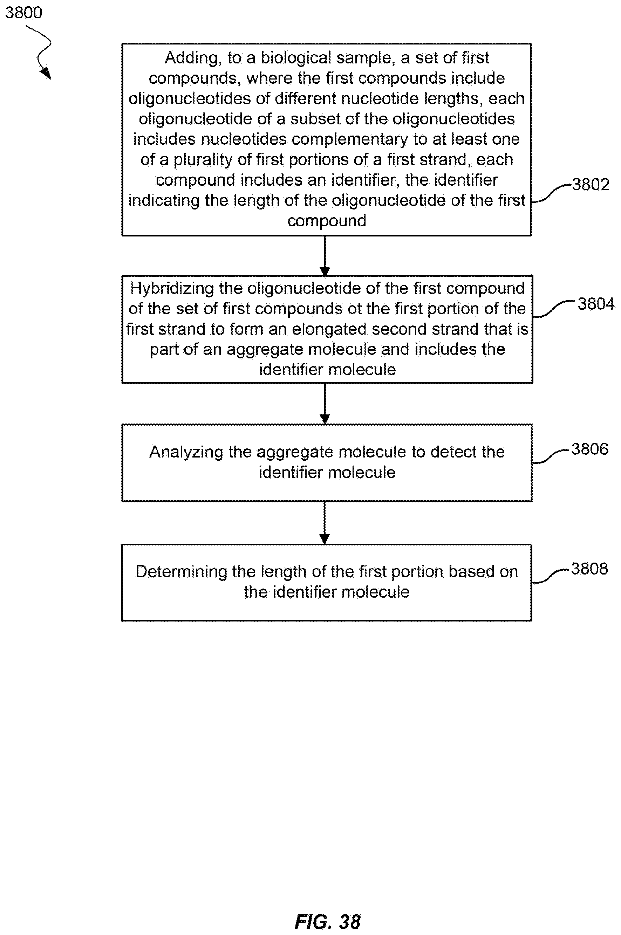

**FIG. 38** shows a method 3800 of analyzing a biological sample obtained from an individual. The biological sample may include a plurality of nucleic acid molecules. The plurality of nucleic acid molecules may be cell-free. Each nucleic acid molecule of the plurality of nucleic acid molecules may be double-stranded with a first strand having a first portion and a second strand. The first portion of the first strand of at least some of the plurality of nucleic acid molecules may overhang the second strand, may not be hybridized to the second strand, and may be at a first end of the first strand.

At block 3802, a set of first compounds may be added to the biological sample. The set of first compounds may include oligonucleotides of different nucleotide lengths. Each oligonucleotide of a subset of the oligonucleotides comprises nucleotides may be complementary to at least one of a plurality of the first portions. The subset may include the set of all the oligonucleotides. The oligonucleotides may include nucleotdies of an Alu sequence.

Each first compound of the set of first compounds may include an identifier molecule. The identifier molecule may indicate a length of the oligonucleotide of the first compound. The identifier molecule may be a fluorophore. In some embodiments, the identifier molecule may include a sequence that was predetermined to correspond to the length of the oligonucleotide.

At block 3804, the oligonucleotide of a first compound of the set of first compounds may be hybridized to the first portion of the first strand to form an elongated second strand that is part of an aggregate molecule and includes the identifier molecule. Hybridizing may be performed for each nucleic acid molecule of the plurality of nucleic acid molecules.

At block 3806, the aggregate molecule may be analyzed to detect the identifier molecule. The aggregate molecule may be analyzed as a double-stranded molecule or may be denatured so that a single-stranded molecule is analyzed. The analysis may be by sequencing or detecting a fluorescence signal. The method may further include sequencing the elongated second strand to produce reads corresponding to the identifier molecule. The analysis may be performed for each nucleic acid molecule of the plurality of nucleic acid molecules.

At block 3808, the length of the first portion may be determined based on the identifier molecule. The determination may involve referring to a reference that links a particular identifier molecule with a particular length. The determination may be performed for each nucleic acid molecule of the plurality of nucleic acid molecules.

The hybridization-based method 3800 can allow access to both 5' and/or 3' protruded ends (single strand part) by synthesizing different strands of hybridizing probes. However, the DNA polymerase based methods may be only suited for 5' protruded single-strand end due to its directionality of elongation.

The length determined in block 3808 may be used as the measured property in any of the methods described with FIG. 1. Thus, a jagged end value can be determined using method 3800.

Method 3800 may also be applied to the spiked-in sequences used to determine a quantity of jagged ends as described above in Section III(E) and with FIG. 18. A known quantity of nucleic acid molecules with known jagged end lengths and known sequences can be added. The lengths of the jagged ends can then be determined, as described in method 3800. Once the jagged end value is measured, the quantities of jagged ends in the biological sample can be determined using the known quantity of the spike-in sequences.

### V. JAGGED END ANALYSIS WITH MASSIVELY PARALLEL BISULFITE SEQUENCING

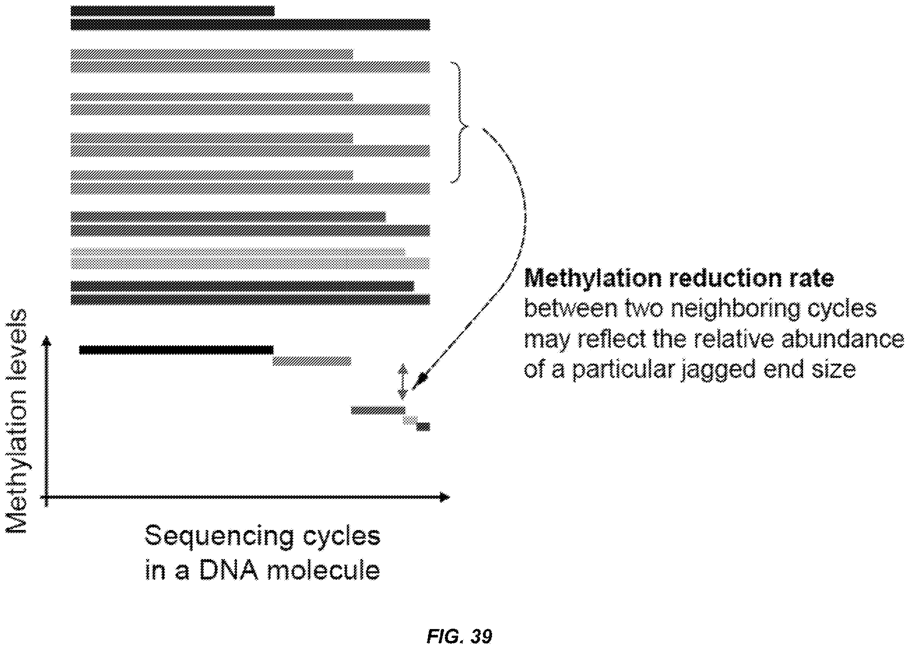

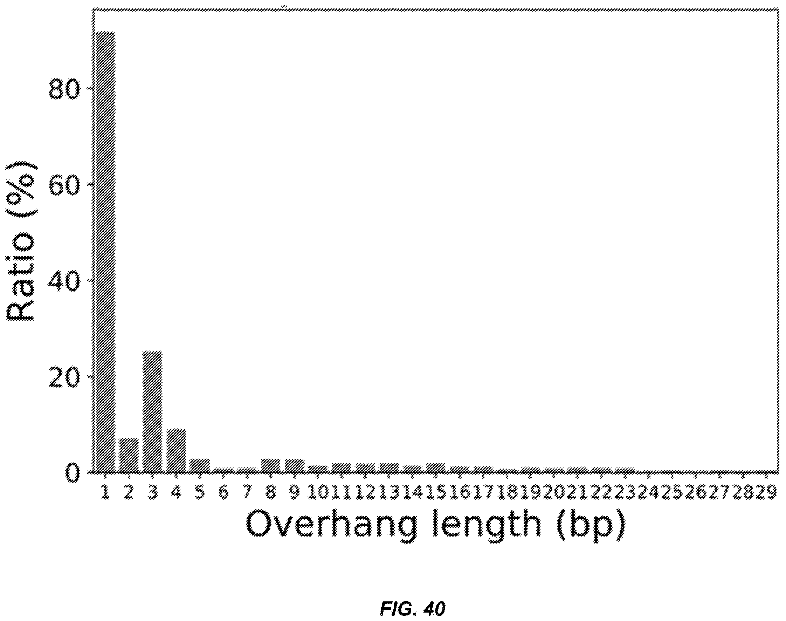

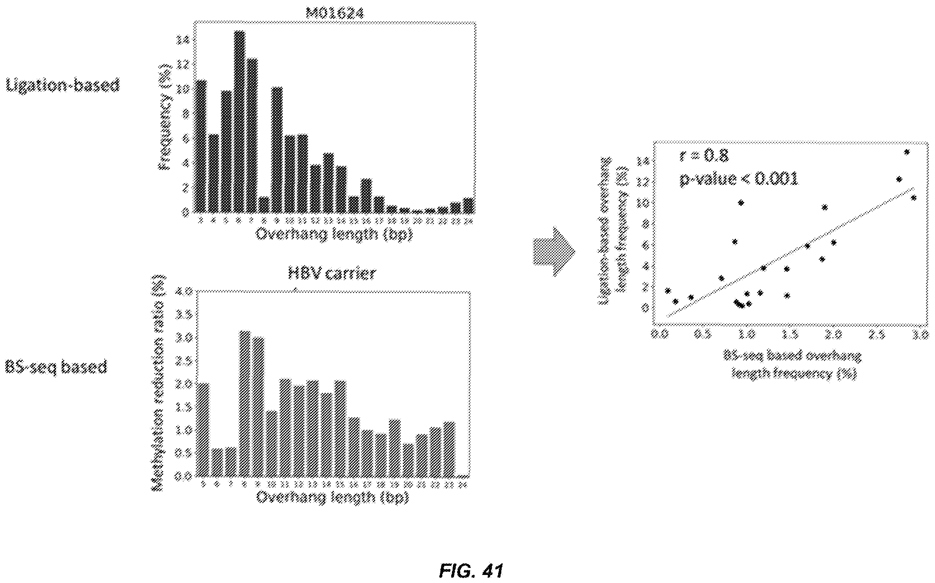

Another embodiment, the relative overhang abundance of a particular size can also be estimated from massively parallel bisulfite sequencing **(****FIG. 39****).** The higher the abundance of an overhang with a particular size, the more the reduction of methylation levels compared with the previous cycle would be. For example, the difference in methylation level between the last cycle and the second last cycle would reflect the relative abundance the 1-nt overhang. As shown in **FIG. 40****,** the predominant plasma DNA molecules would bear 1-nt overhang. The frequencies of overhang lengths measured by the ligation-based and BS-seq based approaches are well-correlated **(****FIG. 41****).**

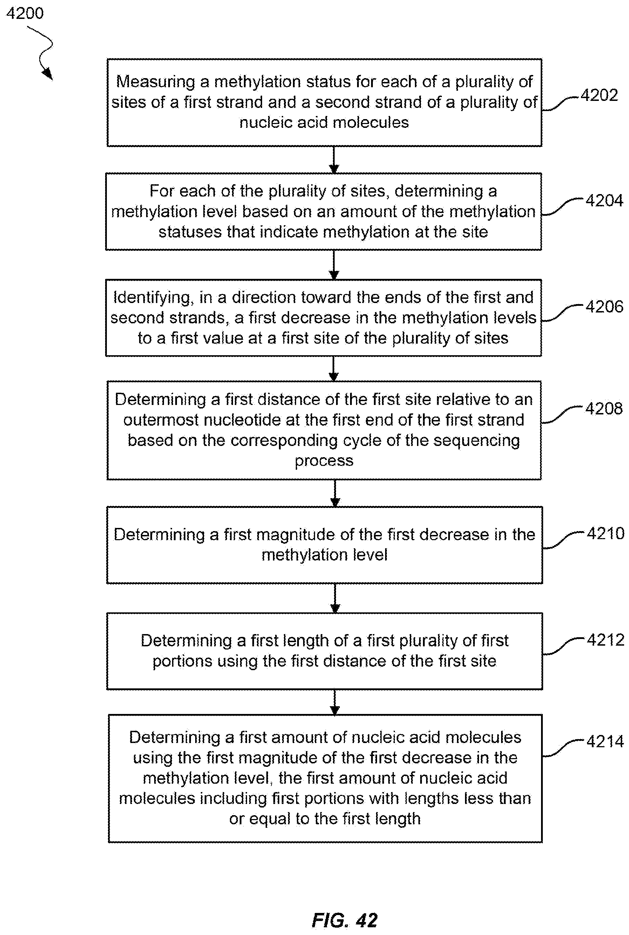

**FIG. 42** shows a method 4200 of analyzing a biological sample obtained from an individual. The biological sample may include a plurality of nucleic acid molecules. The plurality of nucleic acid molecules may be cell-free. Each nucleic acid molecule of the plurality of nucleic acid molecules may be double-stranded with a first strand having a first portion and a second strand. The first portion of the first strand of at least some of the plurality of nucleic acid molecules may overhang the second strand, may not be hybridized to the second strand, and may be at a first end of the first strand.

At block 4202, a methylation status is measured for each of a plurality of sites of a first strand and a second strand of the plurality of nucleic acid molecules. Each site of the plurality of sites may correspond to a cycle of a sequencing process. The plurality of sites may cover ends of the first and second strands. The ends of the first and second strands may include the first end of the first strand. In some embodiments, the methylation status may be measured without separating the strands. For example, the methylation status may be measured using a nanopore. In other embodiments, only one strand may be amplified and sequenced.

In some embodiments, a first compound including one or more nucleotides may be hybridized to the first portion of the first strand. The one or more nucleotides may be unmethylated. The first compound may be attached to a first end of the second strand to form an elongated second strand with a first end including the first compound. The first compound may have a first end not contacting the second strand. The first strand may be separated from the elongated second strand. The methylation status may be measured using site of the elongated second strand.

At block 4204, a methylation level is determined for each of the plurality of sites based on an amount of methylation statuses that indicate methylation at the site. In some embodiments, the amount of methylation statuses that indicate methylation at the site may be determined from the amount of methylation statuses that indicate no methylation at the site.

At block 4206, a first change in the methylation levels to a first value at a first site of the plurality of sites is identified in a direction toward the end of the first and second strands. The first change may be an increase or decrease in the methylation levels.

At block 4208, a first distance of the first site relative to an outermost nucleotide at the first end of the first strand is determined based on the corresponding cycle of the sequencing process.

At block 4210, a first magnitude of the first decrease in the methylation level is determined.

At block 4212, a first length of a first plurality of first portions using the first distance of the first site is determined.

At block 4214, a first amount of nucleic acid molecules is determined using the first magnitude of the first decrease in the methylation level, the first amount of nucleic acid molecules comprising first portions with lengths less than or equal to the first length.

Blocks 4206 to 4214 may be repeated. For example, method 4200 may include identifying, in the direction toward the ends of the first and second strands, a second change in the methylation level to a second value at a second site of the plurality of sites. The second change may be an increase or a decrease but should be the same type of change as the first change. The second site may be at a second distance relative to the outermost nucleotide at the first end of the first strand. The second distance is less than the first distance. The second value is lower than the first value. The second magnitude of the second change in methylation level may be determined. A second length of a second plurality of first portions using the second distance of the second site may be determined. A second amount of nucleic acid molecules using the second magnitude of the second change in the methylation level may be determined. The second amount of nucleic acid molecules includes first portions with lengths less than or equal to the second length of the second plurality of first portions. The first amount includes first portions with lengths greater than the second length.

The lengths and/or amounts determined in this method may be used as the measured property in any of the methods described with FIG. 1.

### VI. SIZE-BASED OVERHANG ANALYSIS

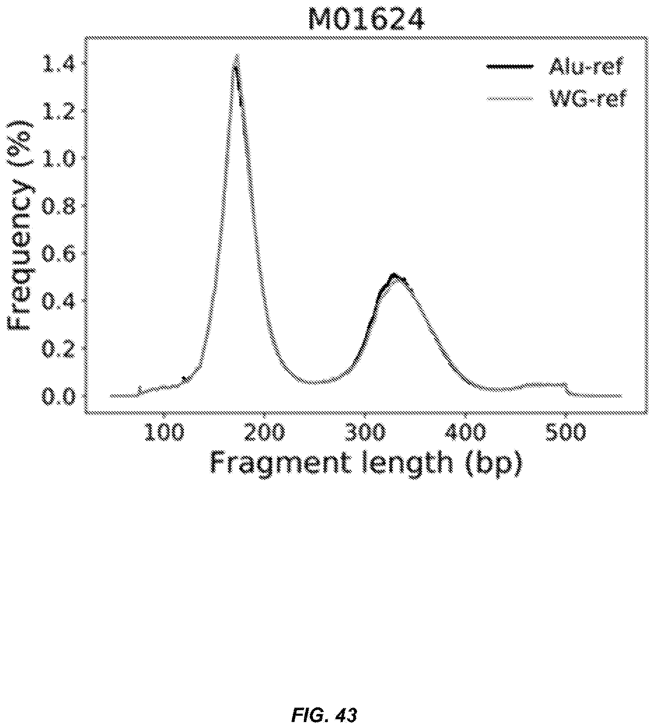

The size of fragments with jagged ends may be measured after analysis with plasma DNA end ligation. After the sequenced fragments which are supposed to carry the unique parts (normally present in read1) adjacent to the common sequence are uniquely aligned to human reference genome with a maximum of two mismatches, the read2 normally bearing the common sequence which are highly repetitive in a human genome could be still unambiguously located in the regions proximal to read1 by taking advantage of read1 mapping information. Therefore, the original fragment size can be inferred with the use of the outermost genomic coordinates of a mapped fragment. The fragments being analyzed also showed a 166 bp major peak and a second peak at ~320 bp in the size profile **(****FIG. 43****).**

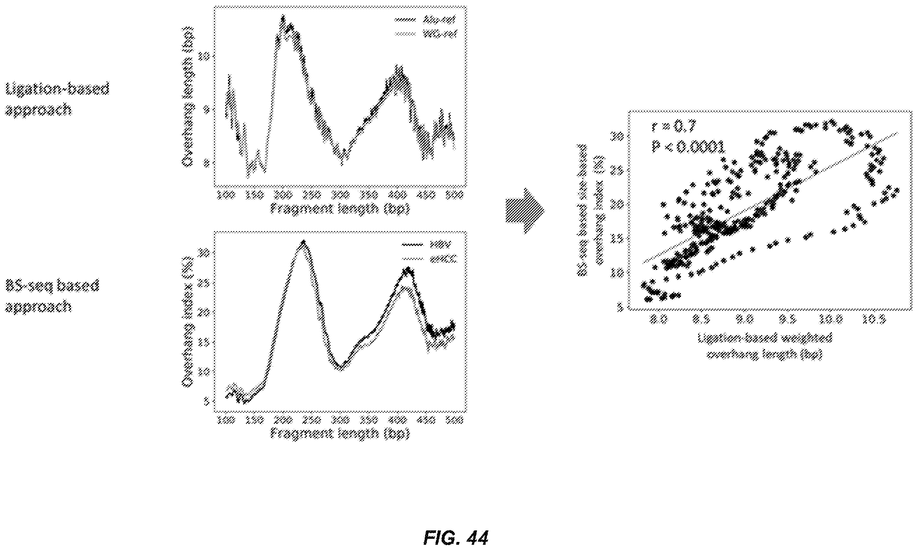

Once the fragment size information is obtained, we can quantify the relationship between the overhang length and fragment size for plasma DNA molecules. In one embodiment, we partition the plasma DNA molecules into different size ranges and quantify the relative overhang length (average or weighed average) in each size range, for example including but not limited to, 100 bp, 101 bp, 102 bp, 103 bp, 104 bp, 105 bp, 106 bp, 107 bp, 108 bp, 109 bp, 110 bp, 120 bp, 130 bp, 140 bp, 150 bp, 160 bp, 170 bp, 180 bp, 190 bp, 200 bp, 300 bp, 400 bp, 500 bp, 600 bp etc. or <100 bp, <110 bp, <120 bp, <130 bp, <140 bp, <150 bp, <160 bp, <170 bp, <180 bp, <190 bp, <200 bp etc. or >210 bp, >220 bp, >230 bp, >240 bp, >250 bp, >260 bp, >270 bp, >280 bp, >290 bp, >300 bp etc. or ratios between any combinations. The relative overhang length may be quantified by a ratio, difference, or a linear or nonlinear combination adjusted by a set of weighting coefficients (e.g., a linear transformation or logit transformation). In **FIG. 44****,** the overhang lengths are shown to be a wave-like single across different fragment sizes. The maximum of overhang length was located at ~200 bp in the results generating from the ligation-based approach. The similar patterns could be reproduced (r=0.7, p<0.0001) in the results originating from the BS-seq based approach (FIG. 44). Fragment size analysis may be used in combination with other techniques described herein to analyze jagged ends.

### VII. CELL-FREE DNA DAMAGE ANALYSIS AND ITS CLINICAL APPLICATIONS

As described for FIG. 1, a jagged end value can be used to determine a level of a condition. Examples for cancer and auto-immune diseases are provided.

### A. Overhang index between cancer and non-cancer subjects

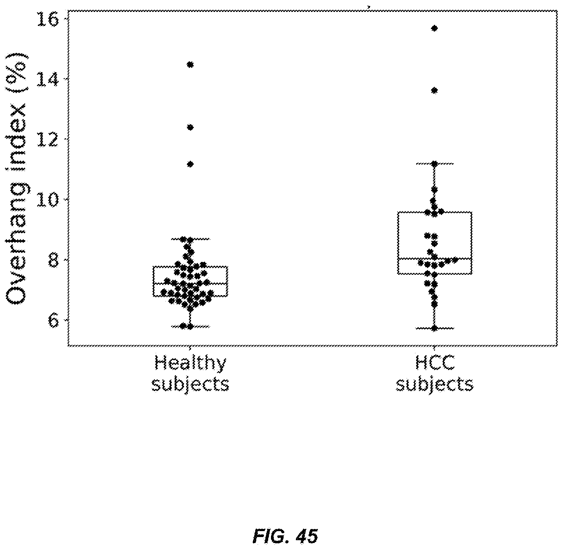

We further analyzed overhang indices in 47 healthy and 28 HCC subjects, respectively. The massive parallel paired-end bisulfite sequencing (75 bp x 2) was used to sequence those samples to a median of 132.9 million paired reads (range: 1.2-261.8 million). In FIG. 45, we observed there was a significant elevation of overhang index for those fragments with a size between 120 and 140 bp in HCC subjects compared with healthy subjects (P-value: 0.048, Mann-Whitney test), suggesting that the overhang index could be used for informing the likelihood of a patient having cancers.

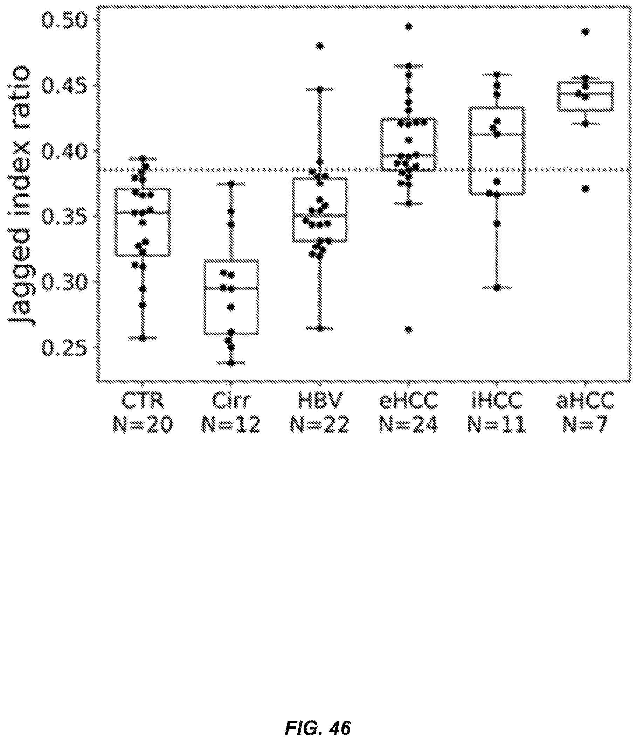

FIG. 46 shows the jagged index ratio across different clinical conditions. The jagged index ratio is determined using the jagged end value for sizes of 140 to 160 bp compared to jagged end values for all other sizes. To determine the diagnostic performance in detecting the cancer using cell-free DNA jagged end index, using the massively parallel bisulfite sequencing technology, we sequenced 20 healthy controls (CTR), 12 cirrhotic subjects (Cirr), 22 HBV carriers (HBV), 24 early stage HCC (eHCC), 11 intermediate stage HCC (iHCC), and 7 advanced stage HCC (aHCC). If we adopted a cutoff of 0.38 in terms of jagged index ratio, we could achieve an overall specificity of 91% and sensitivity of 74%. For particular conditions, we could achieve 90%, 100%, and 86% specificities for CTR, Cirr and HBV, respectively; and 75%, 54%, and 85% sensitivities for eHCC, iHCC and aHCC, respectively.

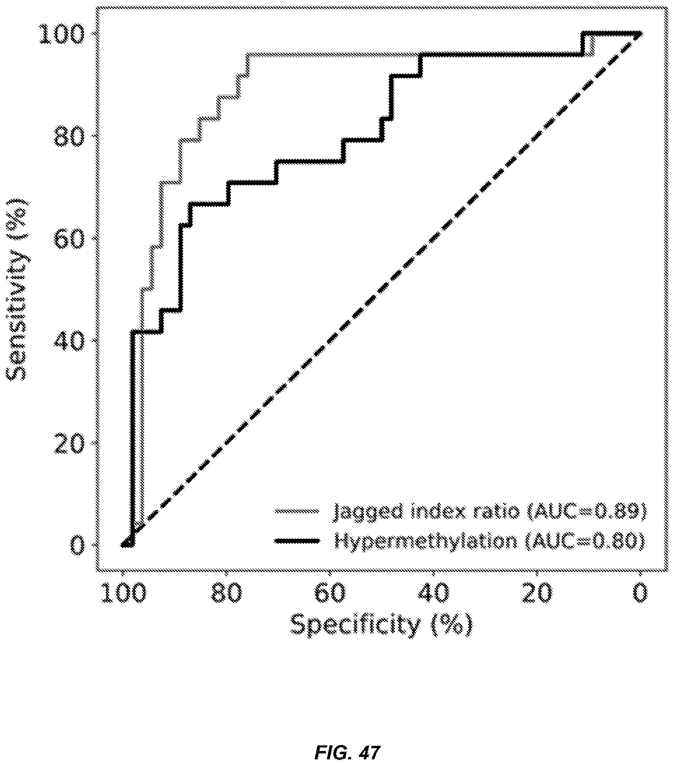

FIG. 47 shows receiver operating characteristic (ROC) for the jagged index ratio approach and for using hypermethylation on CpG islands for HCC. The performance using the jagged index ratio was shown to be superior to the conventional approach using hypermethylation of CpG islands with the jagged index ratio having an area under the curve (AUC) of 0.89 compared to 0.80 for hypermethylation.

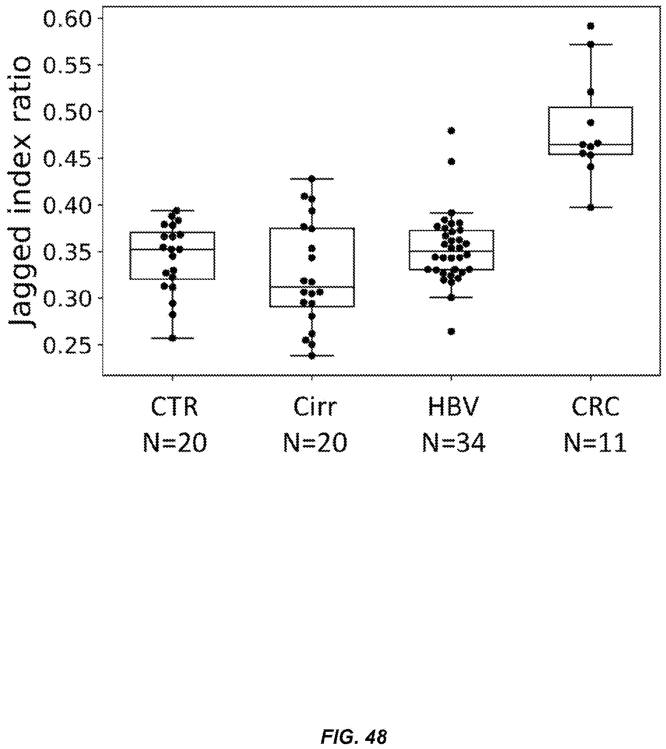

FIG. 48 shows the jagged index ratio across different clinical conditions. The jagged index ratio is determined using the jagged end value for sizes of 140 to 160 bp compared to jagged end values for all other sizes. To determine the diagnostic performance in detecting the cancer using cell-free DNA jagged end index, using the massively parallel bisulfite sequencing technology, we sequenced 20 healthy controls (CTR), 20 cirrhotic subjects (Cirr), 34 HBV carriers (HBV), and 11 colorectal cancer subjects (CRC). The jagged index ratio in patients with CRC (mean 0.48) was found to be significantly higher (p-value < 0.0001) than non-cancerous patients (mean: 0.35).

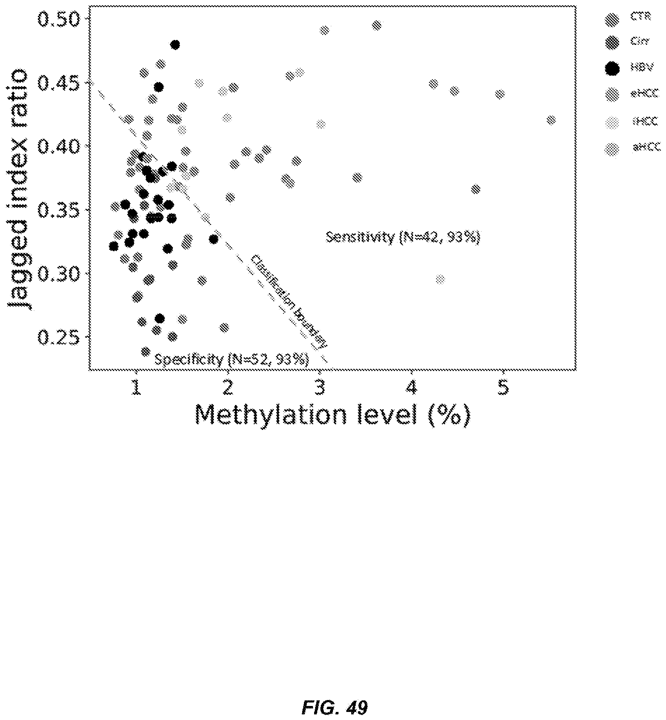

FIG. 49 shows that the combined analysis using both hypermethylation and jagged index ratio could improve classification of a clinical condition. To explore the synergistic effect by combinatorial use of hypermethylation and jagged index ratio, we constructed a scatter plot between hypermethylation (x-axis) and jagged index ratio (y-axis). In order to determine hypermethylation, first, we identified CpG sites in the genome that are found to be "stably unmethylated" among a list of healthy organs. These sites in cancer patients may become methylated. The methylation levels may depend on cancer progression (e.g., cancer stages). Stably unmethylated CpG sites in healthy organs include the following reference tissues: CD4, CD8, erythroblast, macrophage, monocytes, naive B-cell and neutrophil, NK cells, and liver. The methylation levels may be required to be <2% (or another percent) in those reference tissues. About 1 million CpG sites distributed across the genome fulfilled these criteria.

When we analyze a sample, the cell-free DNA library is bisulfite converted. The cell-free DNA molecules are sequenced and then aligned to a reference genome. We then determined the methylation density at the approximately 1 million CpG sites. The methylation density is measured using approaches described in US Patent Publication No. 2014/0080715 A1, filed March 15, 2013. The methylation density may be the percentage of methylated cytosine among all cytosines present on the sequenced cell-free DNA molecules aligned with a defined genomic region. In FIG. 49, the methylation density is determined as one aggregate number for the 1 million CpG sites. The methylation level for non-cancer plasma samples would be expected to be low. When the plasma sample contains tumor-derived cell-free DNA, the methylation level would be expected to increase.

The best separating boundary between HCC and non-HCC was indicated by the dashed line. A sensitivity of 93% at the specificity of 93% would be achieved, suggesting much better improvement in detecting HCC patients with the simultaneous use of methylation and jagged end signals in comparison to the use of single metric (only hypermethylation or jagged index ratio). The combined analysis may be used for other clinical conditions other than HCC.

Accordingly, FIGS. 46-48 show example data for determining a level of a condition (e.g., as described in FIG. 1) using a jagged end value, where the condition is cancer, e.g., HCC or CRC.

### B. Differential overhang index between patients with and without autoimmune diseases

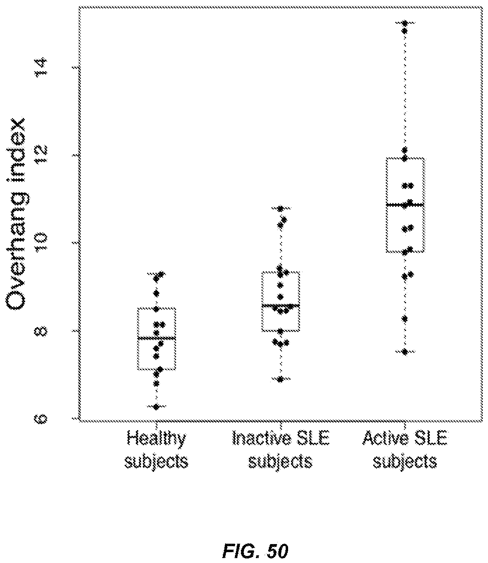

We analyzed overhang indices in 14 healthy, 21 inactive systemic lupus erythematosus (SLE) inactive and 19 active SLE subjects. The massively paired-end bisulfite sequencing was used to sequence those samples to a median of 129.5 million paired reads (range: 26.4-191.4 million). The overhang index was quantified with the use of molecules with a size of between 120 and 140 bp for each sample using the aforementioned method. In FIG. 50, we observed there was a significant elevation of overhang index seen in active SLE subjects compared with healthy subjects (P-value < 0.0001) and inactive SLE subjects (P-value = 0.0006), suggesting that the overhang index could be used for informing the likelihood of a patient having autoimmune diseases and monitoring following treatments. Accordingly, FIG. 50 shows example data for determining a level of a condition (e.g., as described in FIG. 1) using a jagged end value, where the condition is an auto-immune disease, specifically SLE.

### C. The relationship between overhang indices and size ranges

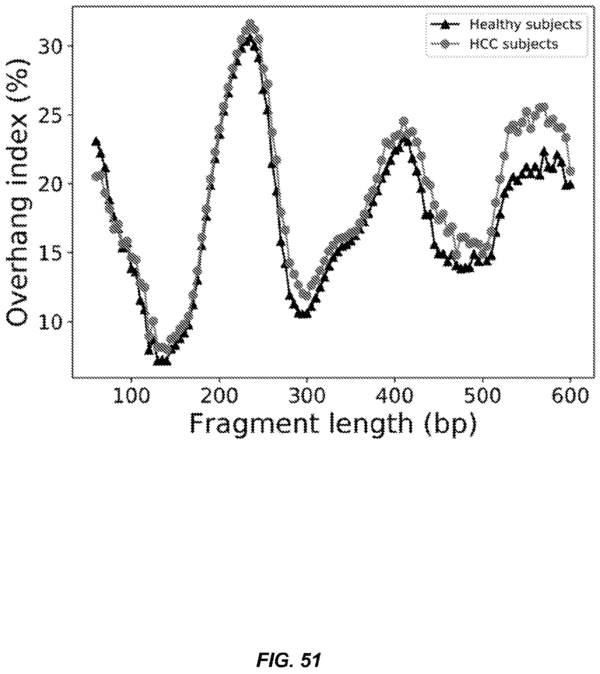

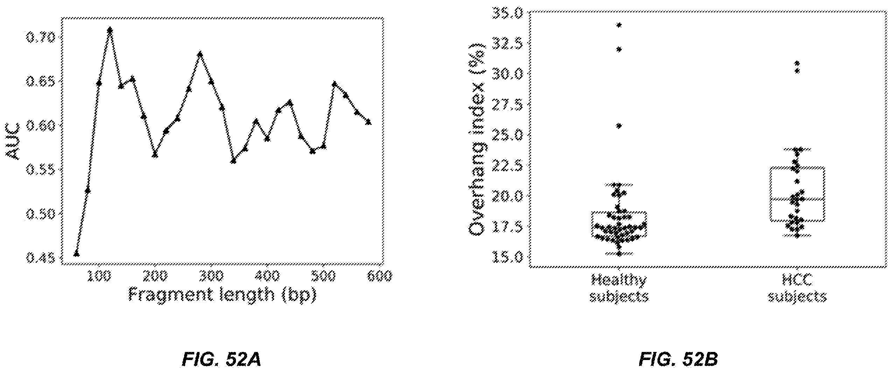

We further study the relationship between overhang indices and size ranges to be analyzed. It has been demonstrated that nonhematopoietically derived DNA is shorter than hematopoietically derived DNA in plasma (Zheng YW et al. Clin Chem. 2012;58:549-58). To visualize and study the relationship between overhang indices and fragment sizes, we pooled all sequenced fragments from healthy subjects and HCC subjects, respectively, to obtain relatively higher sequencing coverage. Interestingly, the overhang index was unevenly distributed across the different size ranges being analysis in both healthy and HCC subjects **(****FIG. 51****),** showing a wave-like nonrandom patterns. There were multiple major peaks occurring at around 80 bp, 240 bp, 400 bp, and 560 bp, respectively. The distance between two adjacent major peaks in FIG. 51 was found to be around 160 bp, suggesting that such overhang indices might be related with nucleosome structures. The maximum of overhang index was present at 230 bp in both HCC and control subjects. The overhang indices of HCC subjects were generally higher than healthy subjects across different size ranges, and the difference in overhang index between control and HCC subjects was not even, suggesting a particular size ranges might enhance the separation between HCC and healthy subjects. So we reasoned that different size ranges might give rise to different discriminating power for distinguishing cancer subjects, monitoring immune diseases and noninvasive prenatal testing etc. To this end, we partitioned the plasma DNA molecules into different size windows including, but not limited to, 60-80 bp, 80-100 bp, 100-120 bp, 120-140 bp, 140-160 bp, 160-180 bp, 180-200 bp, 200-220 bp, 220-240 bp, 240-260 bp, 260-280 bp, 280-300 bp, 300-320 bp, 320-340 bp, 340-360 bp, 380-400 bp, 420-440 bp, 440-460 bp, 480-500 bp, 520-540 bp, 560-580 bp, and 580-600 bp, and quantified overhang indices among different subjects. **FIG. 52A** showed the area under curve values of receiver operating characteristic (ROC) analysis for overhang indices across different size ranges between healthy controls and HCC patients. A best discrimination between healthy and cancer subjects was achieved at the size range of 120-140 bp while all fragments without size selection in silico showed less discriminating power **(****FIG. 52B****,** p-value=0.2, Mann-Whitney test) suggesting that the size-range based analysis would improve the performance of overhang index based cancer detection.

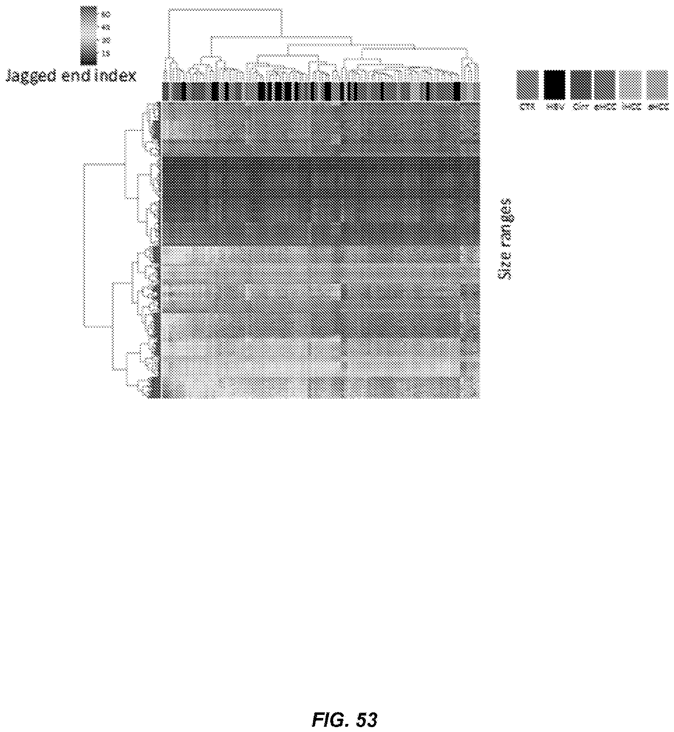

FIG. 53 shows a heatmap of jagged index across different size ranges for samples with different conditions. The cell-free DNA molecules show enormous diversity in terms of sizes, which can range from, but are not limited to, 50 bp to 600 bp. The jagged index can measured in a group of molecules with the same size. Therefore, each plasma DNA sample would harbor 600 groups of different sizes, corresponding 600 jagged indices. Such 600-dimensional jagged index vector could be used for hierarchical clustering, machine learning, and deep learning analysis. FIG. 53 showed that 600-dimensional jagged index generally allowed for distinguishing the cluster of HCC patients from the cluster of non-HCC patients, suggesting that size-banded high-dimensional jagged end indices may bear the information for detecting patients with cancer.

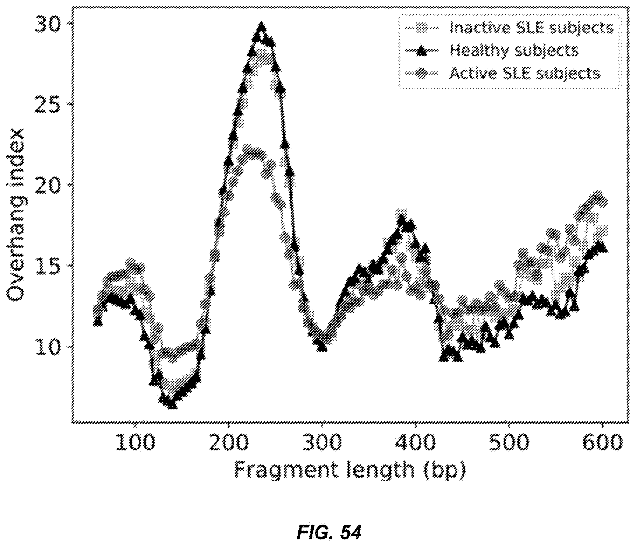

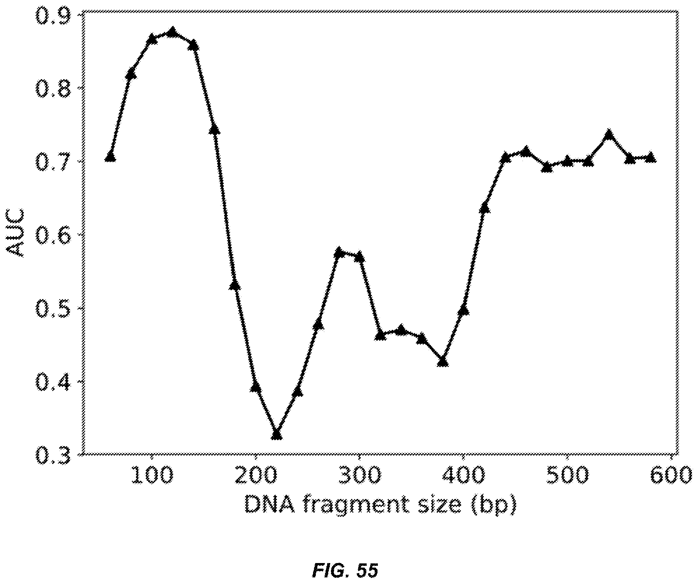

We also applied the size-range based analysis to active systemic lupus erythematosus (SLE) patients. Interestingly, we also found that there were multiple similar peaks occurring at 80 bp, 240 bp, 400 bp, and 560 bp in inactive and active SLE patients **(****FIG. 54****)** and the size range of 140-160 bp yielded a best power in differentiating active SLE patients **(****FIG. 55****).**

In another embodiment, the ratio of two overhang indices derived from different size ranges would be used for differentiating disease subjects from non-disease subjects. The patterns of overhang index across different size ranges could be used as features to train the classifier distinguishing disease from healthy statues through machine learning algorithms.

### D. Differential overhang index between pre- and post-operative plasma DNA of a HCC patient.

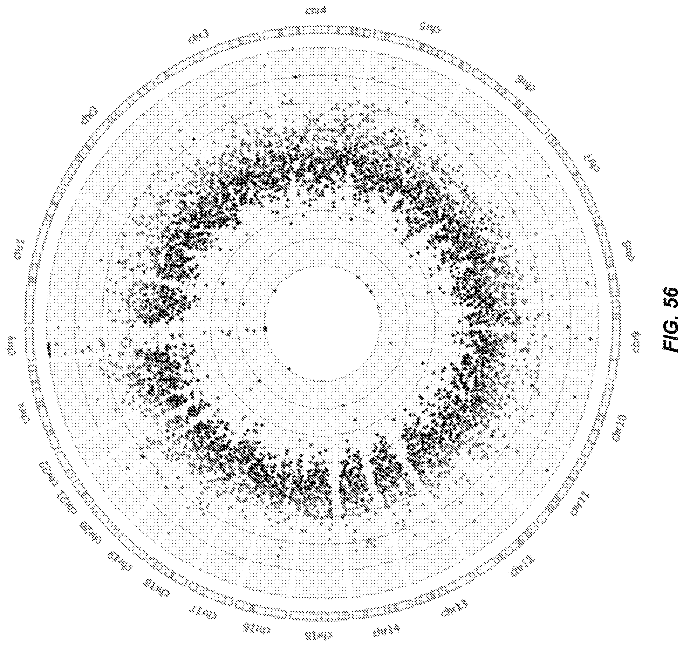

We also conducted the overhang analysis on pre- and post-surgery plasma DNA samples of one HCC patient by using those molecules with a size of between 120 and 140 bp. As a result, the overhang index of pre-surgery plasma DNA with its mean value of 8.9 was found to be significantly higher than post-surgery plasma DNA with a mean of 7.4 (P-value < 0.0001) in a genome-wide manner **(****FIG. 56****),** indicating that the overhang indices present in plasma DNA would be associated with different clinical conditions.

### E. Overhang index at genomic regions of interest would inform the tissue of origin

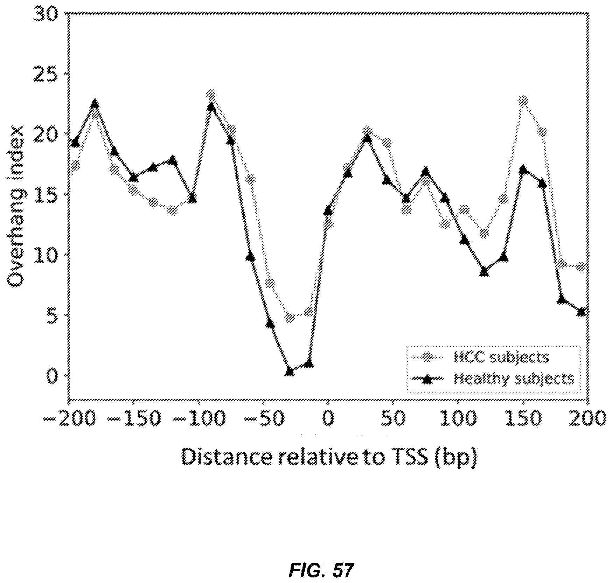

We further study the hypothesis that overhang index of plasma DNA in a set of particular genomic regions would enhance the deciphering of the tissue of origin of plasma DNA which may reflect the identity of a tumor or origin and allow cancer detection. To this end, we implemented approaches to investigate the properties of the overhang index across different tissue-specific open chromatin regions including but not limited to transcription start sites (TSS), DNase I hypersensitive regions, and enhancer or super-enhancer regions. Overhang indices were found to be unevenly distributed around TSS regions. The overhang indices proximal to TSS was relatively lower than those distal to TSS **(****FIG. 57****).** The overhang index of the data pooled from HCC subjects was a bit higher than those pooled from healthy subjects (FIG. 57), suggesting that different genomic regions would give different discriminating power between HCC and healthy subjects.

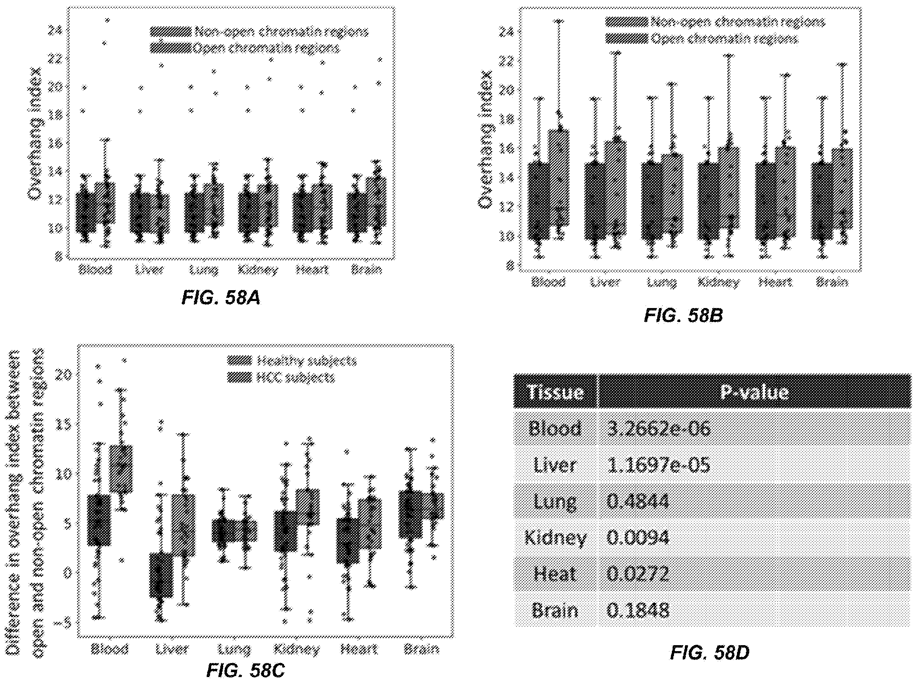

We also investigated the overhang indices between open chromatin regions and non-chromatin regions across different tissues/organs. The open chromatin regions were annotated in ENCODE project (The ENCODE Project Consortium. Nature. 2012;489:57-74). In general, the overhang index appeared to be higher in open chromatin regions than non-open chromatin regions **(****FIG. 58A****-****FIG. 58B****).** The most significant difference in overhang index between open and non-open chromatin regions was located to the blood lineage **(****FIG. 58C****-****FIG. 58D****).** The secondary significant difference in overhang index between open and non-open chromatin regions was pointed to the liver tissue **(****FIG. 58C****-****FIG. 58D****).** This result suggested that the analysis of overhang index of plasma DNA would reveal the tissues involving cancers.

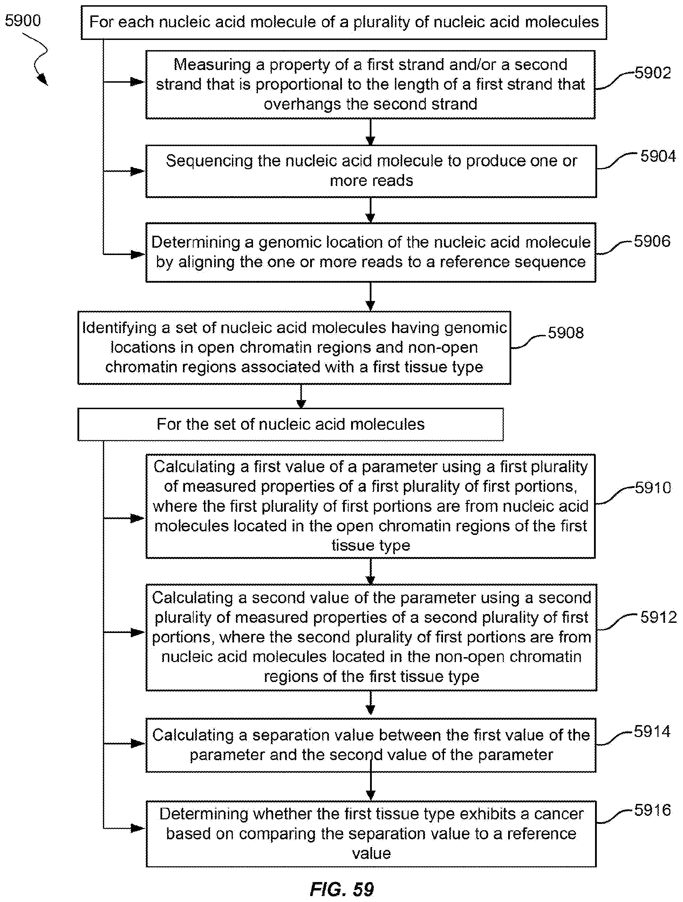

**FIG. 59** shows a method 5900 of analyzing a tissue type by analyzing a biological sample obtained from an individual. The biological sample may include a plurality of nucleic acid molecules. The plurality of nucleic acid molecules may be cell-free. Each nucleic acid molecule of the plurality of nucleic acid molecules may be double-stranded with a first strand having a first portion and a second strand. The first portion of the first strand of at least some of the plurality of nucleic acid molecules may overhang the second strand, may not be hybridized to the second strand, and may be at a first end of the first strand.

At block 5902, a property of the first strand and/or the second strand that is proportion to the length of a first strand that overhangs the second strand is measured. The property may be measured by any technique described herein. The property may be measured for each nucleic acid molecule of the plurality of nucleic acid molecules.

At block 5904, each nucleic acid molecule of the plurality of nucleic acid molecules is sequenced to produce one or more reads. The sequencing may be performed in various ways, e.g., as described herein. Example techniques may use probes, sequencing by synthesis, ligation, and nanopores.

At block 5906, a genomic location of each nucleic acid molecule of the plurality of nucleic acid molecules is determined, e.g., by aligning the one or more reads to a reference sequence or by using provides that are specific to particular genomic locations.

At block 5908, a set of nucleic acid molecules having genomic locations in open chromatin regions and non-open chromatin regions associated with a first tissue type are identified. Chromatin regions are described in US Application No. 16/402,910 filed May 3, 2019. As examples, the tissue type may include blood, liver, lung, kidney, heart, or brain. The open chromatin regions and non-open chromatin regions associated with the first tissue type may be retrieved from a database.

At block 5910, for the set of nucleic acid molecules, a first value of a parameter is calculated using a first plurality of measured properties of a first plurality of first portions. The first plurality of first portions are from nucleic acid molecules located in the open chromatin regions of the first tissue type. The measured property may be any jagged end value described herein. The parameter may be a statistical property of the measured property. For example, the parameter may be a mean, median, mode, or percentile of the measured properties.

At block 5912, for the set of nucleic acid molecules, a second value of the parameter is calculated using a second plurality of measured properties of a second plurality of first portions. The second plurality of first portions are from nucleic acid molecules located in the non-open chromatin regions of the first tissue type.

At block 5914, a separation value between the first value of the parameter and the second value of the parameter may be calculated. As examples, the separation value may include or be a difference between the first value and the second value or a ratio of the first value and the second value. Examples of various ratios and other separation values are provided herein, e.g., in the Terms section.

At block 5916, the first tissue type may be determined whether the first tissue type exhibits the cancer based on comparing the separation value to a reference value. The reference value may be determined using reference samples from reference subjects known to have cancer affecting a certain tissue and/or from reference subjects known to not have cancer affecting a certain tissue type. The first tissue type may be determined to exhibit the cancer, determined not to exhibit the cancer, or may be indeterminate.

In some embodiments, the determination can be performed using a machine learning model, e.g., as described for block 108 of FIG. 1.

### VIII. DNA CIRCULARIZATION FOR ASSESSING JAGGED ENDS

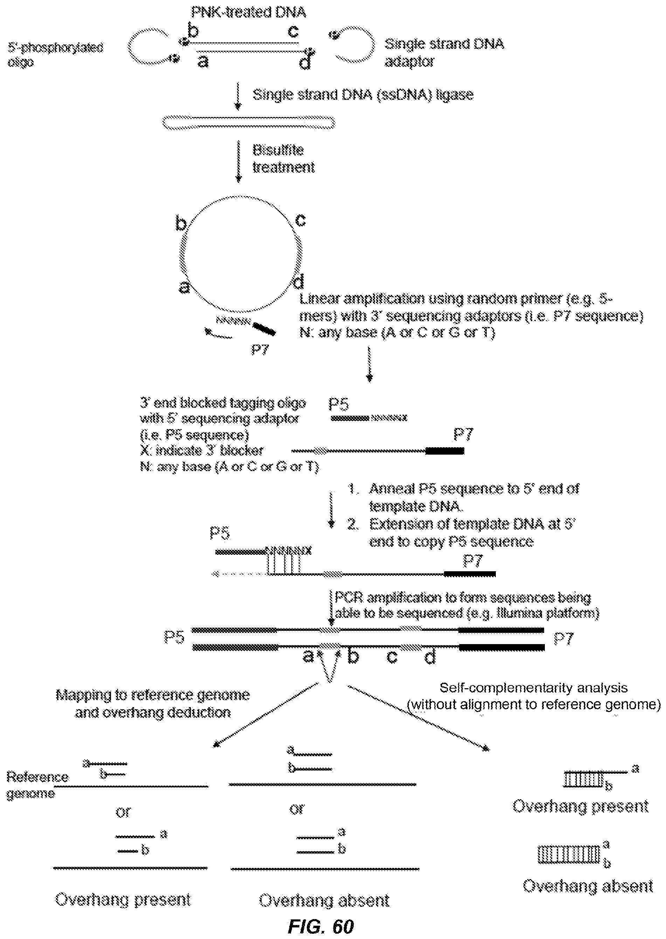

**FIG. 60** showed another embodiment for directly determining the overhangs for each DNA molecule by adding one extra single-stranded molecular adaptors to both sticky ends. Afterward, we use the sodium bisulfite to treat the double-stranded DNA with closed single-stranded ends such that the duplex structure will be disrupted to form the single-stranded circular DNA. Such single-stranded circular DNA molecules will be subject to random tagging-based amplification. The amplified product will be sheared by sonication to generate short fragments which will be sequenced subsequently. The original overhang information can be inferred from the junctions next to the extra added adaptor after aligning to the human reference genome.

FIG. 60 shows a direct assessment of plasma DNA sticky ends/overhangs through circularization of plasma DNA. The plasma DNA will be ligated with single strand DNA adaptors (yellow) through single-strand DNA (ssDNA) ligase. The bisulfite treatment will make the Watson (top strand) and Crick stands (bottom strand) no longer complementary because almost all cytosines from non-CpG sites in both strands would be converted to uracils, leading to form circularized single strand DNA molecules. Such circularized single strand DNA could be amplified using random primers (e.g. 5-mers) tagged with 3' sequencing adaptors (e.g. Illumina P7, blue), producing a number of linear DNA molecules which may comprise the single strand DNA adaptor (yellow). The DNA sequences flanking the originally ligated single strand adaptor would allow for inferring the jagged ends. To enable the linear DNA molecules to be suited for sequencing, the 5' sequencing adaptor (red, e.g. Illumina P5, red) will be incorporated via annealing and PCR-based extension. Then the molecules tagged with P5 and P7 adaptors will be amplified and sequenced. The sequences ("a" and "b" indicated by red arrows) flanking the original single strand adaptor (yellow) will be determined through alignment or self-complementarity analysis by studying the relative positions of "a" and "b" sequences as shown in the schematic. The "c" and "d" sequences in circularized molecules can be analyzed through the similar strategy as it is used for analyzing "a" and "b" sequences.

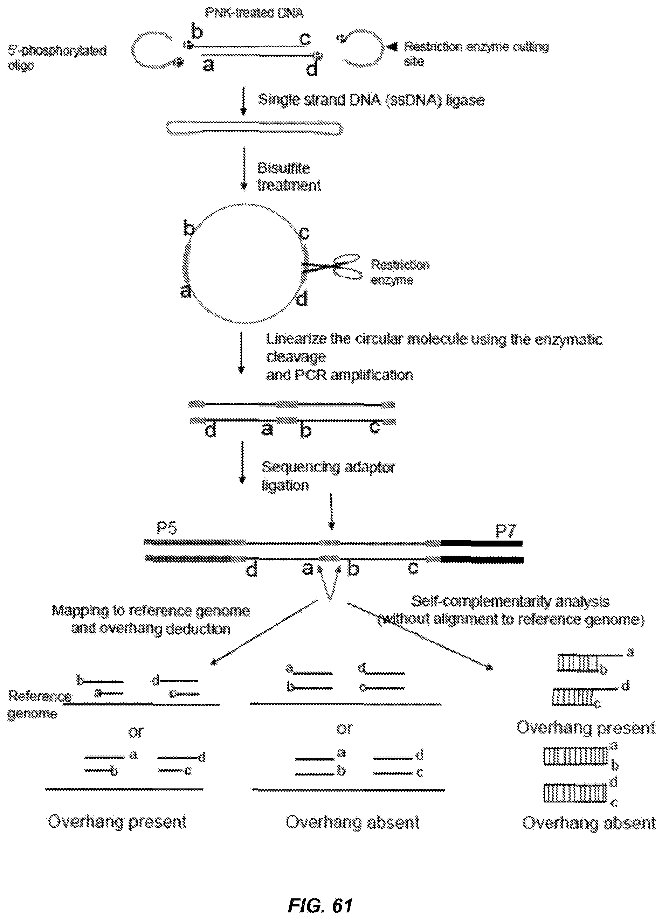

**FIG. 61** shows a technique similar to that in FIG. 60 but using a restriction enzyme. As with FIG. 60, the plasma DNA will be ligated with single strand DNA adaptors (yellow) through single-strand DNA (ssDNA) ligase. However, one of the single-strand DNA adaptors harbors the restriction enzyme cutting site. The bisulfite treatment will make the Watson (top strand) and Crick stands (bottom strand) no longer complementary because almost all cytosines from non-CpG sites in both strands would be converted to uracils, leading to form circularized single strand DNA molecules. A corresponding restriction enzyme would be used for cutting the circularized DNA molecules to produce the linearized DNA molecules. The linearized DNA molecules could be amplified via the universal sequences on adaptors (yellow). The amplified DNA molecules could be ligated with sequencing adaptors for sequencing. The "a", "b", "c" and "d" parts in sequencing reads could be used for inferring the jagged ends by comparing the relative end positions as illustrated in the schematic. This method allows for determining jagged ends on both ends of a DNA molecule.

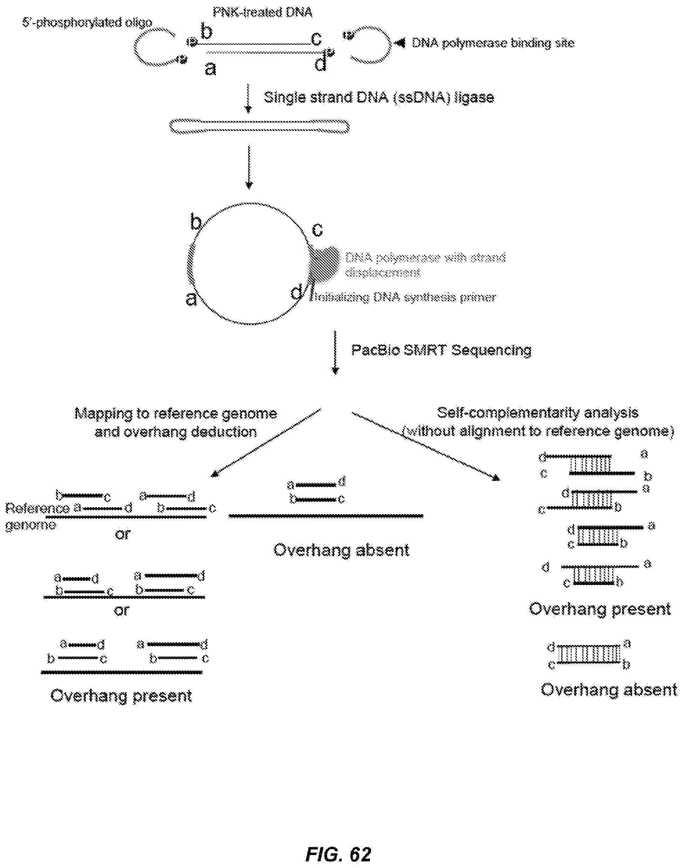

**FIG. 62** shows a technique similar to that in FIG. 60 but using a polymerase binding site. As with FIG. 60, the plasma DNA will be ligated with single strand DNA adaptors (yellow) through single-strand DNA (ssDNA) ligase. However, one of the single-strand DNA adaptors harbors a DNA polymerase binding site that would facilitate single DNA molecule sequencing (e.g. PacBio SMRT sequencing). Thus, the circularized molecule without bisulfite treatment can be bound to DNA polymerase in PacBio SMRT well and initialize the single molecule sequencing. The entire circularized molecule would be sequenced multiple times via "rolling". Each full run of rolling would generate so-called subreads. The consensus sequence would be produced by a number of subreads. The sequencing errors will be minimized by analyzing consensus sequences. Comparing the "ab" and "cd" entire sequences allows for determining the jagged ends in a single base resolution. This method could avoid bisulfite treatment, thus reducing DNA degradation during analysis. The forms of jagged ends can be present in, but not limited to, one of the forms illustrated in the schematic. The molecules carrying jagged ends would be shown to be non-blunt at least at one end of the molecule. Such an approach can detect any forms of jagged and blunt ends at the single molecule level.

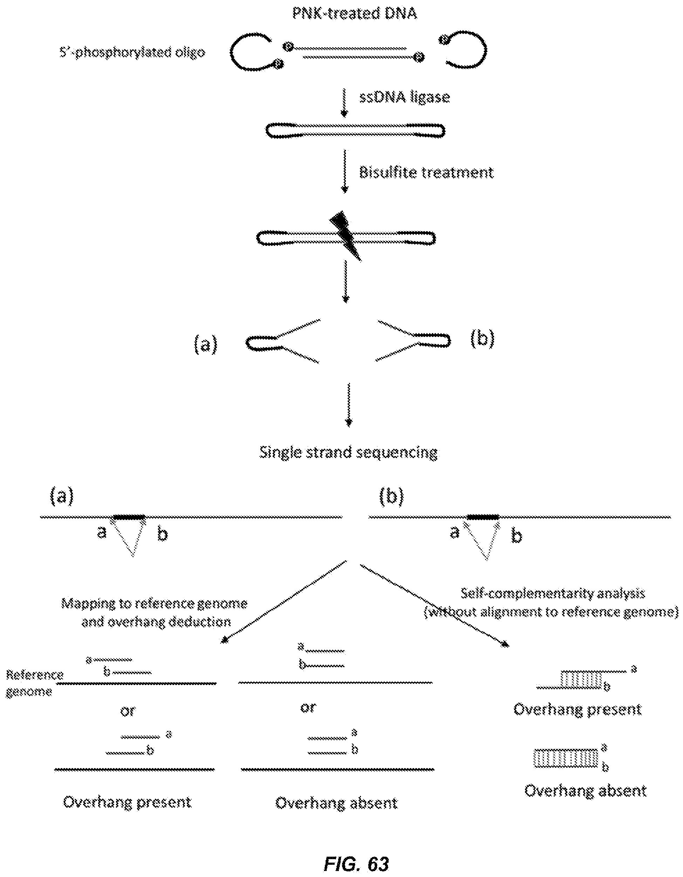

**FIG. 63** shows an embodiment that directly assesses overhangs but skips a random tagging step. Random tagging can be avoided because a considerable portion of DNA molecules will be fragmented during sodium bisulfite treatment, and the fragments allow direct sequencing of the DNA to detect the overhang information after sodium bisulfite treatment.

In FIG. 63, the plasma DNA jagged ends/overhangs are directly assessed through circularization of plasma DNA without random tagging amplification. The red arrows indicate the junctions between DNA and extra inserted adaptors, which would be used for inferring the overhangs by comparing the extent of complementarity between the bases directly adjacent to the junctions pointed out by the red arrows. With the reference to junctions, the end next to the junction of the left short sequence being interrogated for overhang will be labeled by "a"; the end next to the junction of the right short sequence being interrogated will be labeled by "b". After aligning to the short sequences labeled by "a" and "b" to a human reference genome, the offset of genomic coordinates between ends initially labeled with "a" and "b" will directly reflect the overhang present in plasma. Such overhang inference can also be done without alignment to reference genome because the left short sequence and the right short sequence directly adjacent to junctions could be partially complementary. The non-complementary single strand formed between "a" and "b" ends indicates the overhang.

### A. Example method cleaving circular nucleic acid molecule

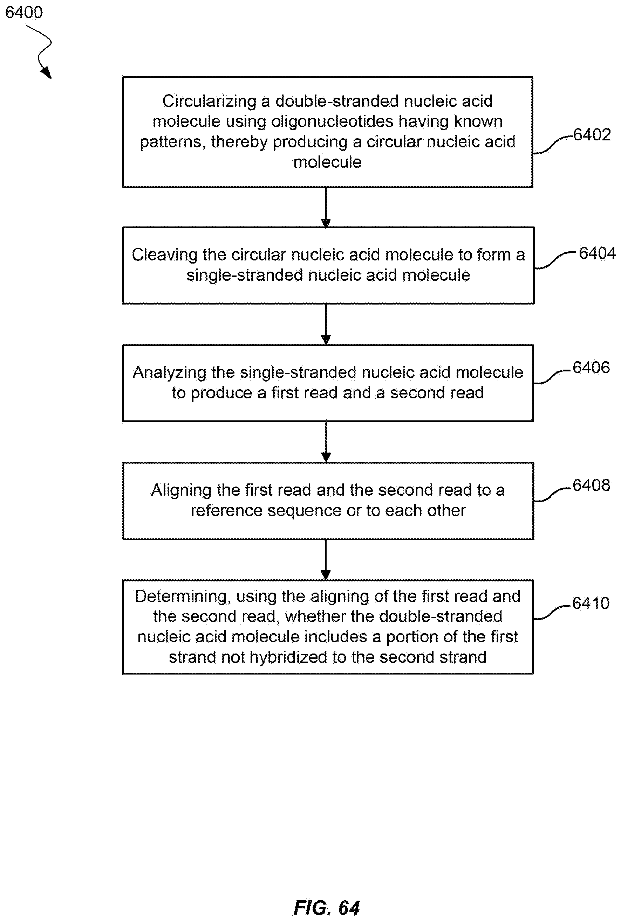

**FIG. 64** shows a method 6400 of analyzing a biological sample obtained from an individual. The biological sample may include a double-stranded nucleic acid molecule. The double-stranded nucleic acid molecules may be cell-free. The double-stranded nucleic acid molecule has a first strand and a second strand. The double-stranded nucleic acid molecule has a first end and a second end opposite the first end.

At block 6402, the double-stranded nucleic acid molecule is circularized using oligonucleotides having known patterns. A circular nucleic acid molecules is produced. The circular nucleic acid molecule may include the molecule in FIG. 60 or FIG. 61 after bisulfite treatment or the molecule after ssDNA ligase in FIG. 63, even if the molecule itself is not a perfect circle.

A circular nucleic acid molecule may be formed by attaching a first oligonucleotide to the first strand and the second strand at the first end. A second oligonucleotide may be attached to the first strand and the second strand at the second end. The second oligonucleotide may include a second known pattern of nucleotides. The circular nucleic acid molecule may include the first strand, the second strand, the first compound, and the second compound.

At block 6404, the circular nucleic acid molecule is cleaved to form a single-stranded nucleic acid molecule.

At block 6406, the single-stranded nucleic acid molecule is analyzed to produce a first read and a second read. The single-stranded nucleic acid molecule may include a first section including a pattern of nucleotides of the first strand at the first end to which the first read corresponds. The single-stranded nucleic acid molecule may also include a first nucleotide having a first known pattern of nucleotides. The single-stranded nucleic acid molecule may further include a second section including a second pattern of nucleotides of the second strand at the first end to which the second read corresponds. Analyzing the single-stranded nucleic acid molecule may also produce reads corresponding to the first oligonucleotide. The reads may be produced by sequencing the single-stranded nucleic acid molecule.

In some embodiments, analyzing the single-stranded nucleic acid molecule may include random tagging of the single-stranded nucleic acid molecule. A third oligonucleotide may be annealed to the single-stranded nucleic acid molecule. The third oligonucleotide may be a 3' end blocking tagging oligonucleotide, as in FIG. 60. The single-stranded nucleic acid molecule may be amplified to add sequencing adapters.

At block 6408, the first read and the second read are aligned to a reference sequence or to each other. The reference sequence may be a human reference genome.

At block 6410, whether the double-stranded nucleic acid molecule includes a portion of the first strand not hybridized to the second strand is determined using the aligning of the first read and the second read.

Method 6400 may further include determining the length of the portion of the first strand not hybridized to the second strand. Determining the length may use the aligning. The length may be the measured property in any of the methods described with FIG. 1.

### B. Example method analyzing circular nucleic acid molecule

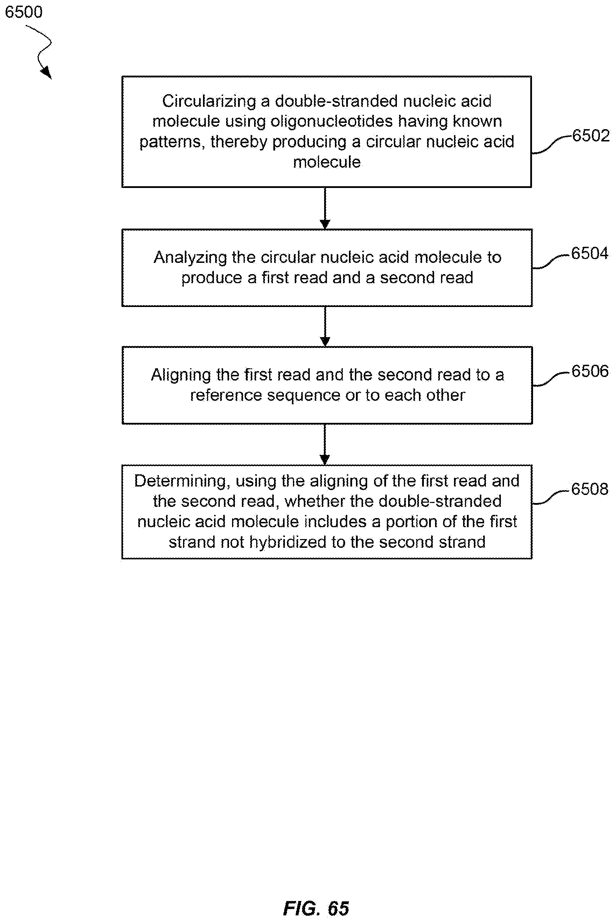

FIG. 65 shows a method 6500 of analyzing a biological sample obtained from an individual. The biological sample may include a double-stranded nucleic acid molecule. The double-stranded nucleic acid molecules may be cell-free. The double-stranded nucleic acid molecule has a first strand and a second strand. The double-stranded nucleic acid molecule has a first end and a second end opposite the first end.

At block 6502, the double-stranded nucleic acid molecule is circularized using oligonucleotides having known patterns. A circular nucleic acid molecules is produced. The circular nucleic acid molecule may include the molecule in FIG. 62.

A circular nucleic acid molecule may be formed by attaching a first oligonucleotide to the first strand and the second strand at the first end. A second oligonucleotide may be attached to the first strand and the second strand at the second end. The second oligonucleotide may include a second known pattern of nucleotides. The circular nucleic acid molecule may include the first strand, the second strand, the first compound, and the second compound.

At block 6504, the single-stranded nucleic acid molecule is analyzed to produce a first read and a second read. The single-stranded nucleic acid molecule may include a first section including a pattern of nucleotides of the first strand at the first end to which the first read corresponds. The single-stranded nucleic acid molecule may also include a first nucleotide having a first known pattern of nucleotides. The single-stranded nucleic acid molecule may further include a second section including a second pattern of nucleotides of the second strand at the first end to which the second read corresponds.

Analyzing the single-stranded nucleic acid molecule may also produce reads corresponding to the first oligonucleotide. The reads may be produced through single molecule sequencing of the circular nucleic acid molecule. A polymerase may be bound to the first oligonucleotide, and the polymerase may initialize single molecule sequencing, as described with FIG. 62 and the PacBio SMRT well. Method J00 may exclude bisulfite treatment.

At block 6506, the first read and the second read are aligned to a reference sequence or to each other. The reference sequence may be a human reference genome.

At block 6508, whether the double-stranded nucleic acid molecule includes a portion of the first strand not hybridized to the second strand is determined using the aligning of the first read and the second read.

Method 6500 may further include determining the length of the portion of the first strand not hybridized to the second strand. Determining the length may use the aligning. The length may be the measured property in any of the methods described with FIG. 1.

### IX. INOSINE-BASED SEQUENCING FOR ASSESSING THE CELL-FREE DNA OVERHANGS

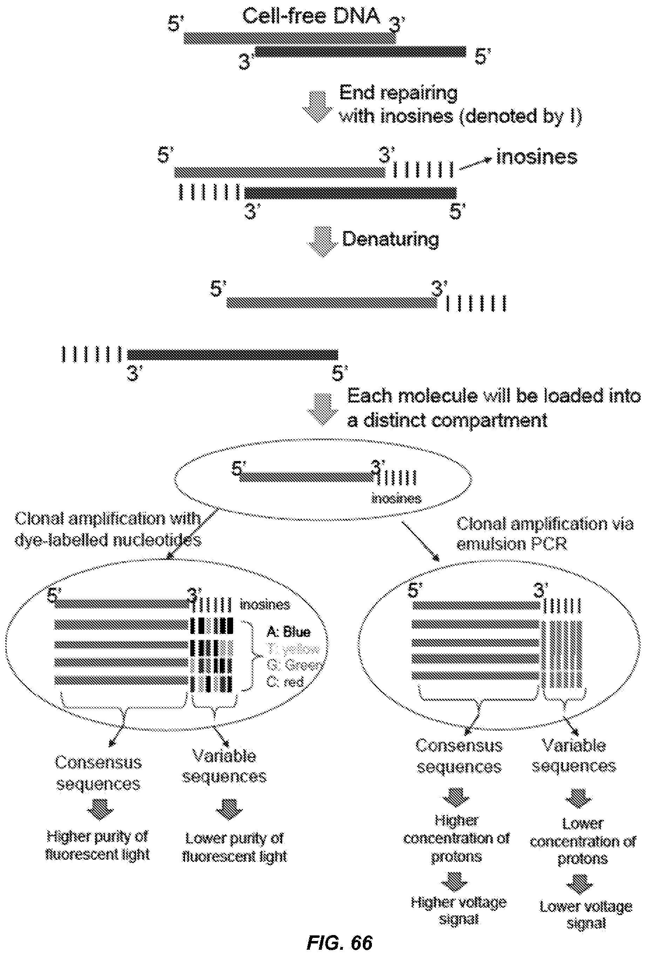

FIG. 66 shows how inosine based sequencing can be used to assess the jagged ends. Inosine can be used during end repair instead of the conventional dNTP. As shown in FIG. 66, inosine bases will be incorporated into the 3' end of strand exhibiting indentation relative to the opposite stand, indicated by a stretch of "I".

Because of the ability of inosine (I) to base pair (hybridize) with each of the four bases, the jagged ends of plasma DNA would be filled in with a series of inosines during end repairing if only inosines are mixed together with DNA polymerase. The DNA polymerase will synthesize DNA from 5' to 3'. Thus the 5' protruded strand will serve as DNA template to facilitate the incorporation of inosines onto the 3' end of the opposite strand. Once the DNA molecules carrying the jagged ends filled in with inosines, there are multiple ways to detect such a series of inosine on the opposite strand of 5' protruded ends. (1) Such a molecule can be ligated with sequencing adaptors. Adaptors-tagged molecules can be denatured into single-strand DNA molecules and loaded onto a compartment which containing adaptors (i.e. well, flowcell, droplet).

One compartment would only contain one molecule. In a media, there are millions of such compartments. The molecule in a compartment will be amplified by DNA polymerase mixed with 4 types of nucleotides (As, Cs, Gs, and Ts) which will be labeled by 4 types of dyes, respectively. The non-I bases (consensus sequence) in a compartment will generate higher purity of lights emitted from dyes activated by lasers than that of I bases corresponding the original jagged ends. The purity of fluorescent light can be defined by the brightest base intensity divided by the sum of the brightest and second-brightest base intensities. (2) The clonally amplified molecules in a compartment can be conducted in the Illumina sequencing platform. The sequencing results derived from jagged ends will contain much higher sequencing errors compared with the consensus sequence, thus allowing for differentiating the jagged ends for each molecule. On the other hand, the sequencing quality (base quality) will reduce dramatically on the region of jagged ends, which can be also used for inferring the jagged ends.

Another embodiment to detect inosines in a molecule use ion semiconductor sequencing or PacBio SMRT sequencing. For ion semiconductor sequencing, the emulsion PCR can be carried on in a compartment (microwell) using native nucleotides instead of using dye-labeled nucleotides. During sequencing, nucleotide species are added to the wells one at a time and a standard elongation reaction is performed. Each base incorporation, a single proton (H+) is generated as a by-product which would be converted to an electronic voltage signal by the semiconductor. The major electronic signals will be significantly reduced in the jagged ends compared with other regions due to the fact that the effective concentration of a particular type DNA template is diluted during clonal amplification in emulsion PCR. On the other hand, the baseline of background electronic signal would be higher along jagged end regions than that of consensus region because the addition of every new nucleotide would have chance being incorporated into one of the variable sequences whereas there would be only one type of nucleotides being properly incorporated during consensus regions every 4 nucleotides being rotated. In PacBio SMRT sequencing, the error rate will increase in the jagged ends when constructing consensus sequences from subreads. Other types of sequencing technologies might be also useful for the detection of such analogs being filled in during end repaired, for example, but not limited to ligation-based sequencing.

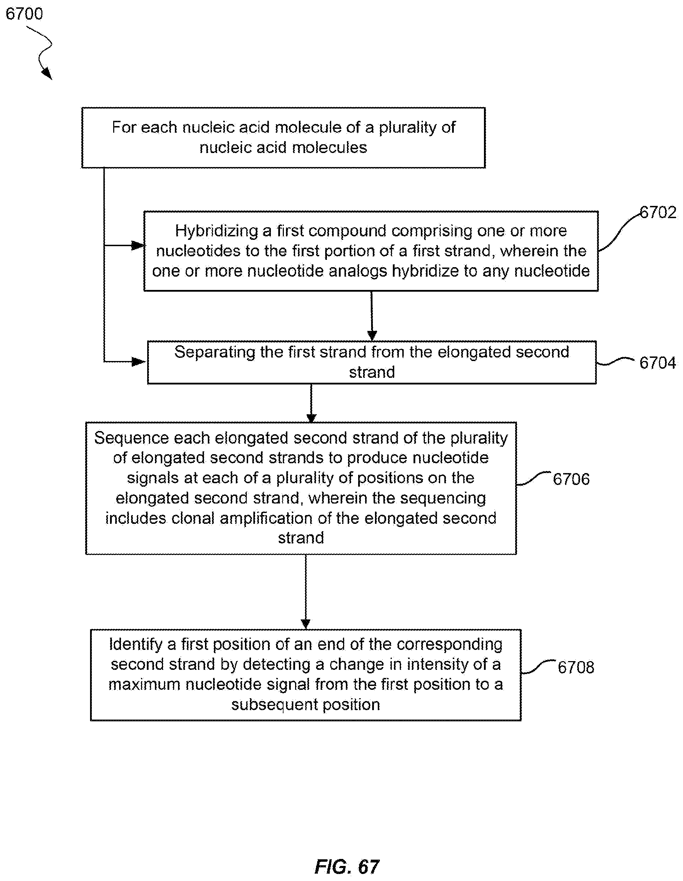

FIG. 67 shows a method 6700 for measuring a jagged end of a double-stranded nucleic acid molecule according to embodiments of the present invention. Method 6700 may be performed on jagged ends as described herein.

At block 6702, for each nucleic acid molecule of the plurality of nucleic acid molecules, a first compound comprising one or more nucleotide analogs is hybridized to the first portion of the first strand. The first compound and the second strand can form an elongated second strand. The one or more nucleotide analogs can hybridize to any nucleotide.

At block 6704, the first strand is separated from the first compound and the second strand.

At block 6706, each elongated second strand of the plurality of elongated second strands is sequenced to produce nucleotide signals at each of a plurality of positions on the elongated second strand. As examples, the nucleotide signals can be fluorescent or electrical signals. As described above, the sequencing can include clonal amplification of the elongated second strand, such that different bases may occur at the end of the elongated second strand.

At block 6708, for each elongated second strand of the plurality of elongated second strands, a first position of an end of the corresponding second strand is identified by detecting a change in intensity of a maximum nucleotide signal from the first position to a subsequent position. As described above, the change can be associated with an overall drop in signal quality as all of the nucleotides (bases) will have a similar intensity, since they all hybridize to the analog with equal probability (frequency).

The change in intensity can be greater than a threshold. The change in intensity greater than the threshold can be required to be sustained for N positions relative to the first position, where N is an integer greater than one, such as 2, 3, 4, 5, 6, 7, 8, 9, 10, etc.. The change in intensity of a maximum nucleotide signal can be relative to a second highest nucleotide signal. The change in intensity of a maximum nucleotide signal can be measured as a quality score of a base call at the first position.

### X. AGING AND OVERHANG

The ability to predict human aging from molecular profiles has important implications in a number of areas, including but not limited to, disease treatment, prevention, aging, drug responses as well as forensics. The inconsistency between chronological ages and cell-free molecular profile based age prediction would hint the disease and healthy statuses, and may be a biomarker for longevity or lack of longevity. FIG. 68 illustrates that plasma DNA overhang profiles could be used for predicting aging. The overhang index ratio was calculated by the overhang index of molecules within a range of 120 to 140 bp against that of all molecules without any size selection.

Accordingly, in some embodiments, the jagged end value can be compared to a reference value, and the age of the individual can be determined based on the comparison. For example, a reference value can be determined from a calibration curve 6802 fit to calibration data points 6804 or from any of the calibration data points 6804. Accordingly, the reference value can obtained using nucleic acid molecules from one or more reference subjects having known ages whose calibration samples are measured for a jagged end value. In some implementations, the plurality of nucleic acid molecules have sizes within a particular size range.

### XI. EXAMPLE SYSTEMS

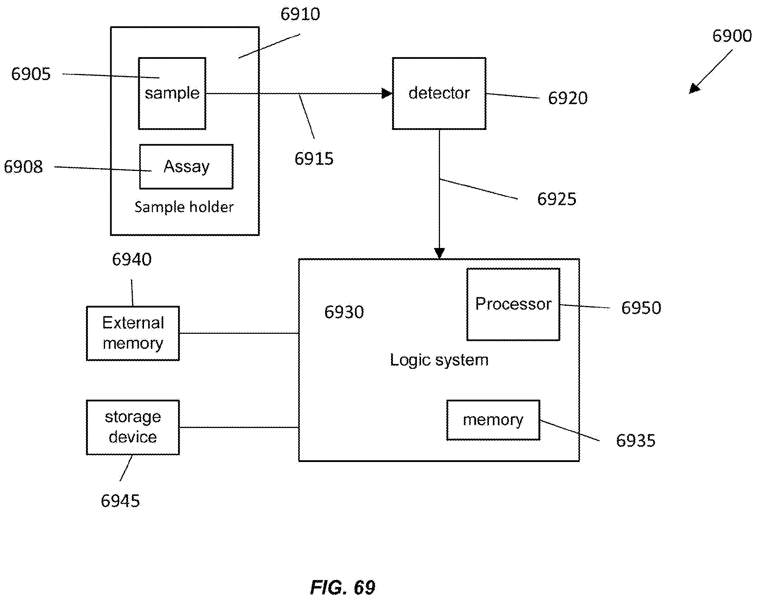

FIG. 69 illustrates a measurement system 6900 according to an embodiment of the present invention. The system as shown includes a sample 6905, such as cell-free DNA molecules within a sample holder 6910, where sample 6905 can be contacted with an assay 6908 to provide a signal of a physical characteristic 6915. An example of a sample holder can be a flow cell that includes probes and/or primers of an assay or a tube through which a droplet moves (with the droplet including the assay). Physical characteristic 6915 (e.g., a fluorescence intensity, a voltage, or a current), from the sample is detected by detector 6920. Detector 6920 can take a measurement at intervals (e.g., periodic intervals) to obtain data points that make up a data signal. In one embodiment, an analog-to-digital converter converts an analog signal from the detector into digital form at a plurality of times. Sample holder 6910 and detector 6920 can form an assay device, e.g., a sequencing device that performs sequencing according to embodiments described herein. A data signal 6925 is sent from detector 6920 to logic system 6930. Data signal 6925 may be stored in a local memory 6935, an external memory 6940, or a storage device 6945.

Logic system 6930 may be, or may include, a computer system, ASIC, microprocessor, etc. It may also include or be coupled with a display (e.g., monitor, LED display, etc.) and a user input device (e.g., mouse, keyboard, buttons, etc.). Logic system 6930 and the other components may be part of a stand-alone or network connected computer system, or they may be directly attached to or incorporated in a device (e.g., a sequencing device) that includes detector 6920 and/or sample holder 6910. Logic system 6930 may also include software that executes in a processor 6950. Logic system 6930 may include a computer readable medium storing instructions for controlling system 6900 to perform any of the methods described herein. For example, logic system 6930 can provide commands to a system that includes sample holder 6910 such that sequencing or other physical operations are performed. Such physical operations can be performed in a particular order, e.g., with reagents being added and removed in a particular order. Such physical operations may be performed by a robotics system, e.g., including a robotic arm, as may be used to obtain a sample and perform an assay.

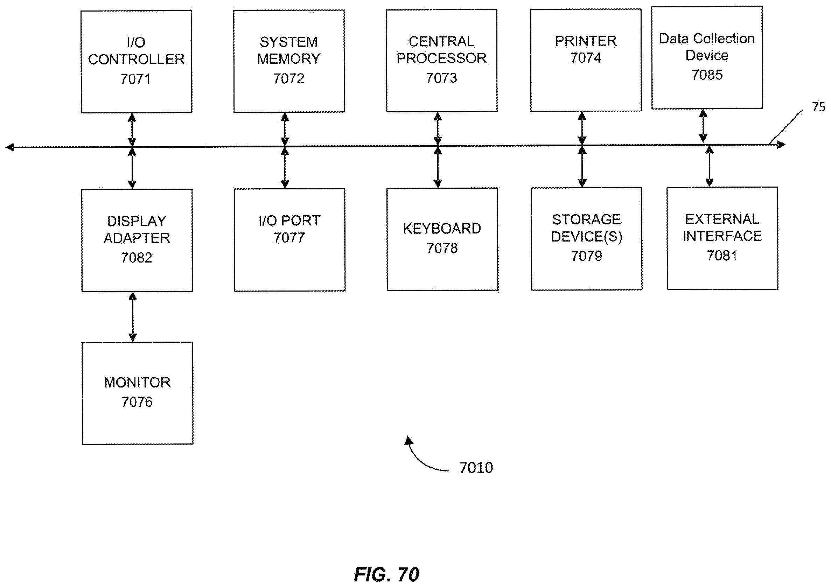

Any of the computer systems mentioned herein may utilize any suitable number of subsystems. Examples of such subsystems are shown in FIG. 70 in computer system 10. In some embodiments, a computer system includes a single computer apparatus, where the subsystems can be the components of the computer apparatus. In other embodiments, a computer system can include multiple computer apparatuses, each being a subsystem, with internal components. A computer system can include desktop and laptop computers, tablets, mobile phones and other mobile devices.

The subsystems shown in FIG. 70 are interconnected via a system bus 75. Additional subsystems such as a printer 74, keyboard 78, storage device(s) 79, monitor 76 (e.g., a display screen, such as an LED), which is coupled to display adapter 82, and others are shown. Peripherals and input/output (I/O) devices, which couple to I/O controller 71, can be connected to the computer system by any number of means known in the art such as input/output (I/O) port 77 (e.g., USB, FireWire^{®}). For example, I/O port 77 or external interface 81 (e.g. Ethernet, Wi-Fi, etc.) can be used to connect computer system 10 to a wide area network such as the Internet, a mouse input device, or a scanner. The interconnection via system bus 75 allows the central processor 73 to communicate with each subsystem and to control the execution of a plurality of instructions from system memory 72 or the storage device(s) 79 (e.g., a fixed disk, such as a hard drive, or optical disk), as well as the exchange of information between subsystems. The system memory 72 and/or the storage device(s) 79 may embody a computer readable medium. Another subsystem is a data collection device 85, such as a camera, microphone, accelerometer, and the like. Any of the data mentioned herein can be output from one component to another component and can be output to the user.

A computer system can include a plurality of the same components or subsystems, e.g., connected together by external interface 81, by an internal interface, or via removable storage devices that can be connected and removed from one component to another component. In some embodiments, computer systems, subsystem, or apparatuses can communicate over a network. In such instances, one computer can be considered a client and another computer a server, where each can be part of a same computer system. A client and a server can each include multiple systems, subsystems, or components.

Aspects of embodiments can be implemented in the form of control logic using hardware circuitry (e.g. an application specific integrated circuit or field programmable gate array) and/or using computer software with a generally programmable processor in a modular or integrated manner. As used herein, a processor can include a single-core processor, multi-core processor on a same integrated chip, or multiple processing units on a single circuit board or networked, as well as dedicated hardware. Based on the disclosure and teachings provided herein, a person of ordinary skill in the art will know and appreciate other ways and/or methods to implement embodiments of the present invention using hardware and a combination of hardware and software.

Any of the software components or functions described in this application may be implemented as software code to be executed by a processor using any suitable computer language such as, for example, Java, C, C++, C#, Objective-C, Swift, or scripting language such as Perl or Python using, for example, conventional or object-oriented techniques. The software code may be stored as a series of instructions or commands on a computer readable medium for storage and/or transmission. A suitable non-transitory computer readable medium can include random access memory (RAM), a read only memory (ROM), a magnetic medium such as a hard-drive or a floppy disk, or an optical medium such as a compact disk (CD) or DVD (digital versatile disk) or Blu-ray disk, flash memory, and the like. The computer readable medium may be any combination of such storage or transmission devices.

Such programs may also be encoded and transmitted using carrier signals adapted for transmission via wired, optical, and/or wireless networks conforming to a variety of protocols, including the Internet. As such, a computer readable medium may be created using a data signal encoded with such programs. Computer readable media encoded with the program code may be packaged with a compatible device or provided separately from other devices (e.g., via Internet download). Any such computer readable medium may reside on or within a single computer product (e.g. a hard drive, a CD, or an entire computer system), and may be present on or within different computer products within a system or network. A computer system may include a monitor, printer, or other suitable display for providing any of the results mentioned herein to a user.

Any of the methods described herein may be totally or partially performed with a computer system including one or more processors, which can be configured to perform the steps. Thus, embodiments can be directed to computer systems configured to perform the steps of any of the methods described herein, potentially with different components performing a respective step or a respective group of steps. Although presented as numbered steps, steps of methods herein can be performed at a same time or at different times or in a different order. Additionally, portions of these steps may be used with portions of other steps from other methods. Also, all or portions of a step may be optional. Additionally, any of the steps of any of the methods can be performed with modules, units, circuits, or other means of a system for performing these steps.

The specific details of particular embodiments may be combined in any suitable manner without departing from the scope of embodiments of the invention. However, other embodiments of the invention may be directed to specific embodiments relating to each individual aspect, or specific combinations of these individual aspects.

The above description of example embodiments of the present disclosure has been presented for the purposes of illustration and description. It is not intended to be exhaustive or to limit the disclosure to the precise form described, and many modifications and variations are possible in light of the teaching above.

A recitation of "a", "an" or "the" is intended to mean "one or more" unless specifically indicated to the contrary. The use of "or" is intended to mean an "inclusive or," and not an "exclusive or" unless specifically indicated to the contrary. Reference to a "first" component does not necessarily require that a second component be provided. Moreover, reference to a "first" or a "second" component does not limit the referenced component to a particular location unless expressly stated. The term "based on" is intended to mean "based at least in part on."

## Claims

1. A method of analyzing a biological sample obtained from an individual, the biological sample including a plurality of nucleic acid molecules, the plurality of nucleic acid molecules being cell-free, each nucleic acid molecule of the plurality of nucleic acid molecules being double-stranded with a first strand having a first portion and a second strand, wherein the first portion of the first strand of at least some of the plurality of nucleic acid molecules has no complementary portion from the second strand, is not hybridized to the second strand, and is at a first end of the first strand, the method comprising:
for each nucleic acid molecule of the plurality of nucleic acid molecules:
measuring a property of the first strand and/or the second strand that is proportional to a length of the first strand that overhangs the second strand;
determining a jagged end value using the measured properties of the plurality of nucleic acid molecules, wherein the jagged end value provides a collective measure that a strand overhangs another strand in the plurality of nucleic acid molecules;
comparing the jagged end value to a reference value; and
determining a level of a condition of the individual based on the comparison.

2. The method of claim 1, wherein the condition comprises a disease, a disorder, or a pregnancy.

3. The method of claim 2, wherein the condition is a cancer, an auto-immune disease, or a pregnancy-related condition.

4. The method of any one of the preceding claims, wherein the first end is a 5' end.

5. The method of any one of the preceding claims, further comprising:
measuring sizes of nucleic acid molecules, wherein the plurality of nucleic acid molecules has sizes within a specified range.

6. The method of claim 5, wherein the specified range is 140 to 160 bp.

7. The method of claim 5 or 6, wherein:
the plurality of nucleic acid molecules is a first plurality of nucleic acid molecules, and
the specified range is a first specified range,
the method further comprising:
measuring the property of a strand of each nucleic acid molecule of a second plurality of nucleic acid molecules, wherein the second plurality of nucleic acid molecules has sizes with a second specified range,
wherein determining the jagged end value comprises calculating a ratio using the measured properties of the first plurality of nucleic acid molecules and the measured properties of the second plurality of nucleic acid molecules.

8. The method of any one of the preceding claims, wherein the property is a methylation status at one or more sites at end portions of the first strands, the second strands, or the first strands and the second strands of each of the plurality of nucleic acid molecules, and wherein the jagged end value includes a methylation level over the plurality of nucleic acid molecules at one or more sites of end portions of the first strands, the second strands, or the first strands and the second strands.

9. The method of claim 8, wherein a higher methylation level is correlated with a longer length of the first strand that overhangs the second strand.

10. The method of any one of the preceding claims, further comprising:
analyzing nucleic acid molecules to produce reads,
aligning the reads to a reference genome,
wherein:
the plurality of nucleic acid molecules has reads within a certain distance range relative to a transcription start site.

11. The method of any one of the preceding claims, wherein the measured property is length.

12. The method of any one of the preceding claims, wherein the reference value is determined using one or more reference samples of subjects that have the condition.

13. The method of any one of the preceding claims, wherein the reference value is determined using one or more reference samples of subjects that do not have the condition.

14. The method of any one of the preceding claims, wherein a machine learning model is used to perform the comparing of the jagged end value to the reference value and the determining of the level of the condition of the individual.

15. A computer program product comprising instructions that when executed control a computer system to perform the method of any one of the preceding claims.

## Patentansprüche

1. Verfahren zum Analysieren einer biologischen Probe, die von einem Individuum erhalten wurde, wobei die biologische Probe eine Mehrzahl von Nukleinsäuremolekülen beinhaltet, wobei die Mehrzahl von Nukleinsäuremolekülen zellfrei ist, wobei jedes Nukleinsäuremolekül der Mehrzahl von Nukleinsäuremolekülen doppelsträngig mit einem ersten Strang mit einem ersten Abschnitt und einem zweiten Strang ist, wobei der erste Abschnitt des ersten Strangs von wenigstens einigen der Mehrzahl von Nukleinsäuremolekülen keinen komplementären Abschnitt von dem zweiten Strang aufweist, nicht mit dem zweiten Strang hybridisiert ist und sich an einem ersten Ende des ersten Strangs befindet, wobei das Verfahren umfasst:
für jedes Nukleinsäuremolekül der Mehrzahl von Nukleinsäuremolekülen:
Messen einer Eigenschaft des ersten Strangs und/oder des zweiten Strangs, die proportional zu einer Länge des ersten Strangs ist, der den zweiten Strang überhängt;
Bestimmen eines gezackten Endwertes unter Verwendung der gemessenen Eigenschaften der Mehrzahl von Nukleinsäuremolekülen, wobei der gezackte Endwert ein kollektives Maß bereitstellt, dass ein Strang einen anderen Strang in der Mehrzahl von Nukleinsäuremolekülen überhängt;
Vergleichen des gezackten Endwertes mit einem Referenzwert; und
Bestimmen eines Niveaus eines Zustands des Individuums auf Grundlage des Vergleichs.

2. Verfahren nach Anspruch 1, wobei der Zustand eine Erkrankung, eine Krankheit oder eine Schwangerschaft umfasst.

3. Verfahren nach Anspruch 2, wobei der Zustand ein Krebs, eine Autoimmunerkrankung oder ein schwangerschaftsbedingter Zustand ist.

4. Verfahren nach einem der vorhergehenden Ansprüche, wobei das erste Ende ein 5'-Ende ist.

5. Verfahren nach einem der vorhergehenden Ansprüche, das ferner umfasst:
Messen der Größen von Nukleinsäuremolekülen, wobei die Mehrzahl von Nukleinsäuremolekülen Größen innerhalb eines spezifizierten Bereichs aufweist.

6. Verfahren nach Anspruch 5, wobei der spezifizierte Bereich 140 bis 160 bp beträgt.

7. Verfahren nach Anspruch 5 oder 6, wobei:
die Mehrzahl von Nukleinsäuremolekülen eine erste Mehrzahl von Nukleinsäuremolekülen ist, und
der spezifizierte Bereich ein erster spezifizierter Bereich ist,
wobei das Verfahren ferner umfasst:
Messen der Eigenschaft eines Strangs jedes Nukleinsäuremoleküls einer zweiten Mehrzahl von Nukleinsäuremolekülen, wobei die zweite Mehrzahl von Nukleinsäuremolekülen Größen mit einem zweiten spezifizierten Bereich aufweist,
wobei das Bestimmen des gezackten Endwertes das Berechnen eines Verhältnisses unter Verwendung der gemessenen Eigenschaften der ersten Mehrzahl von Nukleinsäuremolekülen und der gemessenen Eigenschaften der zweiten Mehrzahl von Nukleinsäuremolekülen umfasst.

8. Verfahren nach einem der vorhergehenden Ansprüche, wobei die Eigenschaft ein Methylierungsstatus an einer oder mehreren Stellen an Endabschnitten der ersten Stränge, der zweiten Stränge oder der ersten Stränge und der zweiten Stränge von jedem der Mehrzahl von Nukleinsäuremolekülen ist, und wobei der gezackte Endwert ein Methylierungsniveau über die Mehrzahl von Nukleinsäuremolekülen an einer oder mehreren Stellen von Endabschnitten der ersten Stränge, der zweiten Stränge oder der ersten Stränge und der zweiten Stränge ist.

9. Verfahren nach Anspruch 8, wobei ein höheres Methylierungsniveau mit einer längeren Länge des ersten Strangs korreliert, der den zweiten Strang überhängt.

10. Verfahren nach einem der vorhergehenden Ansprüche, das ferner umfasst:
Analysieren von Nukleinsäuremolekülen für die Produktion von Reads,
Ausrichten der Reads an einem Referenzgenom,
wobei:
die Mehrzahl der Nukleinsäuremoleküle Reads innerhalb eines bestimmten Abstandsbereichs relativ zu einer Transkriptionsstartstelle aufweist.

11. Verfahren nach einem der vorhergehenden Ansprüche, wobei die gemessene Eigenschaft die Länge ist.

12. Verfahren nach einem der vorhergehenden Ansprüche, wobei der Referenzwert unter Verwendung von einer oder mehreren Referenzproben von Subjekten bestimmt wird, die den Zustand haben.

13. Verfahren nach einem der vorhergehenden Ansprüche, wobei der Referenzwert unter Verwendung einer oder mehrerer Referenzproben von Subjekten bestimmt wird, die den Zustand nicht haben.

14. Verfahren nach einem der vorhergehenden Ansprüche, wobei ein maschinelles Lernmodell verwendet wird, um das Vergleichen des gezackten Endwertes mit dem Referenzwert und das Bestimmen des Zustands des Individuums durchzuführen.

15. Computerprogrammprodukt, das Anweisungen umfasst, die, wenn sie ausgeführt werden, ein Computersystem steuern, um das Verfahren nach einem der vorhergehenden Ansprüche durchzuführen.

## Revendications

1. Procédé d'analyse d'un échantillon biologique prélevé sur un individu, l'échantillon biologique incluant une pluralité de molécules d'acide nucléique, la pluralité de molécules d'acide nucléique étant acellulaires, chaque molécule d'acide nucléique de la pluralité de molécules d'acide nucléique étant double brin avec un premier brin ayant une première portion et un deuxième brin, dans lequel la première portion du premier brin d'au moins certaines de la pluralité de molécules d'acide nucléique n'a pas de portion complémentaire issue du deuxième brin, n'est pas hybridée au deuxième brin, et se trouve à une première extrémité du premier brin, le procédé comprenant :
pour chaque molécule d'acide nucléique de la pluralité de molécules d'acide nucléique :
la mesure d'une propriété du premier brin et/ou du deuxième brin qui est proportionnelle à une longueur du premier brin qui dépasse le deuxième brin ;
la détermination d'une valeur d'extrémité irrégulière à l'aide des propriétés mesurées de la pluralité de molécules d'acide nucléique, dans lequel la valeur d'extrémité irrégulière fournit une mesure collective selon laquelle un brin dépasse un autre brin dans la pluralité de molécules d'acide nucléique ;
la comparaison de la valeur d'extrémité irrégulière à une valeur de référence ; et
la détermination d'un niveau d'une affection de l'individu sur la base de la comparaison.

2. Procédé selon la revendication 1, dans lequel l'affection comprend une maladie, un trouble, ou une grossesse.

3. Procédé selon la revendication 2, dans lequel l'affection est un cancer, une maladie auto-immune, ou une affection liée à la grossesse.

4. Procédé selon l'une quelconque des revendications précédentes, dans lequel la première extrémité est une extrémité 5'.

5. Procédé selon l'une quelconque des revendications précédentes, comprenant en outre :
la mesure de tailles de molécules d'acide nucléique, dans lequel la pluralité de molécules d'acide nucléique a des tailles dans une plage spécifiée.

6. Procédé selon la revendication 5, dans lequel la plage spécifiée est de 140 à 160 pb.

7. Procédé selon la revendication 5 ou 6, dans lequel :
la pluralité de molécules d'acide nucléique est une première pluralité de molécules d'acide nucléique, et
la plage spécifiée est une première plage spécifiée,
le procédé comprenant en outre :
la mesure de la propriété d'un brin de chaque molécule d'acide nucléique d'une deuxième pluralité de molécules d'acide nucléique, dans lequel la deuxième pluralité de molécules d'acide nucléique a des tailles ayant une deuxième plage spécifiée,
dans lequel la détermination de la valeur d'extrémité irrégulière comprend le calcul d'un rapport à l'aide des propriétés mesurées de la première pluralité de molécules d'acide nucléique et des propriétés mesurées de la deuxième pluralité de molécules d'acide nucléique.

8. Procédé selon l'une quelconque des revendications précédentes, dans lequel la propriété est un état de méthylation en un ou plusieurs sites au niveau de portions d'extrémité des premiers brins, des deuxièmes brins, ou des premiers brins et des deuxièmes brins de chacune de la pluralité de molécules d'acide nucléique, et dans lequel la valeur d'extrémité irrégulière inclut un niveau de méthylation sur la pluralité de molécules d'acide nucléique en un ou plusieurs sites de portions d'extrémité des premiers brins, des deuxièmes brins, ou des premiers brins et des deuxièmes brins.

9. Procédé de la revendication 8, dans lequel un niveau de méthylation plus élevé est corrélé à une plus grande longueur du premier brin qui dépasse le deuxième brin.

10. Procédé selon l'une quelconque des revendications précédentes, comprenant en outre :
l'analyse de molécules d'acide nucléique pour produire des lectures,
l'alignement des lectures sur un génome de référence,
dans lequel :
la pluralité de molécules d'acide nucléique a des lectures dans une certaine plage de distance par rapport à un site de début de transcription.

11. Procédé selon l'une quelconque des revendications précédentes, dans lequel la propriété mesurée est la longueur.

12. Procédé selon l'une quelconque des revendications précédentes, dans lequel la valeur de référence est déterminée à l'aide d'un ou de plusieurs échantillons de référence de sujets qui sont atteints de l'affection.

13. Procédé selon l'une quelconque des revendications précédentes, dans lequel la valeur de référence est déterminée à l'aide d'un ou de plusieurs échantillons de référence de sujets qui ne sont pas atteints de l'affection.

14. Procédé selon l'une quelconque des revendications précédentes, dans lequel un modèle d'apprentissage automatique est utilisé pour effectuer la comparaison de la valeur d'extrémité irrégulière à la valeur de référence et la détermination du niveau de l'affection de l'individu.

15. Produit de programme informatique comprenant des instructions qui, lorsqu'elles sont exécutées, commandent un système informatique pour effectuer le procédé selon l'une quelconque des revendications précédentes.
